# EUROPEAN PATENT SPECIFICATION

(11) **EP 4 448 548 B1**
(45) Date of publication and mention of the grant of the patent: **22.10.2025**
(21) Application number: 22847091.0
(22) Date of filing: 12.12.2022
(51) Int. Cl.: C07K 14/005, A61K 39/015, A61K 39/12, C07K 14/47, C12N 7/00

(54) **BACTERIOPHAGE LAMBDA-VACCINE SYSTEM**
BAKTERIOPHAGEN-LAMBDA-IMPFSTOFFSYSTEM
SYSTÈME DE VACCIN DU BACTÉRIOPHAGE LAMBDA

(30) Priority: 13.12.2021 US 202163289018 P
(43) Date of publication of application: 23.10.2024
(73) Proprietor: The United States of America, as represented by The Secretary, Department of Health and Human Services, Bethesda, MD 20892-7788 (US)
(72) Inventor: ADHYA, Sankar L., Bethesda, Maryland 20892 (US); COURT, Donald L., Frederick, Maryland 21702-1201 (US); LI, Xintian, Monrovia, Maryland 21770 (US); RAJAURE, Manoj, Federick, Maryland 21704 (US)
(74) Representative: Tombling, Adrian George
(86) International application number: PCT/US2022/081383
(87) International publication number: WO 2023/114727

(56) References cited:
- WO-A1-2009/135295
- WO-A1-2019/102265
- WO-A1-2021/188818
- STERNBERG NAT ET AL: "Display of peptides and proteins on the surface of bacteriophage lambda", PROCEEDINGS OF THE NATIONAL ACADEMY OF SCIENCES, NATIONAL ACADEMY OF SCIENCES, vol. 92, no. 5, 1 January 1995 (1995-01-01), pages 1609 - 1613, XP002203953, ISSN: 0027-8424, DOI: 10.1073/PNAS.92.5.1609
- GONZÁLEZ-MORA ALEJANDRO ET AL: "Bacteriophage-Based Vaccines: A Potent Approach for Antigen Delivery", VACCINES, vol. 8, no. 3, 4 September 2020 (2020-09-04), pages 504, XP093036275, Retrieved from the Internet <URL:https://www.ncbi.nlm.nih.gov/pmc/articles/PMC7565293/pdf/vaccines-08-00504.pdf> DOI: 10.3390/vaccines8030504
- NICASTRO JESSICA ET AL: "Bacteriophage lambda display systems: developments and applications", APPLIED MICROBIOLOGY AND BIOTECHNOLOGY, SPRINGER BERLIN HEIDELBERG, BERLIN/HEIDELBERG, vol. 98, no. 7, 19 January 2014 (2014-01-19), pages 2853 - 2866, XP035329092, ISSN: 0175-7598, [retrieved on 20140119], DOI: 10.1007/S00253-014-5521-1
- MERRIL C R ET AL: "LONG-CIRCULATING BACTERIOPHAGE AS ANTIBACTERIAL AGENTS", PROCEEDINGS OF THE NATIONAL ACADEMY OF SCIENCES, NATIONAL ACADEMY OF SCIENCES, vol. 93, no. 8, 16 April 1996 (1996-04-16), pages 3188 - 3192, XP002044135, ISSN: 0027-8424, DOI: 10.1073/PNAS.93.8.3188
- KATSURA ET AL: "Structure and inherent properties of the bacteriophage lambda head shell - VI. DNA-packaging-defective mutants in the major capsid protein", JOURNAL OF MOLECULAR BIOLOGY, ACADEMIC PRESS, UNITED KINGDOM, vol. 205, no. 2, 20 January 1989 (1989-01-20), pages 397 - 405, XP024010006, ISSN: 0022-2836, [retrieved on 19890120], DOI: 10.1016/0022-2836(89)90350-1
- SCHWAENEN C ET AL: "Automated array-based genomic profiling in chronic lymphocytic leukemia: Development of a clinical tool and discovery of recurrent genomic alterations", PROCEEDINGS OF THE NATIONAL ACADEMY OF SCIENCES, NATIONAL ACADEMY OF SCIENCES, vol. 101, no. 4, 27 January 2004 (2004-01-27), pages 1039 - 1044, XP002345481, ISSN: 0027-8424, DOI: 10.1073/PNAS.0304717101
- ADHYA SANKAR: "Bacteriophage Based-Vaccine System | Technology Transfer", 16 August 2022 (2022-08-16), pages 1 - 4, XP093036291, Retrieved from the Internet <URL:https://www.techtransfer.nih.gov/bundle/nci-e-113-2021> [retrieved on 20230330]

## Description

### FIELD OF THE DISCLOSURE

This relates to vaccines, specifically to a bacteriophage lambda (λ) that includes a lambda genome with a nucleic acid sequence, encoding a fusion protein comprising the phage capsid D protein linked to a heterologous antigenic peptide, encoded within the native *gene D* locus.

### SEQUENCE LISTING

The Sequence Listing is submitted as an Extensible Markup Language (XML) file.

### BACKGROUND

Several bacterial and eucaryotic viruses are capable of displaying proteins and peptides on their virions. Early bacterial viruses (also known as bacteriophages or phages) used for this purpose were the filamentous phage M13 and the temperate phage λ (Smith, Science., doi: 10.1126/science.4001944, 1985; Sternberg, and Hoess, Proc Natl Acad Sci USA 92, 1609-1613 (1995)). Both phages grow in *Escherichia coli* (*E. coli*) and are commonly used as models in the laboratory. Appropriate simple genetic engineering tools have been developed in both systems to make such display processes feasible (Li et al., Nucl. Acids Res., 41: e204 doi:10.1093/nar/gkt1075, 2013). Display phages have been useful in investigating the physiological behavior of both phage and bacterial cells. More recently, the two phages are being used in understanding, detection, and curing of diseases. Among various phages available, the temperate phage λ appears to be the most suitable vector for such purposes because of its well-studied genetics and physiology and for being amenable to superb engineering tricks. However, a need remains for methods to produce λ virion particles that display vaccine antigens with high efficacy on their surface.

### SUMMARY OF THE DISCLOSURE

Bacteriophage λ are disclosed herein that include a head, a tail, and a lambda genome comprising a nucleic acid sequence encoding a fusion protein comprising a D protein linked to heterologous antigen, wherein the heterologous nucleic acid sequence is inserted into a native *gene D* locus adjacent to *gene E* in the lambda genome, and wherein expression of the fusion protein results in the head of the bacteriophage λ comprising the fusion protein.

Host bacterial cells are also disclosed herein that are infected with the bacteriophage λ, wherein the λ genome becomes integrated into a chromosome of the host bacterial cell as a prophage.

In further embodiments, immunogenic compositions are disclosed that include an effective amount of the bacteriophage λ, and a pharmaceutically acceptable carrier. In addition, methods are disclosed for inducing an immune response to the heterologous antigen in a subject. These methods include administering to the subject an effective amount of this immunogenic composition to induce the immune response to the heterologous antigen.

In other embodiments, methods are disclosed for preparing bacteriophage λ. These methods include propagating a host bacterial cell with the prophage λ, inducing the lytic phase, and isolating phage particles.

The foregoing and other features of the disclosure will become more apparent from the following detailed description of several embodiments which proceeds with reference to the accompanying figures.

### BRIEF DESCRIPTION OF THE FIGURES

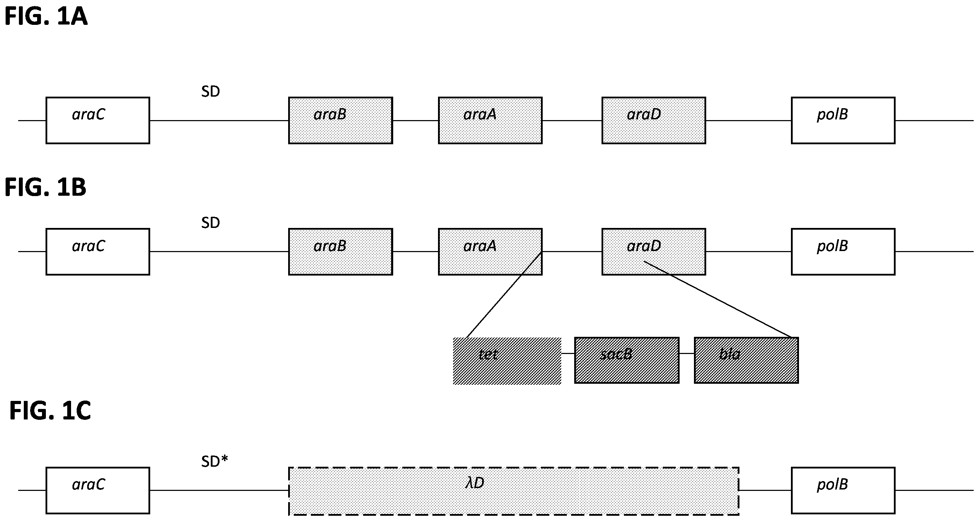

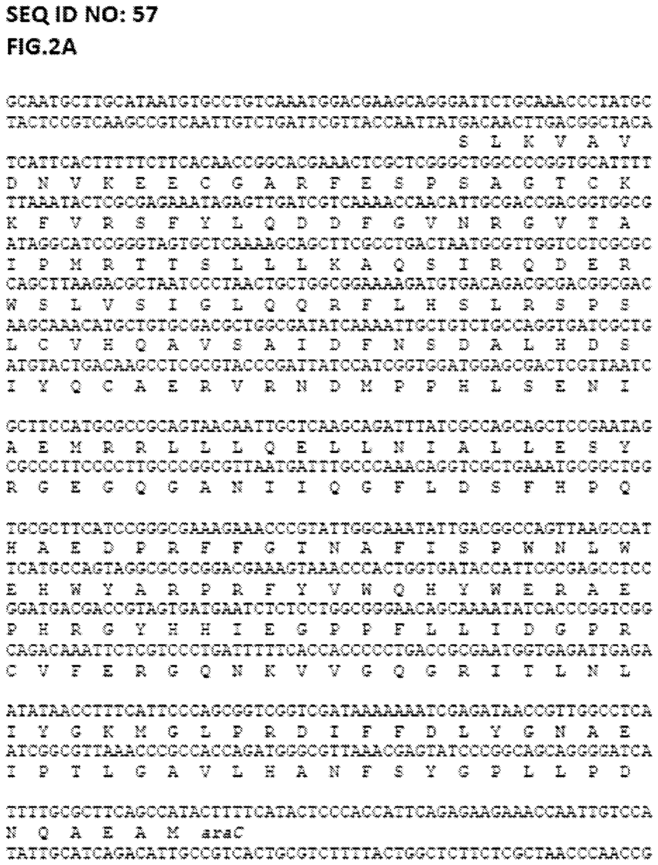

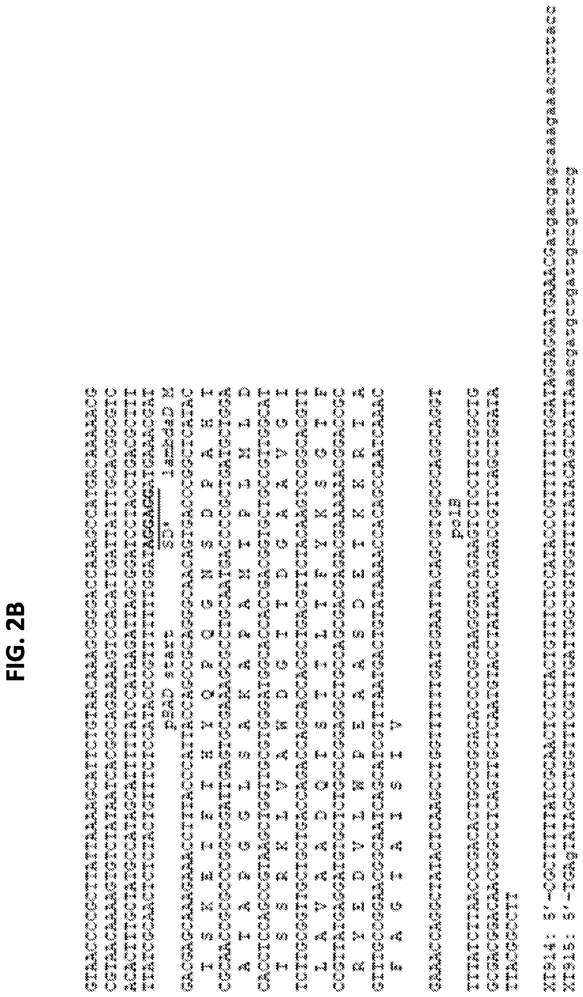

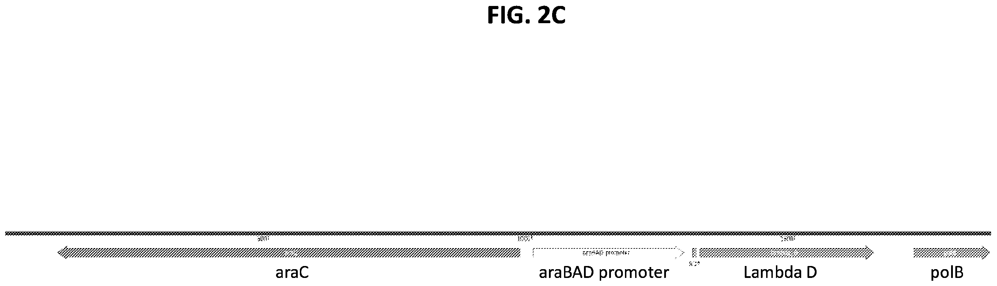

**FIGS. 1A-1C****. Construction of A D gene controlled by the arabinose promoter *P_{BAD}.* (A)** The wild-type arabinose operon *araC araBAD* with the *polB* gene of DNA Pol II downstream. SD indicates the Shine-Dalgarno ribosome binding site. **(B)** Insertion of *tet-sacB bla* cassette downstream of *araA* to replace part of *araD,* which is described Li et al., Nucl. Acids Res., 41: e204 doi:10.1093/nar/gkt1075, 2013. **(C)** Replacement of SD *araB, araA, cat, tetA, bla* and remainder of *araD* with the **λ** D gene and an optimized SD* ribosome binding site. The sequence of construct (C) is described in FIG. 2.
**FIG. 2A-2B****. Sequence of A D gene controlled by the arabinose promoter *P_{BAD}.*** The two black arrows indicate *araC* (SEQ ID NO: 60) and *lambdaD* (SEQ ID NO: 1). The primers XT914 (SEQ ID NO: 65) and XT915 (SEQ ID NO: 66) were used to PCR amplify the **λ***D* gene cassette from phage lambda DNA with flanking homologies to precisely replace the three *araBAD* structural genes by recombineering. The sequence of wild type D (SEQ ID NO: 1) is flanked by the sequences of the bacterial chromosome. Upstream of D is the promoter region of araBAD operon and the downstream of D is the sequence of *polB* gene. The full length nucleotide sequence is SEQ ID NO: 57. Within this sequence, the *araBAD* sequence is AAGAAACCAATTGTCCATATTGCATCAGACATTGCCGTCACTGCGTCTTTTACTGGCTC TTCTCGCTAACCCAACCGGTAACCCCGCTTATTAAAAGCATTCTGTAACAAAGCGGGA CCAAAGCCATGACAAAAACGCGTAACAAAAGTGTCTATAATCACGGCAGAAAAGTCC ACATTGATTATTTGCACGGCGTCACACTTTGCTATGCCATAGCATTTTTATCCATAAGA TTAGCGGATCCTACCTGACGCTTTTTATCGCAACTCTCTACTGTTTCTCCAT (SEQ ID NO: 58), the Shine Dalgarno sequence (bold and underlined) exists between the *araBAD* promoter and the start codon of the lambda D protein, shown as ACCCGTTTTTTTGGAT**AGGAGGA**TGAAACG (SEQ ID NO: 59). The *araC* amino acid sequence is SEQ ID NO: 60 and the *polB* amino acid sequence is SEQ ID NO: 61.
**FIG. 2C****. Plasmid map of the** **FIG. 2A** **sequence.**

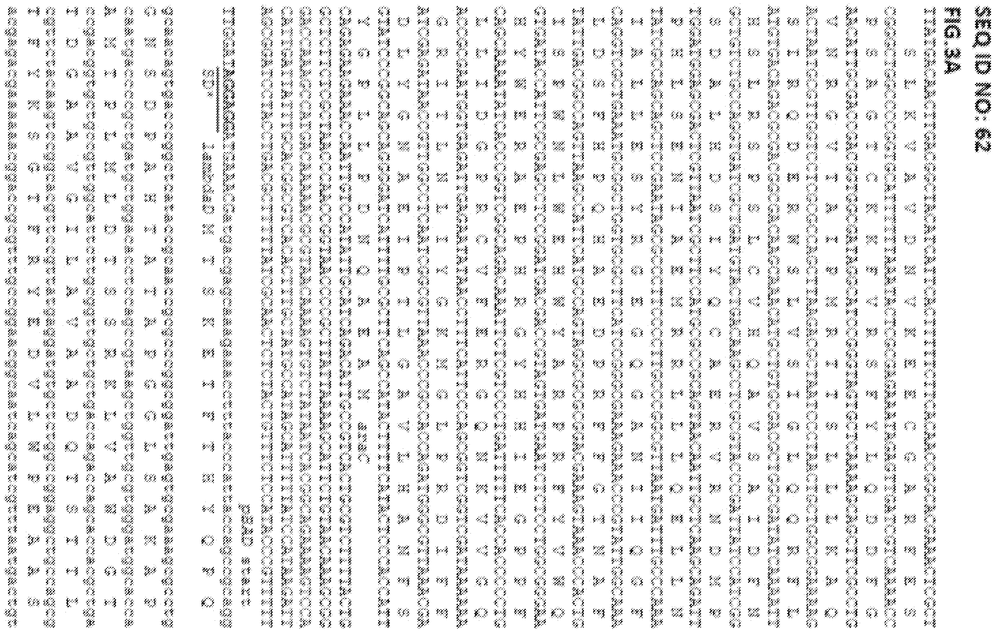

**FIG. 3A-3C****. Sequence of plasmid pLXT42 (SEQ ID NO: 62) containing the λ D gene controlled by the arabinose promoter *P_{BAD}.*** The replication origin (SEQ ID NO: 63) and ampicillin resistance gene (SEQ ID NO: 64) were PCR amplified from pBR322 using primers XT1021 (SEQ ID NO: 67) and XT1022 (SEQ ID NO: 68). These primers also carried homology segments on each side of the arabinose operon controlling λ *D.* This linear plasmid segment with homology flanking the *ara* operon retrieved the *araC* λ *D* genes from the chromosome by recombineering to generate plasmid pLXT42, which expresses high levels of D protein in the presence of arabinose.

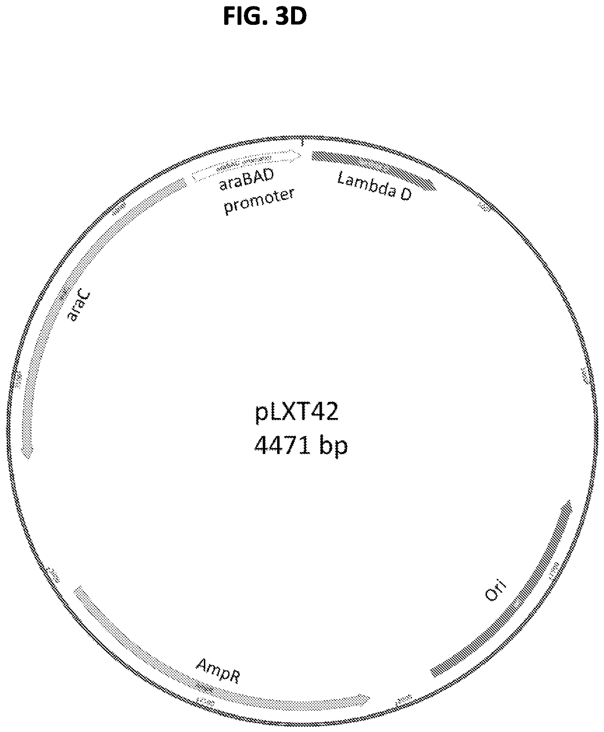

**FIG. 3D****. Plasmid map of the** **FIG. 3A** **sequence.**

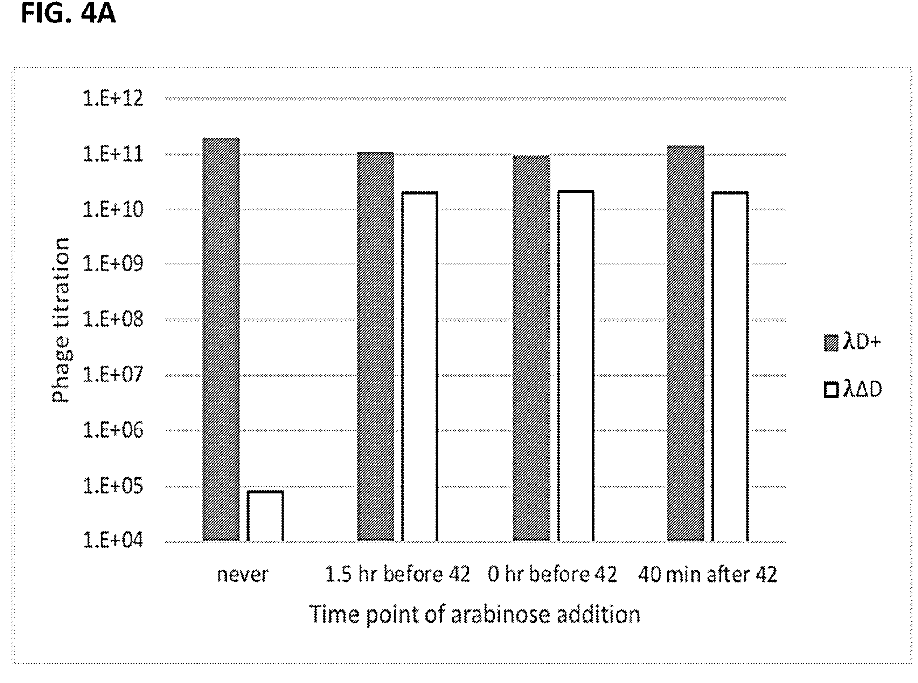

**FIGS. 4A-4B****. (A)** Temporal complementation of λD function provided at different times from the multi-copy plasmid pLXT42 relative to the temperature induction of the λΔ*D* prophage. (**B**) Temporal complementation of λD function is provided by adding arabinose to induce λD protein expression from the arabinose operon on the chromosome at the times indicated following temperature induction of the λ*cI857 Sam7* λΔ*D* prophage in strain XTL1205. Each column represents a time point. Spacing between time points is arbitrary and not proportional to the time shown.

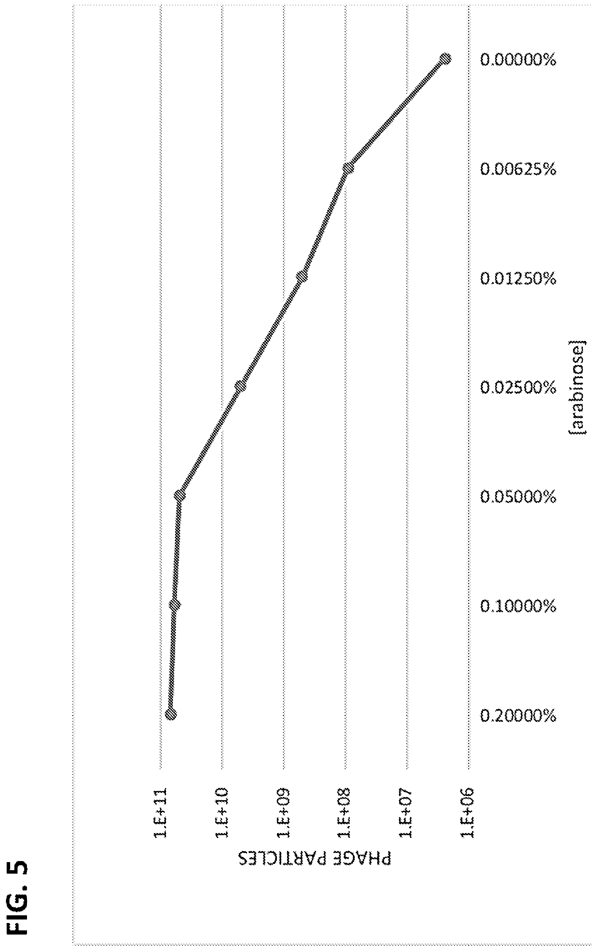

**FIG. 5****.** λD protein complementation by strain XTL1219 carrying the pLXT42 plasmid at decreasing concentrations of arabinose to generate decreasing levels of λD protein expression.

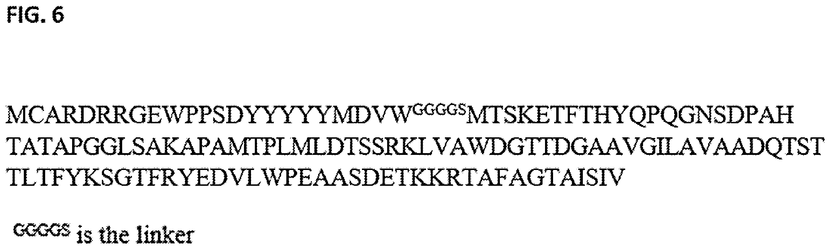

**FIG. 6****.** Amino acid sequence (SEQ ID NO: 18) of the D protein fused to CLL CDR3 antigen at the N-terminus via a linker peptide, which is shown in superscript.

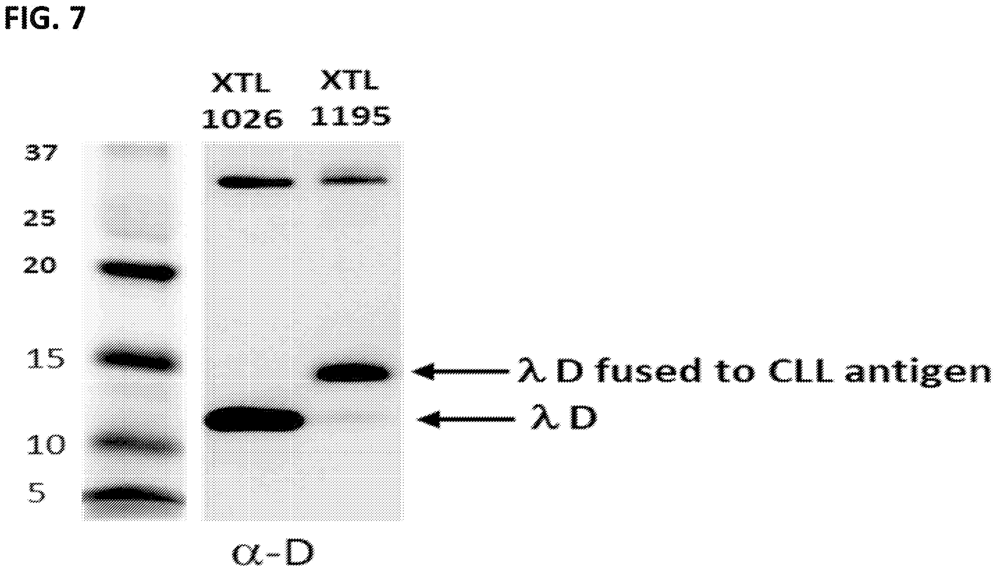

**FIG. 7****.** Comparison of protein mobility between XTL-1026 (λD⁺ non-fusion vector) and XTL-1195 (λD fusion) by western blot with anti-D antibody protein. After phage induction equal volume of phage lysate from each sample were ran in a 4-20% SDS gradient gel under reducing condition and probed with anti-D antibody after its transfer to a nitrocellulose membrane. XTL1026 sample shows the expression of stable D protein and XTL1195 shows the expression of stable λD-fusion product with reduced mobility. Molecular mass of lD is ~11.6kDa. Protein mobility marker is shown in the far left lane.

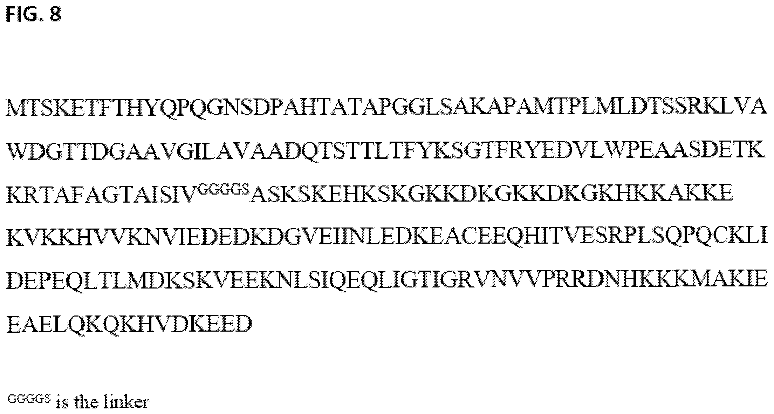

**FIG. 8****.** Amino acid sequence (SEQ ID NO: 20) of C-terminal segment of pfGARP antigen fused to the C-terminal end of the D protein via a linker, which is shown in superscript.

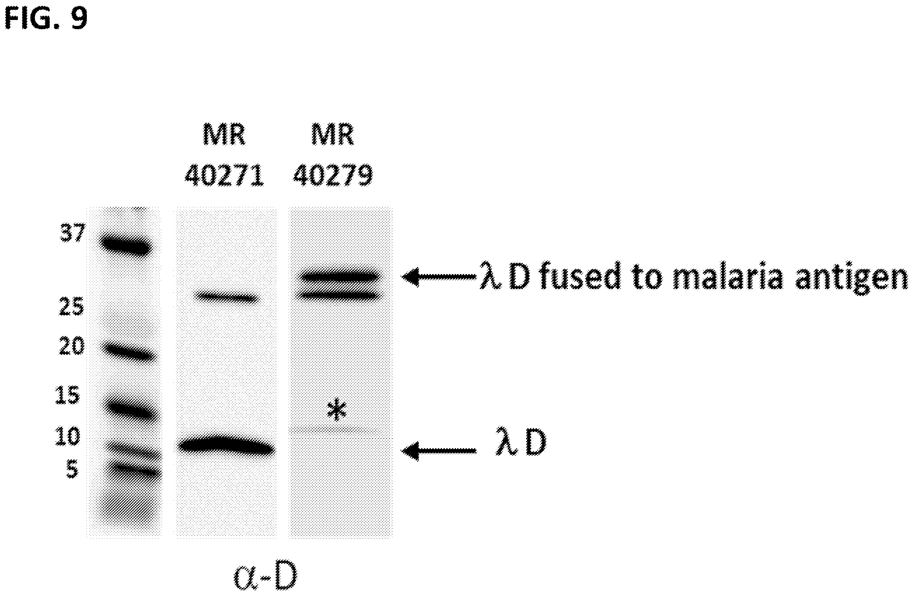

**FIG. 9****.** Comparison of protein mobility between MR40271 (λD⁺ non-fusion vector without a copy of λD in the arabinose locus) and MR40279 (λD fused to malaria antigen) by western blot with anti-D antibody protein. After phage induction equal volume of phage lysate from each sample were run in a 4-20% SDS gradient gel under reducing condition and probed with anti-D antibody after its transfer to a nitrocellulose membrane. Wild-type D protein of a molecular mass of ~11.6kDa is detected in a sample MR40271. The fusion protein product of ~28.5kDa molecular mass is detected in a sample MR40279 right above the cross-reacting background band. The slight degradation of the fusion product marked by "*" is observed in the sample, mostly observed when wild-type copy of D protein is not present to stabilize the phage head. Protein mobility marker is shown in the far left lane.

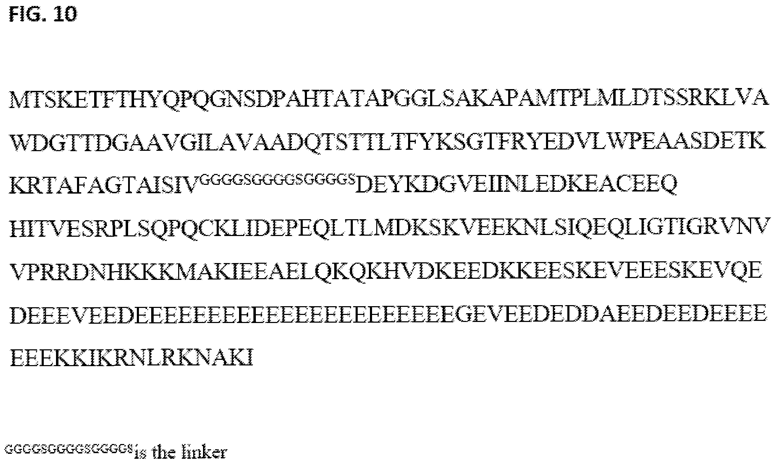

**FIG. 10****.** Amino acid sequence (SEQ ID NO: 20) of C-terminal segment of pfGARP antigen fused to the C-terminal end of the D protein via a linker, which is shown in superscript.

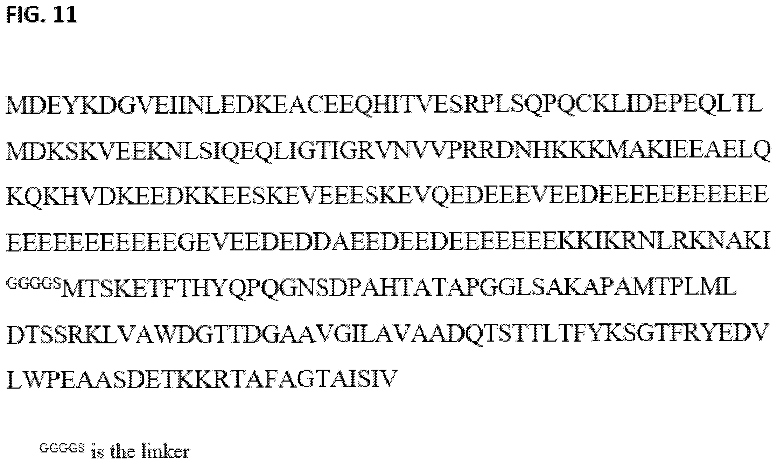

**FIG. 11****.** Amino acid sequence (SEQ ID NO: 21) of the C-terminal segment of pfGARP antigen fused to the N-terminal end of the D protein in strain XTL1107 via a linker shown in superscript.

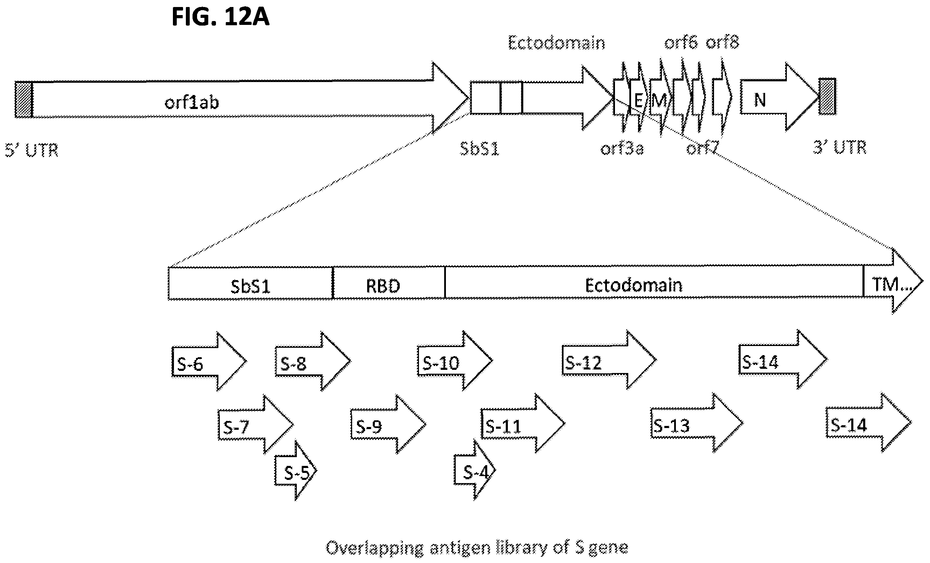

**FIGS. 12A-12C****. (A)** The schematic shows the genetic map of the SARS-Cov-2 virus, highlighting various domains of the spike (*S*) gene. The viral S gene was subdivided into partially overlapping segments each being engineered to display on the lambda phage vector as a D-fusion. **(B)** The peptide sequences in the table represent potential antigenic regions displayed as D-fusions on phage capsid from viral protein ORF3a (ORF3a-1), nucleoprotein (N-1 and N-2), envelope protein (E-16), and various overlapping segments of spike protein (*e.g.*, S-4, S-5, ...S-15). The corresponding length of each peptide displayed on lambda D is listed in the table. SEQ ID NOs: 4 and 22-35 are shown. The arbitrary antigen number for a displaying phage strain in parenthesis is shown on the left column. **(C)** Western blots with fusion phages carrying different subclones of the *S, N,* and *E* genes against anti-D antibody. Shown are numbers of phage constructs with corresponding antigen number at the top of the lanes showing the mobility of the corresponding fusion protein. Equal volume of protein samples taken from crude lysates generated from prophage induction were ran in a 4-20% SDS gradient gel under reducing condition and probed with anti-D antibody after its transfer to a nitrocellulose membrane. λD fusion products with reduced mobility is observed among samples indicated at the top compared to wild-type non-displaying XTL1026. Molecular mass of λD is ~11.6kDa. Protein mobility marker is shown in the far left of each gel.

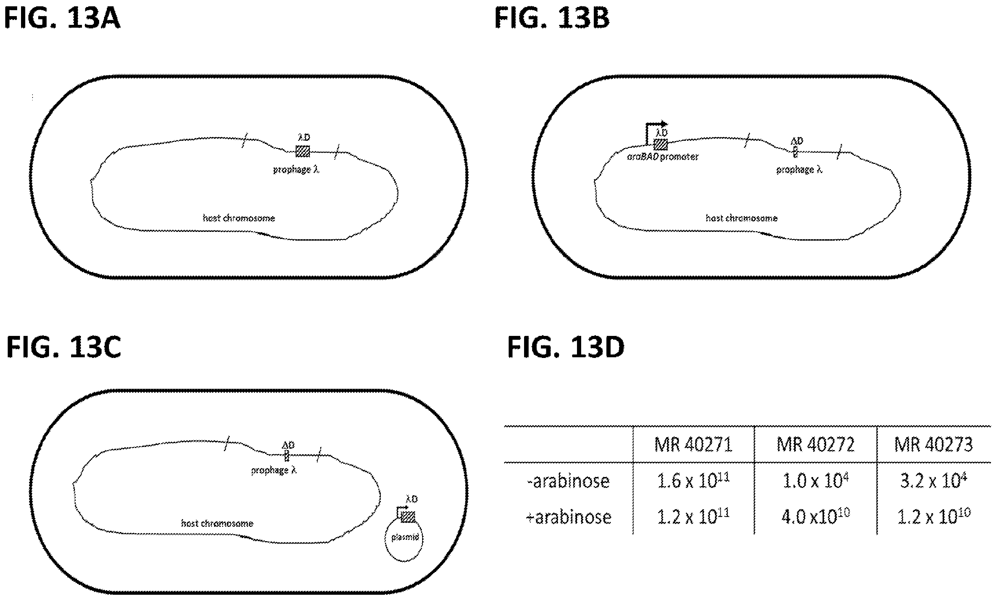

**FIGS. 13A-13E****. (A)** λ lysogen carrying *D*⁺ gene in its native location in the prophage. (**B**) Prophage λ with the *D* gene deleted (Δ*D*) and with the cell containing a *D*⁺gene under arabinose control at the *ara* locus of the bacterial chromosome. **(C)** Prophage λ with the D gene deleted (Δ*D*) and with the cell containing a *D*⁺ gene in a plasmid under the *ara* promoter control. **(D)** Phage titers in plaque forming units per ml (pfu/ml) after prophage induction with wild-type *D* from its native locus (column 2; MR 40271), from the *ara* locus (column3; MR 40272), or from the plasmid (column 4; MR 40273) in the absence (row 2) or presence (row 3) of arabinose. (**E**) Western blot results showing amounts of D proteins using anti-D antibody in the phage lysates corresponding to each genetic context in FIG. 13D. The measurement of phage yield and the corresponding expression of λD in the presence of arabinose in strain MR 40271 is demonstrated as a control.

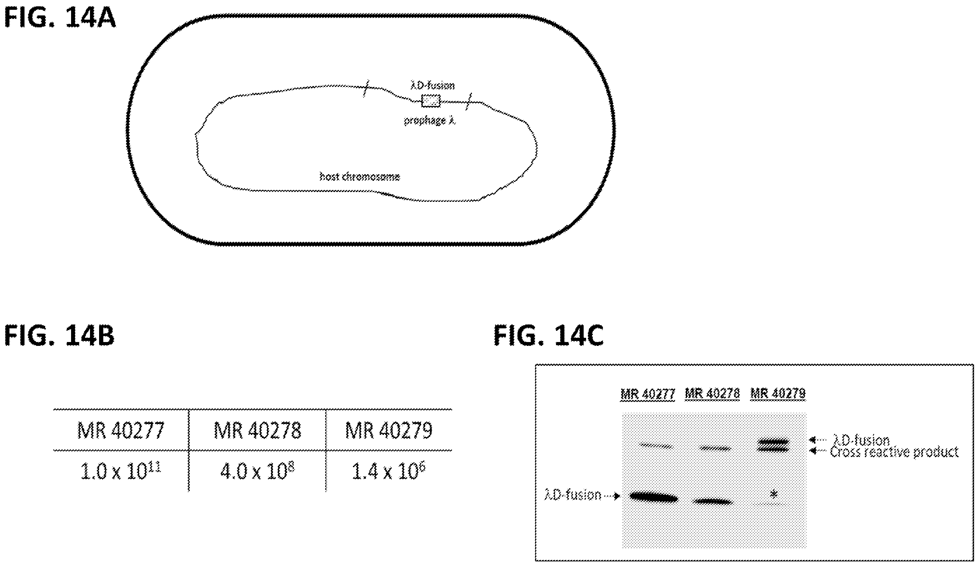

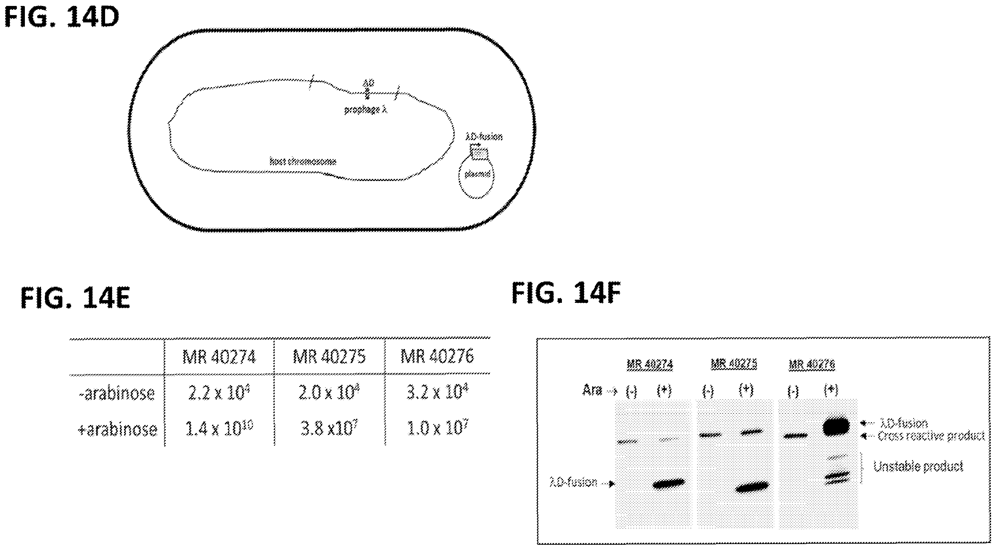

**FIGS. 14A-14F****. (A)** A prophage λ carrying a *D*-fusion gene in the native D-locus. **(B)** Phage titers in pfu/ml after prophage induction of the D-fusion lysogens (column 1: D-COVID fusion, column 2: D-CDR3 fusion, column 3: D-pfGARP fusion). **(C)** Western blot results showing amounts of D-fusion proteins using anti-D antibody in the phage lysates corresponding to columns 1 to 3 in FIG. 14B. "*" symbol in lane 3 represents the degradation product of the 143 aa fused D protein observed right above an unknown cross-reactive product seen in all lanes. **(D)** Prophage λ with the D gene deleted (Δ*D*) in the native phage locus and a *D*-fusion gene placed under the *ara* promoter in a plasmid. **(E)** Phage titers in pfu/ml after prophage induction with different D-fusions from the plasmid (D-covid fusion in strain MR 40274, D-CDR3 fusion in strain MR 40275, and D-pfGARP fusion in strain MR 40276) in the absence (row 2) or presence (row 3) of arabinose. **(F)** Western blot results showing amounts of D-fusion proteins using anti-D antibody in the phage lysates corresponding to each strain in FIG. 14E. The unknown cross-reactive product and the potential degradation of the D-fusion products are indicated in the far left of the gel images.

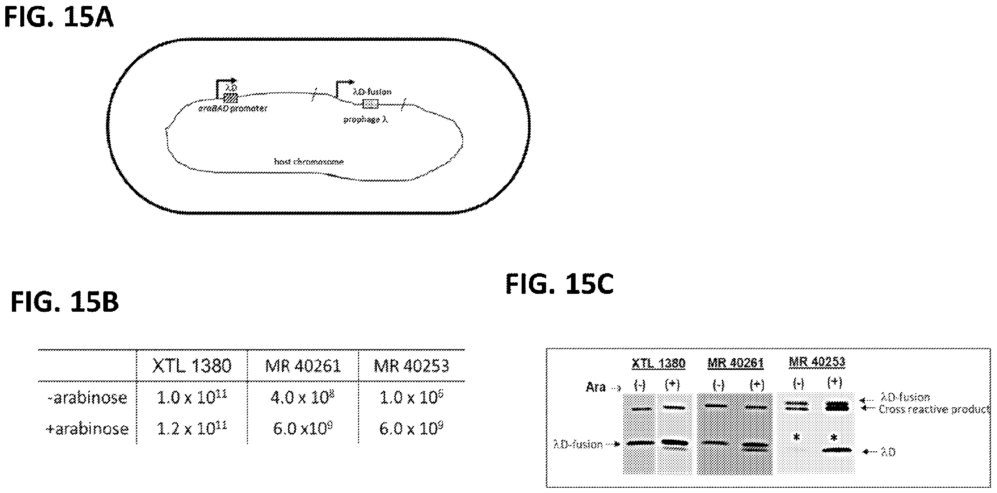

**FIGS. 15A-15C****. (A)** λ lysogens containing different *D*-fusion genes in the native prophage *D*-locus. The chromosome also contains an ectopic λ *D*⁺gene under *ara* promoter control. **(B)** Phage titers in pfu/ml after prophage induction with different D-fusions in the native locus with or without the supplement of wild-type D from the ara locus. Columns 2, 3, and 4 shows the phage titer of native locus D-covid fusion in strain XTL 1380, D-CDR3 fusion in strain MR 40261, and D-pfGARP fusion in strain MR 40253 in the absence (row 2) or presence (row 3) of arabinose. **(C)** Western blot results showing amounts of D-fusion proteins using anti-D antibody in the phage lysates corresponding to each strain in FIG. 15B. The expression of wild-type D in the presence of arabinose along with D-fusion products as judged by their relative mobility are observed in lane 2, 4, and 6. D-fusion products, wild-type D, and cross-reactive product recognized by anti-D antibody are indicated by arrows. The potential degradation product of D-pfGARP fusion is marked by the "*" in the gel, approximately the size of the native λ D protein.

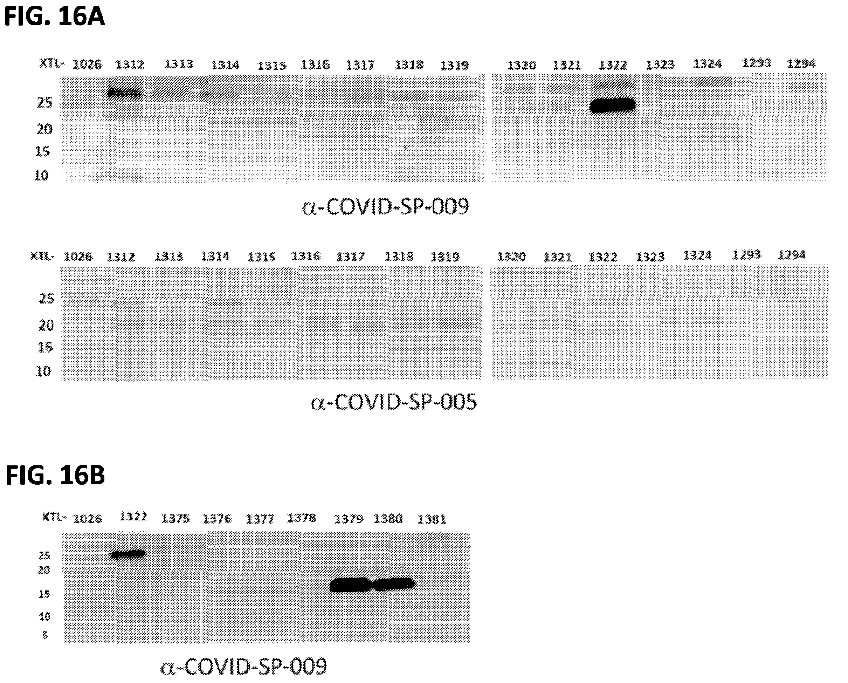

**FIGS. 16A and 16B****.** Epitope mapping of the spike protein using COVID-positive human serum. A library of phage strains, labeled at the top of FIG. 16A, displaying different peptide segments covering the ectodomain of the Wuhan-1 spike protein was run in an SDS-PAGE and transferred into a cellulose membrane for western blot. When all strains were probed with patient serum (COVID-SP-009) for the presence of antibodies, only a 133 aa long fragment overlapping the heptad-repeat segment of spike protein that was displayed in the strain XTL-1322 was detected. XTL-1322 did not react with the serum of a healthy donor (COVID-SP-005). For phage stability, the 133 aa fragment was further dissected into smaller peptide fragments generating a sub-library of phage strains, labeled at the top of FIG. 16B. Upon further probing of this sub-library with the same covid serum (COVID-SP-009), the epitope region was narrowed down into an 11 amino acid overlapping peptide fragment displayed by strain XTL-1379 and XTL-1380.

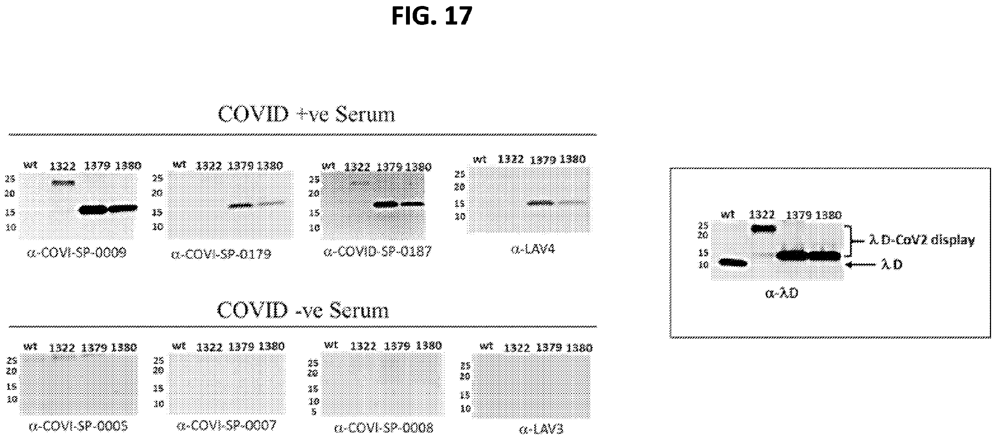

**FIG. 17****.** λ capsid displaying overlapping peptides of SARS-CoV-2 spike protein is recognized by the serum antibody produced among COVID positives only. Shown on the top panel is the Western blot of non-displaying phage protein (wt) and three separate peptide displaying candidates (1322, 1379, and 1380) probed with four separate COVID positive serums indicated in the bottom. The bottom panel is the Western blot result using four separate COVID negative serums indicated at the bottom. Shown in the inset is the corresponding Western blot using an antibody specific to λ displaying coat protein, D. Displaying candidates 1322, 1379, and 1380 are 137, 37, and 27 amino acids longer compared to the wild type λD, respectively. A molecular marker is shown to the left of each blot.

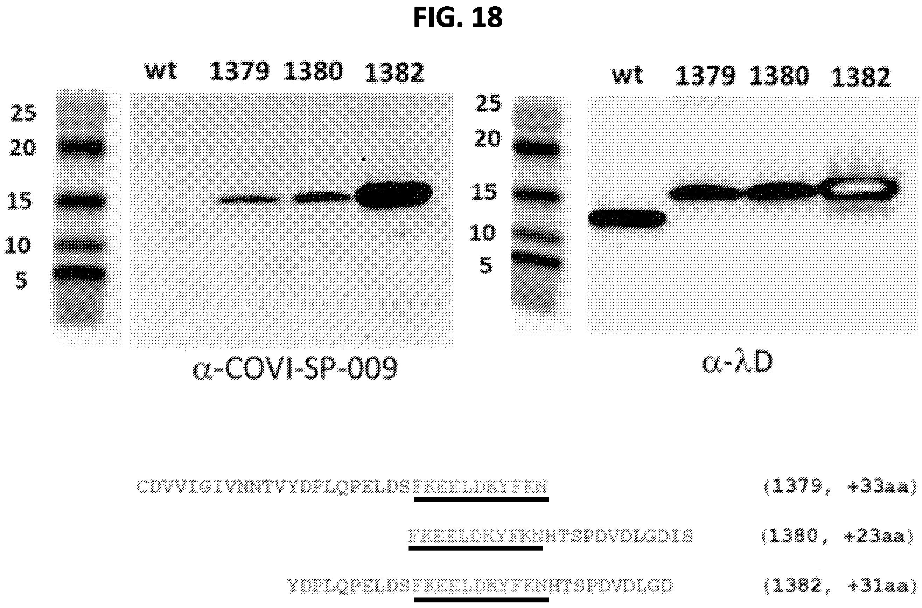

**FIG. 18****.** Construction of a high-affinity epitope displaying phage. The overlapping peptide displayed by strain XTL-1379 and XTL-1380, as shown the bottom panel, were both at the edge of the displayed peptide fragment. A strain displaying a hybrid peptide between the two, covering both flanking sides of the overlapping region, was generated. The Western blot in the top panel indicates that the hybrid epitope displayed by the strain XTL-1382 has a high affinity for the serum antibody compared to the epitopes displayed by XTL-1379 and XTL-1380. The amino acid sequences of the COVID-19 (SARS CoV-2) 1379 (SEQ ID NO: 44), 1380 (SEQ ID NO: 35) and 1382 (SEQ ID NO: 47) antigens are shown.

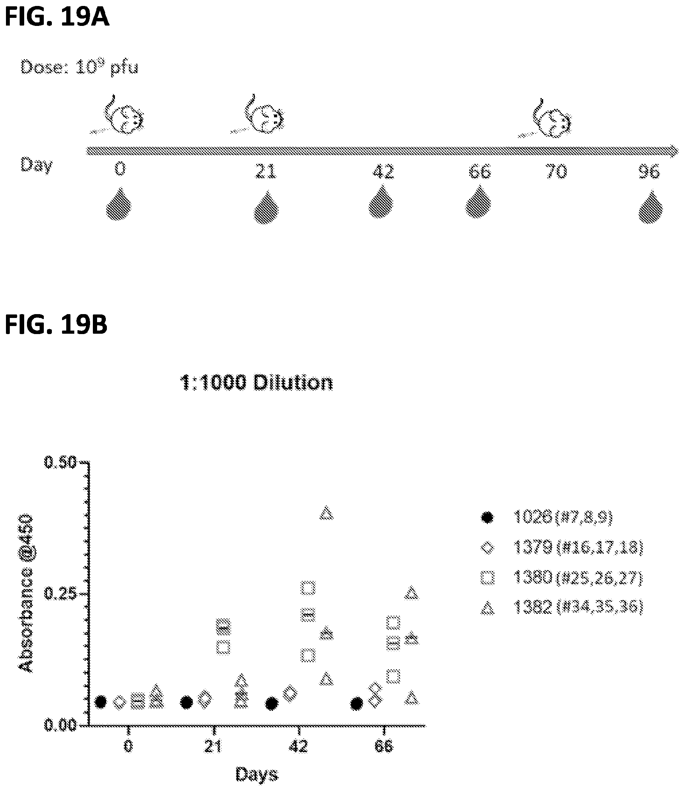

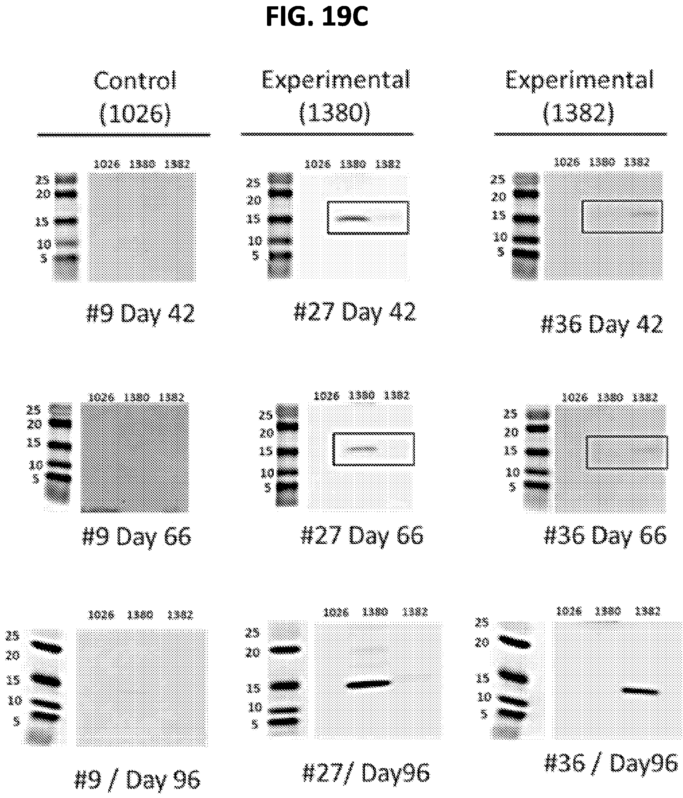

**FIGS. 19A-19C****.** Phage vaccine triggers the generation of antibody specific to the displayed spike epitope. (**A)** Scheme of vaccination and sample collection. A set of 3 BALB/c female mice were intraperitoneally injected with 10⁹ pfu phage particles per dose followed by a second dose in the third week and then boosted on day 70. Blood samples were collected on days 0, 21, 42, 66, 70, and 96. **(B)** Serum were assessed by ELISA for epitope-specific binding antibodies. Compared to the control group, only mice injected with phage strain XTL-1380 and XTL-1382 showed a response of antibody generation. The mice injected with strain XTL-1379 had no response. (**C**) Serum of a single mouse from each test group of 1026, 1380, and 1382 was further analyzed by Western blot for the presence of epitope specific antibody. Following booster dose on day 70, a robust antibody accumulation specific to the injected epitope was observed from the blood sample of day 96. A slight cross reactivity between the 1380 and 1382 epitope was observed in the Western blot, due to the overlapping peptide sequence.

### SEQUENCES

The sequences listed in the accompanying sequence listing are shown using standard letter abbreviations for nucleotide bases and amino acids as defined in 37 C.F.R. 1.822. Only one strand of each nucleic acid sequence is shown, but the complementary strand is understood as included by any reference to the displayed strand.

SEQ ID NO: 1 is an amino acid sequence of an exemplary wild-type bacteriophage λ D protein.

SEQ ID NO: 2 is the amino acid sequence of an exemplary linker.

SEQ ID NO: 3 is the amino acid sequence of an exemplary wild-type bacteriophage λ E protein.

SEQ ID NO: 4 is the amino acid sequence of an exemplary SARS-CoV-2 antigen.

SEQ ID NO: 5 is the amino acid sequence of an exemplary *Plasmodium* antigen.

SEQ ID NO: 6 is the amino acid sequence of an exemplary chronic lymphocytic leukemia antigen.

SEQ ID NOs: 7-17 are oligonucleotide sequences.

SEQ ID NO: 18 is the amino acid sequence of a D protein fused to CLL CDR3 antigen via a linker peptide. See FIG. 6.

SEQ ID NO: 19 is the amino acid sequence of a D protein fused at its C-terminus to the antigenic segment of pfGARP via a linker peptide. See FIG. 8.

SEQ ID NO: 20 is the amino acid sequence of a C-terminal segment of pfGARP antigen fused to the C-terminal end of a D protein via a linker in strain XTL1106. See FIG. 10.

SEQ ID NO: 21 is the amino acid sequence of a C-terminal segment of pfGARP antigen fused to the N-terminal end of the D protein via a linker in strain XTL1107. See FIG. 11.

SEQ ID NOs: 22-35 are additional antigen sequences.

SEQ ID NO: 36 is the amino acid sequence of an exemplary side tail fiber (stf) protein.

SEQ ID NO: 37 is the amino acid sequence of an exemplary tail tube (gpV) protein.

SEQ ID NO: 38 is a 23 amino acid peptide from the Spike protein of SARS CoV-2 virus.

SEQ ID NOs: 39-52 are the amino acid sequences of additional SARS CoV-2 antigens.

SEQ ID NOs: 53-54 are epithelial growth factor receptor version III (EGFRvIII) antigens.

SEQ ID NO: 57 is the nucleic acid sequence of the plasmid shown in FIG. 2A.

SEQ ID NO: 58 is the *araBAD* nucleic acid sequence in the plasmid of FIG. 2A.

SEQ ID NO: 59 is a nucleic acid sequence including the Shine Dalgarno sequence, a portion of the *araBAD* promoter, and the start codon of the lambda D protein.

SEQ ID NO: 60 is the amino acid sequence of *araC.*

SEQ ID NO: 61 is the amino acid sequence of *polB.*

SEQ ID NO: 62 is the nucleic acid sequence of plasmid pLXT42.

SEQ ID NO: 63 is the nucleic acid sequence of the replication origin.

SEQ ID NO: 64 is the nucleic acid sequence of the ampicillin resistance gene.

SEQ ID NOs: 65-68 are the nucleic acid sequence of primers.

SEQ ID NO: 69 is the amino acid sequence of an additional SARS CoV-2 antigen.

### DETAILED DESCRIPTION OF SEVERAL EMBODIMENTS

Bacteriophage λ can be used to display foreign proteins on the surface of the phage particle. The lambda particle contains two major structural components - the phage capsid or head, which encapsulates the genome, and the phage tail, which contains proteins for binding to the bacterial receptor (LamB) and triggering genome injection into the cell.

The bacteriophage λ head (capsid) contains two major structural proteins, E and D, each present in 420 copies per phage particle (Georgopoulos et al. In: Lambda II. Hendrix et al. (editors), Cold Spring Harbor, NY: Cold Spring Harbor Laboratory; 1983. pp. 279-304.). In the lambda genome, the *D* and *E* genes are adjacent and expressed coordinately. The D protein stabilizes the primary E capsid structure. As disclosed herein, functional bacteriophage λ particles have been produced in which the D protein has been joined to other proteins such that the fusion protein is displayed on the surface of the λ capsid. Previously, the D-fusion protein (D-fusion) was generated by expressing the D-fusion from a plasmid construct in the cell during infection by a λ mutant lacking D protein. The D-fusion protein made *in trans* then assembles with the other phage components. For example, U.S. Patent No. 7,410,801 discloses bacteriophage λ that carries heterologous protein fusions to the D protein for bio-panning. In this patent, the *D* gene-fusion segment was cloned into the non-essential *b* segment of the phage genome and expressed from a *lac* promoter. Thus, the D gene fusion is not present in the native D location, and the D fusion protein is produced independently of its normal expression next to gene *E.*

In contrast, the *D* gene fusions described herein are created *in vivo* on a λ prophage where the *D* gene is in its normal position adjacent to gene *E.* Thus, the D fusion protein is expressed coordinately with the major E protein for phage capsid morphogenesis. The lambda prophage was engineered in several ways to simplify subsequent construction of the D-antigen fusion on the phage, increase the number of fused proteins made per phage, to accommodate a large size fusion protein, simplify induction and maximize phage yield from an engineered bacterial host cells, purify the phage, to increase phage vaccine yields, and enhance the *in vivo* circulation of the antigen-displaying particles during vaccination in mammalian hosts.

Bacteriophage λ is a temperate phage that infects *E. coli,* and can take one of two developmental pathways after infection. One path is the vegetative or lytic path whereupon λ usurps the host machinery to express functions required to replicate its genome, to generate structural proteins that encase the genome and to form the mature phage particles. During this process, λ eventually expresses functions that lead to cell lysis and release of particles into the environment. The second path is lysogenic development following infection. In this path, the phage, upon infection, expresses functions that enable the integration of the phage DNA into the bacterial genome and to produce a repressor protein, called CI (C one) that represses the expression of the specific genes that enable the phage to follow the lytic pathway. The integrated phage (or prophage) is maintained in a quiescent state by continued CI repression of these genes and replicates along with the bacterial genome. This quiescent or lysogenic state can be reversed by inactivation of CI, which leads to the expression of genes to excise the λ genome from the bacterial chromosome and start the lytic cycle leading to phage production and cell lysis. This process is called induction. A classical λ mutation in the *cI* gene is normally used to induce λ from the prophage state. This is the *cI857* mutation that causes the CI repressor to become inactive at elevated temperatures (optimally 42°). Thus, λ *cI857* lysogenic cultures growing at 32° are induced to produce phage particles and lyse the cell by simply shifting the culture temperature to 42°.

In wild-type hosts, the time from lambda prophage temperature induction to subsequent lysis and phage release is about 50 minutes and lysis releases about 100 particles per cell. Lysis is dependent upon two phage proteins: the *R* gene product is an endolysin, and the *S* gene product is a holin. The S-holin generates micron-scale holes in the cytoplasmic membrane at about 50 min., which inactivates cellular proton motive force and any ongoing macromolecular syntheses. The holes also allow the soluble endolysin to escape from the cytoplasm and degrade the peptidoglycan outer layer causing complete cell lysis. If the S-holin protein is inactivated, then cytoplasmic endolysin cannot escape to cause lysis, macromolecular synthesis persists, and bacteriophage λ continues to replicate and can ultimately produce about 1,000 particles per cell because of the increased time for phage genome replication.

### Summary of Terms

Unless otherwise noted, technical terms are used according to conventional usage. Definitions of many common terms in molecular biology may be found in Krebs et al. (eds.), Lewin's genes XII, published by Jones & Bartlett Learning, 2017. As used herein, the singular forms "a," "an," and "the," refer to both the singular as well as plural, unless the context clearly indicates otherwise. For example, the term "an antigen" includes singular or plural antigens and can be considered equivalent to the phrase "at least one antigen." As used herein, the term "comprises" means "includes." The term "about" means within five percent, unless context suggests otherwise. It is further to be understood that any and all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described herein. In case of conflict, the present specification, including explanations of terms, will control. In addition, the materials, methods, and examples are illustrative only and not intended to be limiting. To facilitate review of the various embodiments, the following explanations of terms are provided:
**Attachment site (att):** A specific site for recombination that occurs on either a phage or a chromosome. An attachment site on lambda is termed "attP," while an attachment site of a bacterial chromosome is "attB." Integrase mediated recombination of an attP site with an attB site leads to integration of the λ prophage in the bacterial chromosome.
**Bacteriophage** or **Phage:** Bacteriophage are obligate intracellular parasites that multiply inside bacteria by making use of some or all of the host biosynthetic machinery. Though different phages may contain different materials, they all contain nucleic acid and protein, and may be covered by a lipid membrane. A bacteriophage genome typically consists of a single, linear or circular, double- or single-stranded nucleic acid. Depending on the phage, the nucleic acid can be DNA or RNA. Many bacteriophage range in size from 24-200 nm in diameter. Bacteriophage, such as **λ**, contain a capsid head, which is composed of many copies of one or more different proteins.

The native or wild-type genome of λ is 48,502 base pairs in length, and is located in the capsid head. The **b region** in the wild-type lambda genome is flanked upstream by the gene orf-194, which encodes a fiber assembly protein, and downstream by the gene int. The b region includes three non-essential genes, *ea47, ea31* and *ea59.* In wild-type bacteriophage λ, the nucleotide coordinates comprising *ea47, ea 31* and *ea59* is from base pair 22689 to 26973. The S-holin is encoded by lambda *S* gene and generates micron-scale holes in the cytoplasmic membrane at about 50 min., which inactivates cellular proton motive force and any ongoing macromolecular syntheses. The holes also allow the soluble endolysin to escape from the cytoplasm and degrade the peptidoglycan outer layer causing complete cell lysis. If the - *S* gene is genetically inactivated, such as by including a nonsense mutation, missense mutation or a deletion, the S-holin protein is inactivated, then cytoplasmic endolysin cannot escape to cause lysis, macromolecular synthesis persists, and bacteriophage λ continues to replicate and can ultimately produces about 1,000 particles per cell. Sam7 is a nonsense mutation in the *S* gene that results in a prematurely truncated S protein that is defective for producing holes in the cytoplasmic membrane in host cells.

The bacteriophage λ head contains two major structural proteins, **E and D,** each present in 420 copies per phage particle (Georgopoulos et al. In: Lambda II. Hendrix et al. (editors), Cold Spring Harbor, NY: Cold Spring Harbor Laboratory; 1983. pp. 279-304,). In the lambda genome, the *D* and *E* genes are adjacent and expressed coordinately. The D protein stabilizes the primary E capsid structure.

Many phages, including λ, have tails attached to the capsid head. An exemplary amino acid sequence for the D capsid protein is shown in SEQ ID NO: 1, and an exemplary amino acid sequence for the E capsid protein is shown in SEQ ID NO: 3.

The tail is a hollow tube through which the nucleic acid passes during infection and after adsorption to the bacterial cell surface. The tail tube of lambda is encoded by gene *V.* At the end of the tail tube, lambda has a tail tip protein called J, which binds the LamB receptor for adsorption on the *E. coli* cell surface. Lambda also has side tail fibers (encoded by gene *stf*) attached to the end of the tail tube. The side tail fibers (Stf) in addition to protein J is involved in the adsorption of the phage to the host cell by an unknown mechanism. An amino acid sequences for an exemplary Stf protein and V protein are provided in SEQ ID NO: 36 and SEQ ID NO: 37, respectively.

Bacteriophage λ carries the Red recombination system (Beta, Exo, and Gam). These phage systems can work with the bacterial recombination functions or independent of them.

A "naturally-occurring" or "**wild-type**" bacteriophage is a phage isolated from a natural or human-made environment that has not been modified by genetic engineering. A "**recombinant bacteriophage**" is a phage that contains a genome genetically modified by insertion of a heterologous (*e.g*., non-native) nucleic acid sequence into the genome.

A "**lytic**" or virulent bacteriophage can only multiply on bacteria and kill the cell by lysis at the end of the life cycle. Lytic phage, in some embodiments, can be enumerated by a plaque assay. A plaque is a clear area that results in a lawn of bacterial grown on a solid media from the lysis of bacteria. The assay can be performed at a low enough concentration of phage that each plaque arises from a single infectious phage. The infectious particle that gives rise to a plaque is referred to as a PFU (plaque forming unit). A "**lysogenic**" **or temperate** bacteriophage can either multiply through the lytic cycle or enter a quiescent state in the cell. In this quiescent state, most of the phage genes are not transcribed; the phage genome exists in a repressed state. The phage DNA in this repressed state is referred to as a prophage because it has the potential to produce phage. In most cases, the phage DNA integrates into the host chromosome and is replicated along with the host chromosome and passed on to the daughter cells. The cell harboring a prophage is not adversely affected by the presence of the prophage, and the lysogenic state may persist indefinitely. The cell harboring a prophage is referred to as a lysogen.

A prophage is the latent state of a phage in a lysogenic bacterium. "Induction" is the process that converts a prophage into a phage.

**Beta:** The 28 kDa lambda Beta ssDNA binding polypeptide (and nucleic acid encoding lambda beta) involved in double-strand break repair homologous recombination. DNA encoding Beta (*bet*) and polypeptide chains having lambda Beta activity are also referred to herein as *bet.* The lambda Beta protein binds to single-stranded DNA and promotes renaturation of complementary single-strand regions of DNA (see also Karakousis et al., J. Mol. Biol. 276:721-733, 1998; Li et al., J. Mol. Biol. 276:721-733, 1998; Passy et al., PNAS 96:4279-4284, 1999).

**Conservative variants:** "Conservative" amino acid substitutions are those substitutions that do not substantially affect or decrease an activity or antigenicity of the polypeptide, such as a D protein. Specific, non-limiting examples of a conservative substitution include the following examples:

| | |
|---|---|
| Original | Conservative Substitutions |

| | |
|---|---|
| Ala | Ser |
| Arg | Lys |
| Asn | Gln, His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

The term conservative variant also includes the use of a substituted amino acid in place of an unsubstituted parent amino acid, provided that antibodies raised to the substituted polypeptide also immunoreact with the unsubstituted polypeptide, or that a protein retains its function, such that a conservative variant of D protein that can still form the head of bacteriophage λ.

**Contacting:** The process of incubating one agent in the presence of another. Thus, when a bacterial cell is incubated with the agent, such as a bacteriophage λ, under appropriate conditions (such as density, temperature, and medium) for a sufficient period of time, the bacterial cell is "contacted" with the agent. With regard to a bacteriophage and a bacterial host, adsorption of the phage to the host is a result of this contact. The adsorption of phage to a host can lead to injection and infection, but may not lead to infection. A bacteriophage that "targets" a specific bacterial host, such as an *E. coli,* can adsorb to the host when the host is contacted with the bacteriophage.

**Degenerate variant:** A polynucleotide, such as a polynucleotide encoding a D protein, that includes a sequence that is degenerate as a result of the genetic code. There are 20 natural amino acids, most of which are specified by more than one codon. Therefore, all degenerate nucleotide sequences are included in this disclosure as long as the amino acid sequence of the protein encoded by the nucleotide sequence is unchanged.

**De-repressible promoter:** When a repressor is bound to a de-repressible promoter, transcription is substantially decreased as compared to transcription from the de-repressible promoter in the absence of the repressor. By regulating the binding of the repressor, such as by changing the environment, the repressor is released from the de-repressible promoter and transcription increases.

One specific, non-limiting example of a de-repressible promoter is the P_{L} promoter, which is regulated by the repressor *cI.* P_{L} is not activated by an activator, and thus is not an inducible promoter. An "activatable promoter" is a promoter wherein binding of an activator to the promoter increases transcription from the promoter. The arabinose promoter, pBAD is not a simple de-repressible promoter; arabinose inactivates the repressor AraC and converts it to an activator. Thus, pBAD is an activatable promoter.

In one embodiment, the de-repressible promoter is a temperature sensitive de-repressible promoter. A temperature sensitive de-repressible promoter is a promoter that is de-repressed only at a specified temperature, or range of temperatures. In one embodiment, by increasing the temperature, the repressor is released from the promoter, or can no longer bind to the promoter with a high affinity, and transcription is increased from the promoter. One specific, non-limiting example is the induction of P_{L} promoter activity by increasing the temperature of the cell using cI87. Increased temperature inactivates the temperature-sensitive repressor *cI*, allowing genes that are operably linked to the P_{L} promoter to be expressed at increased levels.

In one embodiment, a de-repressible promoter is auto-regulated. One specific, non-limiting example of an auto-regulated de-repressible promoter is P_{L}. If only one copy of a gene encoding cI is present, yet many copies of the P_{L} promoter are present, expression of cI is upregulated such that transcription is blocked from any of the P_{L} promoters.

**Effective amount:** The "effective amount" of a composition is the quantity of a composition sufficient to achieve a desired result. For instance, this can be the amount of a composition containing a sufficient dose of one or more bacteriophage to induce an immune response to a heterologous antigen when administered to a subject. The effective amount of a composition can depend on, for example, enzymatic activity of the composition, the amount of the bacteriophage contained in the composition, the concentration of the bacteriophage in the composition, etc.

**Epidermal Growth Factor Receptor (EGFR):** EGFR (also known as HER1 or ERBB1) is a receptor belonging to the ERBB family of the receptor thymidine kinases (RTKs). *In vivo*, ligand-binding by EGF results in the activation of the RTK/RAS/PI(3)K pathway via receptor phosphorylation, and results in cellular proliferation, angiogenesis, and increased local tissue invasion as well as resistance to apoptosis. A nucleic acid sequence for human EGFR can be found at GENBANK^{®} Accession No. NM_005228.5, June 18, 2019, and GENBANK^{®} Accession No. NC_000007.14 (EGFR in the chromosome), June 14, 2019. EGFR has an external domain (ECD) of 621 amino acids, a single pass transmembrane domain (TM) of 23 amino acids and an enzymatically active intracellular domain (ICD) of 542 amino acids. Ligand binding leads to receptor dimer formation and the activation of the kinase domain which signals to one of several pathways that can promote the growth, survival and spread of mammalian cells. In tumors, the loss of exons 2-7 to produces EGFR variant III (EGFRvIII), which is constitutively active. A cDNA sequence for EGFRvIII can be found at GENBANK^{®} Accession No. NM_001346941, June 18, 2019, and an amino acid sequence can be found at NP_001333870.1, June 14, 2019, Activation of wild-type EGFR involves dimerization which requires ligand binding and a monomer to dimer transition with attendant changes in receptor conformation. Activating mutations can occur in either the ECD or the ICD. The loss of exon 19 to generate a constitutively active enzyme mutant. EGFR can be overexpressed either by gene amplification of less of transcriptional control. High level expression (such as greater than about 50,000 receptors per cell) leads to either misfolding of the receptor or mutations in one of more of the gene copies. Overexpression can result in a two-fold or greater increase in EGFR present in the cell, as compared to a wild-type control. One structural element that is sterically unavailable under normal conditions is the 287-302 (numbering of mature receptor - or 301-326 of full-length receptor) disulfide-limited loop. This loop is exposed in EGFRvIII and may become exposed when receptor expression is very high or when ECD mutations alter wild type structure.

**Epitope:** An antigenic determinant. These are particular chemical groups or peptide sequences on a molecule that are antigenic, *i.e.,* that elicit a specific immune response generating antibody. An antibody binds a particular antigenic epitope. Generally, an enzyme reduces the activation energy of a specific biochemical reaction.

**Exo:** The exonuclease of bacteriophage λ (and the nucleic acid encoding the exonuclease protein) involved in double-strand break repair homologous recombination. See Example 1 and references therein for further description.

**Exogenous:** The term "exogenous" as used herein with reference to nucleic acid and a particular cell refers to any nucleic acid that does not originate from that particular cell as found in nature. Thus, a non-naturally-occurring nucleic acid is considered to be exogenous to a cell once introduced into the cell. Nucleic acid that is naturally occurring also can be exogenous to a particular cell. For example, an entire chromosome isolated from a cell of subject X is an exogenous nucleic acid with respect to a cell of subject Y once that chromosome is introduced into Y's cell.

**Extrachromosomal:** Not incorporated into the chromosome or chromosomes of a cell. In the context of nucleic acids, extrachromosomal indicates a DNA oligonucleotide that is not covalently incorporated into the chromosome or chromosomes of a cell. **Intrachromosomal** refers to material such as an oligonucleotide that is incorporated into the chromosome or chromosomes of a cell, such as a DNA oligonucleotide covalently incorporated into the chromosomal DNA of a cell.

**Flanking:** In the sequence "A-B-A", nucleic acid sequence "A" flanks nucleic acid sequence "B." In one specific, non-limiting example, nucleic acid sequence "A" is located immediately adjacent to nucleic acid "B." In another specific, non-limiting example, a linker sequence of not more than 500 nucleotides is between each copy of "A" and "B," such as a linker sequences of about 200, about 100, about 50, or about 10 nucleotides in length. Nucleotide sequences "A" and "B" can be of any length. "Adjacent" refers to a first nucleic acid sequence next to a second amino acid sequence. Thus, in the sequence A-B-C, A is 5' to B and adjacent to B. However, A is 5' to C but is not adjacent to C. B is 3' of A and 5' of C; B is adjacent to both A and C and is flanked by A and C.

**Functionally Equivalent:** Sequence alterations, such as in a D protein, that yield the same function as described herein. Such sequence alterations can include, but are not limited to, conservative substitutions, deletions, substitutions, and insertions (or combinations thereof) that do not affect the function of the encoded polypeptide, for example so that it is incorporated into the head of the bacteriophage.

**Gam:** A bacteriophage λ protein (and nucleic acid encoding Gam) involved in double-strand break repair homologous recombination. It is believed to inhibit cellular nuclease activity such as that encoded by the recBCD and sbcC system of *E. coli.* Gam function, when expressed in the cell, is extremely toxic to the cell, and prevents growth. Thus, tight controls over its expression can be required. As described herein, P_{L} and cI 857 regulate Gam expression.

**Gene:** A nucleic acid encoding a protein product. In a specific non-limiting example, a gene includes at least one expression control sequence, such as a promoter, enhancer or a repressor. In another specific, non-limiting example, a gene includes at least one intron and at least one exon. "**Genetic inactivation**" is the inclusion of a mutation (such as a deletion, insertion, nonsense or missense mutation) in a gene, such that the encoded protein loses its activity.

**Heterologous:** Of different natural origin, such that inclusion together is a non-natural state. For example, if a host cell is transformed with a gene or protein coding sequence derived from another organism, particularly from another species, that genetic information is heterologous with respect to both the host cell and descendants of the host cell that carry the gene. Similarly, "heterologous" refers to a nucleotide sequence from one species, or a synthetic nucleotide sequence, that is inserted into a nucleic acid molecule from another species. A heterologous nucleic acid sequence can be inserted into a genome at different location that the wild-type genome, so that it is under the control of different regulatory elements.

**Host cell:** A cell used to receive exogenous nucleic acid molecules. In one embodiment a host cell is used to maintain or allow the reproduction of a vector, or to facilitate the manipulation of nucleic acid molecules *in vitro.* A host cell can be a prokaryotic or a eukaryotic cell. In one embodiment, a host cell is an *E. coli* cell. **LamB** is a protein that is the cell surface receptor for bacteriophage λ on *E. coli.* The active form on the LamB protein is a homotrimer that contains nonspecific channels that allow passive diffusion of hydrophilic molecules with molecular weights of less than 600 kD across the outer membrane. The mature LamB protein includes 421 amino acids. In some embodiments, a host cell can be resistant to bacteriophage λ due to one or more missense mutations in the gene encoding LamB, see Werts et al., J. Bacteriol. 176: 941-7, 1994, Disruption of LamB can also be caused by a deletion. **FhuA** is an outer membrane protein of *E. coli.* The structure of this protein is a β-barrel composed of 22 antiparallel β-strands into which a globular domain is incorporated that closes the channel of the β-barrel. Deletions can be made in FhuA to reduce its activity, see Endrib and Braun, J. Bacteriol. 186: 4818-4823, 2004,

**Immune response:** A response of a cell of the immune system, such as a B cell or T cell to a stimulus. In one embodiment, the response is specific for a particular antigen (an "antigen-specific response"). A "parameter of an immune response" is any particular measurable aspect of an immune response, including, but not limited to, cytokine secretion (IL-6, IL-10, IFNy, etc.), immunoglobulin production, dendritic cell maturation, and proliferation of a cell of the immune system. One of skill in the art can readily determine an increase in any one of these parameters, using known laboratory assays. In one specific non-limiting example, to assess cell proliferation, incorporation of ³H-thymidine can be assessed. A "substantial" increase in a parameter of the immune response is a significant increase in this parameter as compared to a control. Specific, non-limiting examples of a substantial increase are at least about a 50% increase, at least about a 75% increase, at least about a 90% increase, at least about a 100% increase, at least about a 200% increase, at least about a 300% increase, and at least about a 500% increase. One of skill in the art can readily identify a significant increase using known statistical methods.

**Immunogenic composition:** A composition comprising an antigen, such as a λ expressing a heterologous antigen, that induces an immune response, such as a measurable CTL response against the antigen, or a measurable B cell response (such as production of antibodies) against the antigen. As such, an immunogenic composition includes one or more antigens (for example, polypeptide antigens) or antigenic epitopes, such as on the head and/or tail of λ. An immunogenic composition can also include one or more additional components capable of eliciting or enhancing an immune response, such as an excipient, carrier, and/or adjuvant. In certain instances, immunogenic compositions are administered to elicit an immune response that protects the subject against symptoms or conditions induced by a pathogen. In one example, an "immunogenic composition" includes a bacteriophage λ that induces a measurable CTL response and/or induces a measurable B cell response (such as production of antibodies) against the heterologous antigen. For *in vivo* use, the immunogenic composition typically includes the bacteriophage λ in pharmaceutically acceptable carriers, and/or other agents. Immunogenic compositions can include adjuvants. However, in some embodiments, an immunogenic composition does not include an adjuvant. In this case lambda phage itself can act as an adjuvant.

**Infectious agent:** An agent that can infect a subject, including, but not limited to, viruses, bacteria, nematodes, protozoa, and fungi. In some examples, the heterologous antigen is from, or the immune response is directed against, an infectious virus, such as : *Retroviridae*; *Picornaviridae* (for example, polio viruses, hepatitis A virus; enteroviruses, human coxsackie viruses, rhinoviruses, echoviruses); *Calciviridae* (such as strains that cause gastroenteritis); *Togaviridae* (for example, equine encephalitis viruses, rubella viruses); *Flaviridae* (for example, dengue viruses, encephalitis viruses, yellow fever viruses); *Coronaviridae* (for example, coronaviruses, such as severe acute respiratory syndrome coronavirus (SARS-CoV), SARS-CoV-2, Middle East respiratory syndrome coronavirus (MERS-CoV), human coronavirus HKU1 (HKU1-CoV), human coronavirus OC43 (OC43-CoV), human coronavirus 229E (229E-CoV), human coronavirus NL63 (NL63-CoV), as well as variants thereof such as variants of SARS-CoV-2 which include alpha (B.1.1.7 and Q lineages); beta (B.1.351 and descendent lineages); delta (B.1.617.2 and AY lineages); gamma (P.1 and descendent lineages); epsilon (B.1.427 and B.1.429); eta (B.1.525); iota (B.1.526); kappa (B.1.617.1); 1.617.3; mu (B.1.621, B.1.621.1) zeta (P.2)), and omicron (such as original lineage: B.1.1.529 and lineages: BA.2, BA.4, BA.5, BQ.1, BQ.1.1, BA.4.6, and BF.7); *Rhabdoviridae* (for example, vesicular stomatitis viruses, rabies viruses); *Filoviridae* (for example, ebola viruses); *Paramyxoviridae* (for example, parainfluenza viruses, mumps virus, measles virus, respiratory syncytial virus); *Orthomyxoviridae* (for example, influenza viruses); *Bungaviridae* (for example, Hantaan viruses, bunga viruses, phleboviruses and Nairo viruses); *Arena viridae* (hemorrhagic fever viruses); *Reoviridae* (*e.g.*, reoviruses, orbiviurses and rotaviruses); *Birnaviridae*; *Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and HSV-2, varicella zoster virus, cytomegalovirus (CMV), herpes viruses); *Poxviridae* (variola viruses, vaccinia viruses, pox viruses); and *Iridoviridae* (such as African swine fever virus); and unclassified viruses (for example, the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e., Hepatitis C); Norwalk and related viruses, Zika virus, and astroviruses).

In some examples, the heterologous antigen is from, or the immune response is directed against, a positive-strand RNA virus, such as Picornaviruses (such as Aphthoviridae [for example foot-and-mouth-disease virus (FMDV)]), Cardioviridae; Enteroviridae (such as Coxsackie viruses, Echoviruses, Enteroviruses, and Polioviruses); Rhinoviridae (Rhinoviruses)); Hepataviridae (Hepatitis A viruses); Togaviruses (examples of which include rubella; alphaviruses (such as Western equine encephalitis virus, Eastern equine encephalitis virus, and Venezuelan equine encephalitis virus)); Flaviviruses (examples of which include Dengue virus, West Nile virus, and Japanese encephalitis virus); Calciviridae (which includes Norovirus and Sapovirus); and Coronaviruses (examples of which include SARS coronaviruses, such as the SARS-CoV-2). In some examples, the heterologous antigen is from, or the immune response is directed against, a negative-strand RNA virus, such as an orthomyxyovirus (such as the influenza virus), Rhabdovirus (such as Rabies virus), and Paramyxovirus (examples of which include measles virus, respiratory syncytial virus, and parainfluenza viruses). In some examples, the heterologous antigen is from, or the immune response is directed against, a DNA virus, such as herpesviruses (such as Varicella-zoster virus, for example the Oka strain; cytomegalovirus; and herpes simplex virus (HSV) types 1 and 2), adenovirus (such as adenovirus type 1 and adenovirus type 41), poxvirus (such as vaccinia virus), and parvovirus (such as Parvovirus B19). In some examples, the heterologous antigen is from, or the immune response is directed against, a retrovirus, such as human immunodeficiency virus type 1 (HIV-1), such as subtype C; HIV-2; equine infectious anemia virus; feline immunodeficiency virus (FIV); feline leukemia viruses (FeLV); simian immunodeficiency virus (SIV); and avian sarcoma virus.

In some examples, the heterologous antigen is from, or the immune response is directed against, an infectious bacteria, such as: *Helicobacter pyloris, Borelia burgdorferi, Legionella pneumophilia, Mycobacteria sps* (such as. *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes* (Group A *Streptococcus*), *Streptococcus agalactiae* (Group B *Streptococcus*), *Streptococcus* (*viridans* group), *Streptococcus faecalis, Streptococcus bovis, Streptococcus (anaerobic sps.), Streptococcus pneumoniae, pathogenic Campylobacter sp., Enterococcus sp., Haemophilus influenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli.*

In some examples, the heterologous antigen is from, or the immune response is directed against, gram-negative bacteria, such as *Escherichia coli* (*e.g.,* K-12 and O157:H7), *Shigella dysenteriae, and Vibrio cholerae.* In some examples, the heterologous antigen is from, or the immune response is directed against, gram-positive bacteria, such as *Bacillus anthracis*, *Staphylococcus aureus*, *Listeria*, pneumococcus, gonococcus, and streptococcal meningitis.

In some examples, the heterologous antigen is from, or the immune response is directed against, an infectious fungi, such as, *Cryptococcus neoformans, Histoplasma capsulatum, Coccidioides immitis, Blastomyces dermatitidis, Chlamydia trachomatis, Candida albicans.*

In some examples, the heterologous antigen is from, or the immune response is directed against, an infectious organism (such as protists), for example: *Plasmodium* (*e.g.*, *Plasmodium falciparum), Leishmania, Acanthamoeba, Giardia, Entamoeba, Cryptosporidium, Isospora, Balantidium, Trichomonas, Trypanosoma (e.g., Trypanosoma brucei*)*, Naegleria,* or *Toxoplasma* (*e.g., Toxoplasma gondii).*

**Inducible promoter:** A promoter whose activity can be increased by the binding of an activator to the promoter. Examples of inducible promoters abound in nature, and a broad range of environmental or hormonal changes may activate or repress them. One specific example of an inducible promoter is pBAD.

**Initiation site for replication:** The site on a nucleic acid sequence wherein DNA replication starts. For example, a bacterial origin of replication is the site on the bacterial DNA wherein DNA replication starts. For example, an initiation site can be a ColE1 initiation site (Tomizawa et al., PNAS 74:1865-69, 1077) or an *E. coli* or *B. subtilis* oriC (see Seitz et al., EMBO Reports 2:1003-1006, 2001).

**Intron:** An intragenic nucleic acid sequence in eukaryotes that is not expressed in a mature RNA molecule. Introns of the present disclosure include full-length intron sequences, or a portion thereof, such as a part of a full-length intron sequence.

**Isolated:** An "isolated" biological component (such as a nucleic acid, peptide, protein, or bacteriophage particles) has been substantially separated, produced apart from, or purified away from other biological components in the cell of the organism in which the component naturally occurs, *e.g.*, other chromosomal and extrachromosomal DNA and RNA, and proteins. Nucleic acids, peptides and proteins which have been "isolated" thus include nucleic acids and proteins purified by standard purification methods. The term also embraces nucleic acids, peptides and proteins prepared by recombinant expression in a host cell as well as chemically synthesized nucleic acids, proteins, and peptides. An "isolated" particle has been substantially separated, produced apart from, or purified away from the environment from which it was produced, such as the reagents and/or reaction.

**Linker sequence:** An amino acid sequence that covalently links two polypeptide domains. Linker sequences can be included in a fusion protein, such as between a heterologous antigen and D-protein. In some embodiments, a linker sequence provides rotational freedom to the linked polypeptide domains and thereby to promote proper domain folding. Linker sequences, which are generally between 2 and 25 amino acids in length, include, but are not limited to, the glycine(4)-serine spacer GGGGS (SEQ ID NO: 3) described by Chaudhary et al., Nature 339:394-397, 1989.

**Mammal:** This term includes both human and non-human mammals. Similarly, the term "patient" or "subject" includes both human and veterinary subjects, such as primates, rats, mice, cats, dogs, sheep, horses, cows, goats, and pigs.

**Nucleic acid molecule or sequence:** A polymer composed of nucleotide units (ribonucleotides, deoxyribonucleotides, related naturally occurring structural variants, and synthetic non-naturally occurring analogs thereof) linked via phosphodiester bonds, related naturally occurring structural variants, and synthetic non-naturally occurring analogs thereof. Thus, the term includes nucleotide polymers in which the nucleotides and the linkages between them include non-naturally occurring synthetic analogs, such as, for example and without limitation, phosphorothioates, phosphoramidates, methyl phosphonates, chiral-methyl phosphonates, 2-O-methyl ribonucleotides, peptide-nucleic acids (PNAs), and the like. Such polynucleotides can be synthesized, for example, using an automated DNA synthesizer. It will be understood that when a nucleotide sequence is represented by a DNA sequence (*i.e.*, A, T, G, C), this also includes an RNA sequence (*i.e.*, A, U, G, C) in which "U" replaces "T."

"Nucleotide" includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide.

Conventional notation is used herein to describe nucleotide sequences: the left-hand end of a single-stranded nucleotide sequence is the 5'-end; the left-hand direction of a double-stranded nucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand;" sequences on the DNA strand having the same sequence as an mRNA transcribed from that DNA and which are located 5' to the 5'-end of the RNA transcript are referred to as "upstream sequences;" sequences on the DNA strand having the same sequence as the RNA and which are 3' to the 3' end of the coding RNA transcript are referred to as "downstream sequences."

"cDNA" refers to a DNA that is complementary or identical to an mRNA, in either single stranded or double stranded form.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (for example, rRNA, tRNA and mRNA) or a defined sequence of amino acids and the biological properties resulting therefrom. Thus, a gene encodes a protein if transcription and translation of mRNA produced by that gene produces the protein in a cell or other biological system. Both the coding strand, the nucleotide sequence of which is identical to the mRNA sequence and is usually provided in sequence listings, and non-coding strand, used as the template for transcription, of a gene or cDNA can be referred to as encoding the protein or other product of that gene or cDNA. Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns. In some examples, a nucleic acid encodes a disclosed antigen.

**Oligonucleotide (oligo):** A single-stranded nucleic acid ranging in length from 2 to about 500 bases, for example, polynucleotides that contain at least 20 or 40 nucleotides (nt). Oligonucleotides are often synthetic but can also be produced from naturally occurring polynucleotides.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein-coding regions, in the same reading frame.

**Origin of replication (*ori*):** A nucleotide sequence at which DNA synthesis for the purpose of replicating the nucleic acid sequence begins. This is generally termed an "*ori*" site. Circular bacterial chromosomes generally have a single *ori* site, whereas there are many *ori* sites on each eukaryotic chromosome. This term includes replicons, which as used herein refers to a genetic element that behaves as an autonomous unit during DNA replication. In bacteria, the chromosome functions as a single replicon, whereas eukaryotic chromosomes contain hundreds of replicons in series.

The *ori* site of plasmids can allow replication in one or more bacterial species, such as a gram negative or a gram-positive species. For example, an *ori* can allow replication in one or more of *E. coli,* Yersinia, or *Salmonella.* Specific, non-limiting examples of an *ori* are a ColEl origin and its derivatives, a pSC101 origin and its derivatives, a pBBR1 origin and its derivatives, and a RK2 origin and its derivatives. In one specific example, a ColE1 origin of replication is described in Tomizawa et al., Proc. Natl. Acad. Sci. (PNAS) 74:1865-69, 1977 (such as -420 to -613 base pairs (upstream) of the initiation site of ColE1 replication).

**Operator sequence:** A specific nucleic acid sequence capable of interacting with a specific repressor, thereby controlling the function of genes in adjacent cistrons and regulator genes. In general, a regulator gene is a gene whose primary function is to control the rate of synthesis of the products of other distant genes. The regulator gene controls the synthesis of a protein repressor, which inhibits the action of an operator gene and thus turns off the operon it controls. The repressor usually is present in small amounts. It may possess two sites, one of which can attach to the operator and one of which can bind an effector molecule. In one embodiment, once a repressor is bound to an effector molecule, the repressor changes shape and cannot attach to the operator. In another embodiment (such as for λ cI857) heat itself can inactivate or denature the intact repressor so that it does not attach to the operator. An operon is a unit of nucleic acid sequence consisting of one or more cistrons that function coordinately under the control of an operator sequence.

Thus, the repressor is a protein, synthesized by a regulator gene, that binds to an operator locus and blocks transcription of that operon. The repressor causes repression of transcription. When de-repressed, transcription and/or translation are increased.

**Phage-based recombination systems:** Bacteria such as *E. coli* encode their own homologous recombination systems, which are used in repair of DNA damage and to maintain a functional chromosome. The viruses or phages that inhabit bacteria often carry their own recombination functions.

**Pharmaceutically acceptable carriers:** The pharmaceutically acceptable carriers useful with the bacteriophage, polypeptides and nucleic acids described herein are conventional. Remington's Pharmaceutical Sciences, by E. W. Martin, Mack Publishing Co., Easton, PA, 15th Edition (1975), describes compositions and formulations suitable for pharmaceutical delivery of the bacteriophage λ, cells, and fusion proteins herein disclosed.

In general, the nature of the carrier can depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g.,* powder, pill, tablet, or capsule forms), conventional non-toxic solid carriers can include, for example, pharmaceutical grades of mannitol, lactose, starch, or magnesium stearate. In addition to biologically-neutral carriers, immunogenic compositions to be administered can contain minor amounts of non-toxic auxiliary substances, such as wetting or emulsifying agents, preservatives, and pH buffering agents and the like, for example sodium acetate or sorbitan monolaurate.

**Plasmid:** A construction of genetic material designed to direct transformation of a targeted cell, such as a host cell. Plasmids include a construction of extrachromosomal genetic material, usually of a circular duplex of DNA which can replicate independently of chromosomal DNA. A plasmid generally contains multiple genetic elements positional and sequentially oriented with other necessary genetic elements such that the nucleic acid in a nucleic acid cassette can be transcribed and when necessary translated in the transfected cells. Plasmids include nucleic acids that are DNA derived from a plasmid vector, cosmids, or phagemids wherein one or more fragments of nucleic acid may be inserted or cloned which encode for particular genes of interest. The plasmid can have a linear or circular configuration.

Plasmids generally contain one or more unique restriction sites. In addition, a plasmid can confer some well-defined phenotype on the host organism which is either selectable or readily detected. Thus, the plasmid can include an expression cassette, wherein a polypeptide is encoded. Expression includes the efficient transcription of an inserted gene, nucleic acid sequence, or nucleic acid cassette with the plasmid. Expression products can be proteins, polypeptides or RNA.

In one embodiment, when a circular plasmid is transferred into a bacterial cell, it can be an autonomously replicating, extra-chromosomal DNA molecule, distinct from the normal bacterial genome and nonessential for bacterial cell survival under nonselective conditions. The term "persistent expression" as used herein refers to introduction of genes into the cell together with genetic elements which enable episomal (extra-chromosomal) replication and/or maintenance of the genetic material in the cell. This can lead to apparently stable transformation of the cell without the integration of the novel genetic material into the chromosome of the host cell.

A plasmid can also introduce genetic material into chromosomes of the targeted cell where it integrates and becomes a permanent component of the genetic material in that cell. Gene expression after stable introduction can permanently alter the characteristics of the cell and its progeny arising by replication leading to stable transformation.

**Polynucleotide:** A double-stranded or single-stranded nucleic acid sequence of any length. Therefore, a polynucleotide includes molecules which are at least 15, at least 50, at least 100, at least 200, at least 500, at least 1000, or at least 5000 nucleotides long (oligonucleotides), as well as a full length cDNA, mRNA, or gene sequence.

**Polypeptide:** A polymer in which the monomers are amino acid residues that are joined together through amide bonds. When the amino acids are alpha-amino acids, either the L-optical isomer or the D-optical isomer can be used. The terms "polypeptide" or "protein" as used herein are intended to encompass any amino acid sequence and include modified sequences such as glycoproteins. A polypeptide includes both naturally occurring proteins, as well as those that are recombinantly or synthetically produced. A polypeptide has an amino terminal (N-terminal) end and a carboxy-terminal end.

**Preventing or treating a disease:** "Preventing" a disease refers to inhibiting the full development of a disease, for example in a person who is known to have a predisposition to a disease such as infection by a pathogen or a cancer. An example of a person with a known predisposition is someone with a history of a tumor in the family, or who has been exposed to factors that predispose the subject to a condition, such as a tumor. "Treatment" refers to a therapeutic intervention that ameliorates a sign or symptom of a disease or pathological condition after it has begun to develop. Treatment can include producing an immune response. Treatment refers to a reduction in size of a tumor, a decrease in the number and/or size of metastases, or a decrease in a symptom of the tumor. Treatment refers to a reduction in a symptom or titer in a subject with an infection, or a reduction in the titer of an infectious agent.

Reducing a sign or symptom of a disease or pathological condition related to a disease, refers to any observable beneficial effect of the treatment. In a non-limiting example, reducing a sign or symptom associated with a tumor (such as pathological angiogenesis) can be evidenced, for example, by a delayed onset of clinical symptoms of the disease in a susceptible subject (such as a subject having a tumor which has not yet metastasized,), a reduction in severity of some or all clinical symptoms of the disease, a slower progression of the disease (for example by prolonging the life of a subject having tumor), a reduction in the number of relapses of the disease, an improvement in the overall health or well-being of the subject, or by other parameters well known in the art that are specific to the particular tumor. Similarly, for an infection, symptoms can be delayed or serious consequences avoided (such as hospitalization), and there can be a reduced titer of the pathogen.

**Prime-boost vaccination:** An immunotherapy including administration of a first immunogenic composition (the primer vaccine) followed by administration of a second immunogenic composition (the booster vaccine) to a subject to induce an immune response. The primer vaccine and/or the booster vaccine include a vector (such as a viral vector, RNA, or DNA vector) expressing the antigen to which the immune response is directed. The booster vaccine is administered to the subject after the primer vaccine; the skilled artisan will understand a suitable time interval between administration of the primer vaccine and the booster vaccine, and examples of such timeframes are disclosed herein. In some embodiments, the primer vaccine, the booster vaccine, or both primer vaccine and the booster vaccine additionally include an adjuvant. In other embodiments, the primer vaccine, the booster vaccine, or both primer vaccine and the booster vaccine do not include an adjuvant.

**Prokaryote:** Cell or organism lacking a membrane-bound, structurally discrete nucleus and other subcellular compartments.

**Promoter:** An array of nucleic acid control sequences which direct transcription of a nucleic acid. A promoter includes necessary nucleic acid sequences near the start site of transcription, such as in the case of a polymerase II type promoter, a TATA element. Enhancer and repressor elements can be located adjacent or distal to the promoter, and can be located as much as several thousand base pairs from the start site of transcription. Examples of promoters include, but are not limited to, the λ P_{L} and P_{R} promoters, the P_{BAD}, the β-actin promoter, and tissue-specific promoters.

**Recombinant nucleic acid molecule:** A nucleic acid molecule which is comprised of segments of DNA joined together by means of molecular biological techniques, or that is produced from such a molecule, such as following replication of a plasmid. Strands of a DNA molecule are said to have 5' ends and 3' ends because mononucleotides are reacted to make oligonucleotides in a manner such that the 5' phosphate of one mononucleotide pentose ring is attached to the 3' oxygen of its neighbor in one direction via a phosphodiester linkage. In a duplex DNA molecule, each strand has a 5' and a 3' end. In either a linear or circular DNA molecule, discrete elements are referred to as being "upstream" or 5' of the "downstream" or 3' elements relative to a reference nucleic acid sequence of a fixed element in DNA that has a polarized direction in a single strand. The promoter and enhancer elements which direct transcription of a linked gene are generally located 5' relative to a strand transcribed into RNA, or upstream of the coding region. However, enhancer elements can exert their effect even when located 3' of the promoter element and the coding region. Transcription termination and polyadenylation signals are located 3' or downstream of the coding region.

**Regulatory element:** A genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals, etc. Regulatory elements include promoter and enhancer elements that are involved in transcription (Maniatis et al., Science 236:1237, 1987).

**Selectable marker:** A nucleic acid (or a protein encoded by the nucleic acid) which can be used to identify a cell, such as a host cell, of interest. Selectable markers include but are not limited to: (1) nucleic acid segments that encode products which provide resistance against otherwise toxic compounds (*e.g*., antibiotics); (2) nucleic acid segments that encode products which are otherwise lacking in the recipient cell (e.g., tRNA genes, auxotrophic markers); (3) nucleic acids that encode products which suppress the activity of a gene product; (4) nucleic acids that encode products which can be readily identified (such as phenotypic markers such as β-galactosidase, green fluorescent protein (GFP), and cell surface proteins); (5) nucleic acids that bind products which are otherwise detrimental to cell survival and/or function; (6) nucleic acids that otherwise inhibit the activity of any of the nucleic acids described in Nos. 1-5 above (*e.g*., antisense oligonucleotides); (7) nucleic acids that bind products that modify a substrate (*e.g*. restriction endonucleases); (8) nucleic acids that can be used to isolate a desired molecule (e.g. specific protein binding sites); (9) a specific nucleotide sequence which can be otherwise non-functional (e.g., for PCR amplification of subpopulations of molecules); and/or (10) DNA segments, which when absent, directly or indirectly confer sensitivity to particular compounds.

In one example, the nucleic acid encodes an enzymatic activity that confers the ability to grow in medium lacking what would otherwise be an essential nutrient (*e.g.*, the TRPl gene in yeast cells). In another example, a selectable marker can confer resistance (or sensitivity) to an antibiotic or drug upon the cell in which the selectable marker is expressed. In a further example, the selectable marker can also be used to confer a particular phenotype upon a host cell.

When a host cell must express a selectable marker to grow in selective medium, the marker is said to be a positive selectable marker (*e.g*., antibiotic resistance genes which confer the ability to grow in the presence of the appropriate antibiotic). Selectable markers can also be used to select against host cells containing a particular gene (*e.g.*, the sacB gene which, if expressed, kills the bacterial host cells grown in medium containing 5% sucrose); selectable markers used in this manner are referred to as negative selectable markers or counter-selectable markers.

**Sequence identity:** The relatedness between two nucleic acid sequences, or two amino acid sequences is expressed in terms of the similarity between the sequences, otherwise referred to as sequence identity or homology. Sequence identity is frequently measured in terms of percentage identity (or similarity or homology); the higher the percentage, the more similar/homologous are the two sequences.

Methods of alignment of sequences for comparison are available. Various programs and alignment algorithms are described in: Smith and Waterman, Adv. Appl. Math. 2:482, 1981; Needleman and Wunsch, J. Mol. Bio. 48:443, 1970; Pearson and Lipman, Methods in Molec. Biology 24:307-331, 1988; Higgins and Sharp, Gene 73:237-244, 1988; Higgins and Sharp, CABIOS 5:151-153, 1989; Corpet et al., Nucleic Acids Research 16:10881-90, 1988; Huang et al., Computer Applications in BioSciences 8:155-65,1992; and Pearson et al., Methods in Molecular Biology 24:307-31,1994. Altschul et al. (Nature Genet., 6:119-29, 1994) presents a detailed consideration of sequence alignment methods and homology calculations.

The NCBI Basic Local Alignment Search Tool (BLAST) (Altschul et al., J. Mol. Biol. 215:403-410, 1990) is available from several sources, including the National Center for Biological Information (NBCI, Bethesda, MD) and on the internet, for use in connection with the sequence analysis programs blastp, blastn, blastx, tblastn and tblastx. It can be accessed at the NCBI website, together with a description of how to determine sequence relatedness using this program.

Homologs of D protein and E protein typically possess at least some (for example, at least 80%, at least 90%, or at least 95%) sequence identity counted over limited or full-length alignment with the amino acid sequence of the protein being evaluated. Homologs of a protein can be identified, for example, using the NCBI Blast 2.0, gapped blastp set to default parameters. For comparisons of amino acid sequences of greater than about 30 amino acids, the Blast 2 sequences function is employed using the default BLOSUM62 matrix set to default parameters, (gap existence cost of 11, and a per residue gap cost of 1). When aligning short peptides (fewer than around 30 amino acids), the alignment should be performed using the Blast 2 sequences function, employing the PAM30 matrix set to default parameters (open gap 9, extension gap 1 penalties). Proteins with even greater similarity to the reference sequence will show increasing percentage identities when assessed by this method, such as at least 70%, at least 75%, at least 80%, at least 90%, at least 95%, at least 98%, or at least 99% sequence identity. When less than the entire sequence is being compared for sequence relatedness, homologs will typically possess at least 75% sequence identity over short windows of 10-20 amino acids, and may possess sequence identities of at least 85% or at least 90% or 95% depending on their similarity to the reference sequence. Methods for determining sequence identity over such short windows are described at the NCBI website

These sequence identity ranges are provided for guidance only; it is entirely possible that strongly significant homologs or other variants could be obtained that fall outside of the ranges provided.

**Transfection/Transformation:** The introduction of foreign DNA into prokaryotic or eukaryotic cells. Transformation of prokaryotic cells may be accomplished by a variety of means known to the art including the treatment of host cells with CaCl₂ to make competent cells, electroporation, etc. Transfection of eukaryotic cells can be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection and biolistics.

**Tumor:** An abnormal growth of cells, which can be benign or malignant. Cancer is a malignant tumor, which is characterized by abnormal or uncontrolled cell growth. Other features often associated with malignancy include metastasis, interference with the normal functioning of neighboring cells, release of cytokines or other secretory products at abnormal levels and suppression or aggravation of inflammatory or immunological response, invasion of surrounding or distant tissues or organs, such as lymph nodes, etc. "Metastatic disease" refers to cancer cells that have left the original tumor site and migrate to other parts of the body for example *via* the bloodstream or lymph system.

The amount of a tumor in an individual is the "tumor burden" which can be measured as the number, volume, or weight of the tumor. A tumor that does not metastasize is referred to as "benign." A tumor that invades the surrounding tissue and/or can metastasize is referred to as "malignant." Examples of hematological tumors include leukemias, including acute leukemias (such as 11q23-positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Examples of solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, glioblastoma astrocytoma, medulloblastoma, craniopharyrgioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer.

**Vaccine:** A preparation (including but not limited to bacteriophage) administered for the prevention, amelioration or treatment of infectious disease. A vaccine induces an immune response to a particular disease of interest.

**Vector:** Nucleic acid molecules that transfer DNA segment(s) from one cell to another. A "vector" is a type of "nucleic acid construct." The term "nucleic acid construct" includes circular nucleic acid constructs such as plasmid constructs, cosmid vectors, etc. as well as linear nucleic acid constructs (*e.g.,* λ phage constructs, PCR products). The nucleic acid construct may include expression signals such as a promoter and/or an enhancer (in such a case, it is referred to as an expression vector). An "expression vector" is a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter and can also include an operator.

### Bacteriophage and Host Cells

Provided herein are bacteriophage λ that include a head, a tail, and a lambda genome that includes a nucleic acid sequence encoding a fusion protein comprising a D protein linked to heterologous antigen, wherein the nucleic acid sequence is inserted into a native *gene D* locus adjacent to *gene E* in the lambda genome, and wherein expression of the fusion protein results in the head of the bacteriophage λ comprising the heterologous antigen.

In wild-type λ, the position of gene D at the native locus is between nucleotide 5747 to 6079, including the start codon (Met) and the stop codon (TAA), with reference to the full length (48,502 base pairs) genome. In wild-type λ, *gene E* is located 55 bp downstream of the stop codon of *gene D* spanning the nucleotide position from 6135 to 7160, with reference to the full length (48,502 base pairs) genome. Thus, the disclosed bacteriophage λ includes a nucleic acid sequence encoding a fusion protein comprising the heterologous antigen and the D protein at the native locus for the D protein. A nucleic acid sequence encoding the heterologous antigen, and optionally a linker sequence, can be inserted in the wild-type gene D using recombineering techniques, see for example, U.S. Patent No. 7,144,734 Optionally, the disclosed bacteriophage λ include wild-type D protein in the head of the bacteriophage; the wild-type D protein is not encoded by the bacteriophage λ genome.

The nucleic acid sequence encoding the heterologous antigen is inserted into the bacteriophage λ genome such that it is expressed as a fusion D protein, from the native D locus. In wild-type bacteriophage λ, the D protein comprises the amino acid sequence of:

MTSKETFTHYQPQGNSDPAHTATAPGGLSAKAPAMTPLMLDTSSRKLVAWDGTTDGAAV
GILAVAADQTSTTLTFYKSGTFRYEDVLWPEAASDETKKRTAFAGTAISIV (SEQ ID NO: 1).

In some embodiments, the D protein comprises an amino acid sequence at least 90%, 91%, 92%, 93%, 94%, 95%, 96%, 97%, 98%, 99% or 99% identical to SEQ ID NO: 1. In a non-limiting example, the D protein is at least 95% identical to SEQ ID NO: 1, such as 98% or 99% identical to SEQ ID NO: 1. The D protein can include at most 1, 2, 3, 4, 5, 6, 7, 8, 9, or 10 conservative amino acid substitutions in SEQ ID NO: 1, provided the D protein is stably incorporated into the head of bacteriophage λ. The D protein can comprise, or consist of, the amino acid sequence of SEQ ID NO: 1.

In some embodiments, the bacteriophage λ genome includes a nucleic acid sequence encoding the D protein and a nucleic acid sequence encoding the heterologous antigen, wherein the nucleic acid sequence encoding the D protein is 5' of the nucleic acid sequence encoding the heterologous antigen in the lambda genome. In other embodiments, the bacteriophage λ genome includes a nucleic acid sequence encoding the D protein and a nucleic acid sequence encoding the heterologous antigen, wherein the nucleic acid sequence encoding the D protein is 3' of the nucleic acid sequence encoding the heterologous antigen in the lambda genome. In some embodiments a nucleic acid sequence encoding the heterologous antigen is inserted either in between nucleotides 5749 and 5750 (with reference to the wild-type bacteriophage λ) at the N terminus of D or between 6076 and 6077 (with reference to the wild-type bacteriophage λ) at the C-terminus of D. In some embodiments, these insertions can be made using recombineering, see, for example, U.S. Patent No. 7,144,734,

Optionally a nucleic acid sequence encoding a linker is inserted between the nucleic acid sequence encoding the D protein and the nucleic acid sequence encoding the heterologous antigen. Thus, in some embodiments, the fusion protein includes a linker between the D protein and the heterologous antigen. The linker can be, for example, 5 to 25 amino acids in length, such as 5 to 15, 5 to 20, or 5 to 10 amino acids in length. In some embodiments, the linker is 5, 6, 7, 8, 9, 10, 11, 12, 13, 14, 15, 16, 17, 18, 19 or 20 amino acids in length. The linker can be, for example, four glycine resides and a serine residue, or multiple copies of this amino acid sequence (GGGGS, SEQ ID NO: 2).

The genome of the bacteriophage λ further includes a gene *E* that encodes the E protein. In some embodiments, gene *E* encodes an E protein comprising a lysine at position 158 of the E protein. An exemplary amino acid sequence of the E protein is provided below (with E158 underlined, which can be mutated to K158):

MSMYTTAQLLAANEQKFKFDPLFLRLFFRESYPFTTEKVYLSQIPGLVNMALYVSPIVSGE
VIRSRGGSTSEFTPGYVKPKHEVNPQMTLRRLPDEDPQNLADPAYRRRRIIMQNMRDEELA
IAQVEEMQAVSAVLKGKYTMTGEAFDPVEVDMGRS[?]ENNITQSGGTEWSKRDKSTYDPT
DDIEAYALNASGVVNIIVFDPKGWALFRSFKAVKEKLDTRRGSNSELETAVKDLGKAVSY
KGMYGDVAIVVYSGQYVENGVKKNFLPDNTMVLGNTQARGLRTYGCIQDADAQREGIN
ASARYPKNWVTTGDPAREFTMIQSAPLMLLADPDEFVSVQLA (SEQ ID NO: 3).

In some embodiments, the bacteriophage λ genome includes a deletion in the b region. The function of b region in the lambda genome is unknown; it is not essential for the bacteriophage λ growth. The b region is flanked by the upstream gene orf-194 encoding putative fiber assembly protein and downstream gene int encoding integration protein. Three hypothetical non-essential genes present in the b region: *ea47, ea31,* and *ea59.* The nucleotide coordinates comprising these three genes are from 22689 to 26973 in wild-type bacteriophage λ.

In some embodiments, the deletion in the bacteriophage λ genome can be about 1 kilobase (kb) to about 5 kb of the b region. In specific non-limiting example, the deletion in the bacteriophage λ genome is about 1 kb to about 4 kb of the b region, such as about 1 kb to 3 kb of the b region, such as 1 kb to about 2 kb of the b region, for example about 1, 2, 3, 4, or 5 kb of the b region. The deletion in the bacteriophage λ genome can be, for example, about 2 kb to about 5 kb, about 3 kb to about 5 kb, or about 4 kb to about 5 kb of the b region. In one non-limiting example, about 4 to about 5 kb of the b region is deleted in the bacteriophage λ genome. In another specific non-limiting example, the entire 4285 bp region between 22689 to 26973 is deleted in the bacteriophage λ genome. In some embodiments, the genes *ea47, ea31* and *ea59* are deleted in the bacteriophage λ genome. In yet other embodiments, only one or two of the genes *ea47, ea31* and *ea59* are deleted in the bacteriophage λ genome.

The disclosed prophages can be temperature sensitive, such that the lytic phase is induced when temperature is increased. In some embodiments, host cells are grown at about 37 °C, and increasing temperature, such as to about 42 °C results in induction of the lytic phase, such that the bacteriophage λ are released by the host cells.

### Exemplary heterologous antigens

The heterologous antigen included in the fusion protein can be any heterologous antigen of interest. In one example, the heterologous antigen is at least 5 amino acids (aa), such as at least 6, 7, 8, 9, 10, 15, 20, 30, 40, 50, 60, 70, 80, 90, 100, 200, 500, 750, 1000, 1250, or 1500 aa, such as 5 to 1500 amino acids in length. In further embodiments, the heterologous antigen can be 8 to 1500 amino acids in length, such as 8 to 100, 8 to 200, 8 to 300, 8 to 400, 8 to 500, 8 to 600, 8 to 700, 8 to 800, 8 to 900, 8 to 1,000, 8 to 1,100, 8 to 1,200, 8 to 1,300 or 8 to 1,400 amino acids in length. In further embodiments, the antigen is 10 to 20, 10 to 30, 10 to 40, 10 to 50, 10 to 60, 10 to 70, 10 to 80, 10 to 90, 10 to 100, 10 to 110, 10 to 120, 10 to 130, 10 to 10, 10 to 150, 10 to 160, 10 to 170, 10 to 180, 10 to 190 or 10 to 200 amino acids in length. In more embodiments, the antigen is 20 to 200 amino acids in length, such as 30 to 200, 40 to 200, 50 to 200, 60 to 200, 70 to 200, 80 to 200, 90 to 200, 100 to 200, 110 to 200, 120 to 200, 130 to 200, 140 to 200, 150 to 200, 160 to 200, 170 to 200, 180 to 200, or 190 to 200 amino acids in length. In a specific non-limiting example, the antigen is 28 to 200 amino acids in length.

The heterologous antigen can be a microbial/infectious agent antigen, such as without limitation an antigen from a bacterial, viral, fungal, parasitic, protozoa, nematode, or mycobacterial species. Microbial antigens include bacterial antigens, viral antigens, fungal antigens, parasitic antigens, protozoa antigens, nematode antigens, and mycobacterial antigens. Examples of bacterial, viral, fungal, parasitic protozoa, nematode, and mycobacterial species are provided herein, without limitation. Such infectious agents can serve as the source of a heterologous antigen.

A viral antigen can be, for example, an antigen from a severe acute respiratory syndrome coronavirus 2 (SARS-CoV-2), human immunodeficiency virus, rotavirus, influenza virus, Dengue virus, Zika virus, Chikungunya virus, or a Foot-and-mouth disease (FMD) virus. The viral antigen can be a porcine virus antigen.

In one non-limiting example, the antigen from the SARS-CoV-2 virus is a spike protein antigen. An exemplary antigen is provided as SEQ ID NO: 4 
KRVDFCGKGYHLMSFPQSAPHGVVFLHVTYVPAQEKNFTTAPAICHDGKAHFPREGVFVS
NGTHWFVTQRNFYEPQIITTDNTFVSGNCDVVIGIVNNTVYDPLQPELDSFKEELDKYFKN
HTSPDVDLGDIS (SEQ ID NO: 4, see S-14 in FIG. 12B).
 Additional SARS CoV-2 antigens of use are provided as SEQ ID NOs: 39-52, see Tables 2 and 3 provided in the Examples section below. In one embodiment, the SARS-CoV-2 antigen includes, or consists of, SEQ ID NO: 44. In another embodiment, the SARS-CoV-2 antigen includes, or consists of, SEQ ID NO: 45. In a further embodiment, the SARS-CoV-2 antigen includes, or consists of, SEQ ID NO: 47. An 11 amino acid epitope is also shown in FIG. 18. In more embodiments, the SARS-CoV-2 antigen includes, or consists of, this epitope (FKEELDKYFKN, SEQ ID NO: 69). These heterologous antigens can be linked to a D protein and included in the bacteriophage λ disclosed herein.

Additional exemplary antigens are provided in SEQ ID NOs: 22-35. These are shown in the table below:

| **Antigen # (Strain)** | **Sequence** | **length (aa)** |
|---|---|---|
| ORF3a-1 (XTL-1292) | DLFMRIFTIGTVTLKQGEIKDATPSDFVRATATIPIQASLPFGWLIVG VA (SEQ ID NO: 22) | 50 |
| N-2 (XTL-1293) | SDNGPQNQRNAPRITFGGPSDSTGSNQNGERSGARSKQRRPQGLPN NTASWFTALTQHGKED (SEQ ID NO: 23) | 62 |
| N-3 (XTL-1294) | GALNTPKDHIGTRNPANNAAIVL (SEQ ID NO: 24) | 23 |
| S-4 (XTL-1312) | TNTSNQVAVLYQDVNCTEVPVAIHADQLTPTWRVYSTGSNVFQTR AG (SEQ ID NO: 25) | 47 |
| S-5 (XTL-1313) | SSSGWTAGAAAYYVGYLQPRTFLL (SEQ ID NO: 26) | 24 |
| S-6 (XTL-1314) | FLLTTKRTMFVFLVLLPLVSSQCVNLTTRTQLPPAYTNSFTRGVYYP DKVFRSSVLHSTQDLFLPFFSNVTWFHAIHVSGTNGTKRFDNPVLPF NDGVYFASTEKSNIIRGWIFGTTLDSKTQSLLIVNNATN (SEQ ID NO: 27) | 133 |
| S-7 (XTL-1315) | NATNVVIKVCEFQFCNDPFLGVYYHKNNKSWMESEFRVYSSANNC TFEYVSQPFLMDLEGKQGNFKNLREFVFKNIDGYFKIYSKHTPINLV RDLPQGFSALEPLVDLPIGINITRFQTLLALHRSYLTPGDS (SEQ ID NO: 28) | 133 |
| S-8 (XTL-1316) | DSSSGWTAGAAAYYVGYLQPRTFLLKYNENGTITDAVDCALDPLS ETKCTLKSFTVEKGIYQTSNFRVQPTESIVRFPNITNLCPFGEVFNAT RFASVYAWNRKRISNCVADYSVLYNSASFSTFKCYGVSPT (SEQ ID NO: 29) | 133 |
| S-9 (XTL-1317) | PTKLNDLCFTNVYADSFVIRGDEVRQIAPGQTGKIADYNYKLPDDF TGCVIAWNSNNLDSKVGGNYNYLYRLFRKSNLKPFERDISTEIYQA GSTPCNGVEGFNCYFPLQSYGFQPTNGVGYQPYRVVVLSFE (SEQ ID NO: 30) | 133 |
| S-10 (XTL-1318) | FELLHAPATVCGPKKSTNLVKNKCVNFNFNGLTGTGVLTESNKKFL PFQQFGRDIADTTDAVRDPQTLEILDITPCSFGGVSVITPGTNTSNQV AVLYQDVNCTEVPVAIHADQLTPTWRVYSTGSNVFQTRA (SEQ ID NO: 31 | 133 |
| S-11 (XTL-1319) | RAGCLIGAEHVNNSYECDIPIGAGICASYQTQTNSPRRARSVASQSII AYTMSLGAENSVAYSNNSIAIPTNFTISVTTEILPVSMTKTSVDCTM YICGDSTECSNLLLQYGSFCTQLNRALTGIAVEQDKNT (SEQ ID NO: 32) | 133 |
| S-12 (XTL-1320) | NTQEVFAQVKQIYKTPPIKDFGGFNFSQILPDPSKPSKRSFIEDLLFNK VTLADAGFIKQYGDCLGDIAARDLICAQKFNGLTVLPPLLTDEMIAQ YTSALLAGTITSGWTFGAGAALQIPFAMQMAYRFNGI (SEQ ID NO: 33) | 133 |
| S-13 (XTL-1321) | NGIGVTQNVLYENQKLIANQFNSAIGKIQDSLSSTASALGKLQDVVN QNAQALNTLVKQLSSNFGAISSVLNDILSRLDKVEAEVQIDRLITGR LQSLQTYVTQQLIRAAEIRASANLAATKMSECVLGQSKR (SEQ ID NO: 34) | 133 |
| S-14 (XTL-1322) | KRVDFCGKGYHLMSFPQSAPHGVVFLHVTYVPAQEKNFTTAPAICH DGKAHFPREGVFVSNGTHWFVTQRNFYEPQIITTDNTFVSGNCDVV IGIVNNTVYDPLQPELDSFKEELDKYFKNHTSPDVDLGDIS (SEQ ID NO: 4) | 133 |
| S-15 (XTL-1323) | LGDISGINASVVNIQKEIDRLNEVAKNLNESLIDLQELGKYEQYIKW PWYIWLGFIAGLIAIVMVTIMLCCMTSCCSCLKGCCSCGSCCKFDED DSEPVLKGVKLHYT (SEQ ID NO: 39) | 108 |
| E-16 (XTL-1324) | MYSFVSEETGTLIVNSVLLFLAFVVFLLVTLAILTALRLCAYCCNIVN VSLVKPSFYVYSRVKNLNSSRVPDLLV (SEQ ID NO: 35) | 75 |

The viral antigen can be one from a metapneumovirus, a norovirus, a papillomavirus, a parvovirus, a SARS virus, a smallpox virus, a picornaviruses, a respiratory syncytial virus, a parainfluenza virus, a measles virus, a hepatitis virus, an Ebola virus, a varicella zoster virus, a rabies virus or a West Nile virus antigen.

Exemplary heterologous antigens can be from a bacterium, such as from *E. coli, Staphylococcal, Streptococcal, Pseudomonas, Clostridium difficile, Legionella, Pneumococcus, Haemophilus, Klebsiella, Enterobacter, Citrobacter, Neisseria, Shigella, Salmonella, Listeria, Pasteurella, Streptobacillus, Spirillum, Treponema, Actinomyces, Borrelia, Corynebacterium, Nocardia, Gardnerella, Campylobacter, Spirochaeta, Proteus, Bacteriodes, H. pylori,* and *B. anthracis*

Exemplary heterologous antigens can be from a fungus, such as from any fungal species that causes an infection, such as, but not limited to, candidiasis, ringworm, histoplasmosis, blastomycosis, paracoccidioidomycosis, crytococcosis, aspergillosis, chromomycosis, mycetoma infections, pseudallescheriasis, and tinea versicolor infection.

Exemplary heterologous antigens can be from a parasite, such as from *Amebiasis, Trypanosoma cruzi, Fascioliasis, Leishmaniasis, Plasmodium, Onchocerciasis, Paragonimiasis, Trypanosoma brucei, Pneumocystis, Trichomonas vaginalis, Taenia, Hymenolepsis, Echinococcus, Schistosomiasis, neurocysticercosis, Necator americanus, Naegleria, Toxoplasma (e.g., Toxoplasma gondii)* and *Trichuris trichuria.*

Exemplary heterologous antigens can be from a mycobacterium, such as from *M*. *tuberculosis* and *M. leprae,* but are not so limited.

In specific non-limiting examples, the heterologous antigen is an antigen from *Plasmodium* or *Leishmania.* An exemplary *Plasmodium* antigen includes SEQ ID NO: 5.

ASKSKEHKSKGKKDKGKKDKGKHKKAKKEKVKKHVVKNVIEDEDKDGVEIINLEDKEAC
EEQHITVESRPLSQPQCKLIDEPEQLTLMDKSKVEEKNLSIQEQLIGTIGRVNVVPRRDNHK
KKMAKIEEAELQKQKHVDKEED

In further embodiments, the heterologous antigen is a tumor antigen. A tumor antigen is expressed preferentially by tumor cells (i.e., it is expressed at higher levels in tumor cells than on non-tumor cells) and in some instances it is expressed solely by tumor cells. The tumor can be a solid tumor. The tumor can be a leukemia or a lymphoma.

The tumor antigen can be from melanoma, bladder cancer, breast cancer, colon cancer, carcinoma, sarcoma, a lymphoma, or a leukemia. The tumor antigen can be from a thyroid cancer, brain tumor, renal cancer, lung cancer, pancreatic cancer, or prostate cancer. The tumor antigen can be from a thyroid cancer. The tumor antigen can be from a lymphoma or a leukemia. In one non-limiting example, the tumor antigen is a chronic lymphocytic leukemia antigen. An exemplary the chronic lymphocytic leukemia antigen is provided below:
CARDRRGEWPPSDYYYYYMDVW (SEQ ID NO: 6).

In some embodiments, the heterologous antigen is a carcinoma antigen. In another embodiment, the heterologous antigen is a glioblastoma antigen, a head and neck carcinoma, a breast carcinoma and/or a bladder carcinoma antigen. In one embodiment, the heterologous antigen is a glioblastoma antigen.

EGFR is frequently involved in the oncogenic progression of human cancer. Various alterations in expression including gene amplifications and activating mutations contribute to oncogenesis. This large receptor has an external domain (ECD) of 621 amino acids, a single pass transmembrane domain (TM) of 23 amino acids and an enzymatically active intracellular domain (ICD) of 542 amino acids. The EGFR is a member of the receptor tyrosine kinase family. Ligand binding leads to receptor dimer formation and the activation of the kinase domain which signals to one of several pathways that can promote the growth, survival and spread of mammalian cells. Activating mutations can occur in either the ECD or the ICD; there are also gene amplifications and large deletions exemplified by the loss of exons 2-7 to produce EGFR variant (v) III or the loss of exon 19 to generate a constitutively active enzyme mutant. The expression of EGFRvIII or the loss of exon 19 are reported for cancer cells only, see PCT Publication No. WO 2021/003297. In more embodiments, the heterologous antigen of use in the disclosed phages is an EGFRvIII antigen. Exemplary EGFRvIII antigens that can be included in the disclosed phages are provided as SEQ ID NOs: 53-57, see also Table 3). In some examples, the tumor is a glioblastoma and the heterologous antigen is an EGFRvIII antigen.

Tumors associated with specific antigens include acute lymphoblastic leukemia (etv6; aml1; cyclophilin b), B cell lymphoma (Ig-idiotype); Burkitt's (Non-Hodgkin's) lymphoma (CD20); glioma (E-cadherin; .alpha.-catenin; .beta.-catenin; .gamma.-catenin; p120ctn), bladder cancer (p21ras), biliary cancer (p21ras), breast cancer (MUC family; HER2/neu; c-erbB-2), cervical carcinoma (p53; p21ras), colon carcinoma (p21ras; HER2/neu; c-erbB-2; MUC family), colorectal cancer (Colorectal associated antigen (CRC)--0017-1A/GA733; APC), choriocarcinoma (CEA), epithelial cell-cancer (cyclophilin b), gastric cancer (HER2/neu; c-erbB-2; ga733 glycoprotein), hepatocellular cancer (α-fetoprotein), Hodgkin's lymphoma (lmp-1; EBNA-1), lung cancer (CEA; MAGE-3; NY-ESO-1), lymphoid cell-derived leukemia (cyclophilin b), melanoma (p15 protein, gp75, oncofetal antigen, GM2 and GD2 gangliosides), myeloma (MUC family; p21ras), non-small cell lung carcinoma (HER2/neu; c-erbB-2), nasopharyngeal cancer (lmp-1; EBNA-1), ovarian cancer (MUC family; HER2/neu; c-erbB-2), prostate cancer (Prostate Specific Antigen (PSA) and its immunogenic epitopes PSA-1, PSA-2, and PSA-3; PSMA; HER2/neu; c-erbB-2), pancreatic cancer (p21ras; MUC family; HER2/neu; c-erbB-2; ga733 glycoprotein), renal (HER2/neu; c-erbB-2), squamous cell cancers of cervix and esophagus (viral products such as human papilloma virus proteins and non-infectious particles), testicular cancer (NY-ESO-1), T cell leukemia (HTLV-1 epitopes), and melanoma (Melan-A/MART-1; cdc27; MAGE-3; p21ras; gp100.sup.Pmel1117). Thus, any of these tumor antigens can be a heterologous antigen.

Exemplary tumor antigens that can be used as the heterologous antigen include, but are not limited to, MART-1/Melan-A, gp100, adenosine deaminase-binding protein (ADAbp), FAP, cyclophilin b, colorectal associated antigen (CRC)--0017-1A/GA733, carcinoembryonic antigen (CEA), CAP-1, CAP-2, etv6, AML1, prostate specific antigen (PSA), PSA-1, PSA-2, PSA-3, prostate-specific membrane antigen (PSMA), T cell receptor/CD3-zeta chain, and CD20. Additional tumor antigens include, but are not limited to, MAGE-A1, MAGE-A2, MAGE-A3, MAGE-A4, MAGE-A5, MAGE-A6, MAGE-A7, MAGE-A8, MAGE-A9, MAGE-A10, MAGE-A11, MAGE-A12, MAGE-Xp2 (MAGE-B2), MAGE-Xp3 (MAGE-B3), MAGE-Xp4 (MAGE-B4), MAGE-C1, MAGE-C2, MAGE-C3, MAGE-C4, MAGE-C5). Tumor antigens that can be used as the heterologous antigen also include, but are not limited to GAGE-1, GAGE-2, GAGE-3, GAGE-4, GAGE-5, GAGE-6, GAGE-7, GAGE-8, GAGE-9. The tumor antigen can also be BAGE, RAGE, LAGE-1, NAG, GnT-V, MUM-1, CDK4, tyrosinase, p53, MUC family, HER2/neu, p21ras, RCAS1, alpha-fetoprotein, E-cadherin, α-catenin, .β-catenin, γ-catenin, p120ctn, gp100.sup.Pmel117, PRAME, NY-ESO-1, cdc27, adenomatous polyposis coli protein (APC), fodrin, Connexin 37, Ig-idiotype, p15, gp75, GM2 ganglioside, GD2 ganglioside, human papilloma virus proteins, a member of the Smad family of tumor antigens, Imp-1, P1A, EBV-encoded nuclear antigen (EBNA)-1, brain glycogen phosphorylase, SSX-1, SSX-2 (HOM-MEL-40), SSX-1, SSX-4, SSX-5, SCP-1 and CT-7, CD20, and c-erbB-2.

The heterologous antigen can be an allergen. Allergens include without limitation pollens, insect venoms, and other proteins. Examples of natural, animal and plant allergens include but are not limited to proteins specific to the following genera: *Canine (Canis familiaris); Dermatophagoides (e.g. Dermatophagoides farinae); Felis (Felis domesticus); Ambrosia (Ambrosia artemiisfolia; Lolium (e.g. Lolium perenne or Lolium multiflorum); Cryptomeria (Cryptomeria japonica); Alternaria (Alternaria alternata); Alder; Alnus (Alnus gultinoasa); Betula (Betula verrucosa); Quercus (Quercus alba); Olea (Olea europa); Artemisia (Artemisia vulgaris); Plantago (e.g. Plantago lanceolata); Parietaria (e.g. Parietaria officinalis or Parietaria judaica); Blattella (e.g. Blattella germanica); Apis (e.g. Apis multiflorum); Cupressus (e.g. Cupressus sempervirens, Cupressus arizonica and Cupressus macrocarpa); Juniperus (e.g. Juniperus sabinoides, Juniperus virginiana, Juniperus communis and Juniperus ashei); Thuya (e.g. Thuya orientalis); Chamaecyparis (e.g. Chamaecyparis obtusa); Periplaneta (e.g. Periplaneta americana); Agropyron (e.g. Agropyron repens); Secale (e.g. Secale cereale); Triticum (e.g. Triticum aestivum); Dactylis (e.g. Dactylis glomerata); Festuca (e.g. Festuca elatior); Poa (e.g. Poa pratensis or Poa compressa); Avena (e.g. Avena sativa); Holcus (e.g. Holcus lanatus); Anthoxanthum (e.g. Anthoxanthum odoratum); Arrhenatherum (e.g. Arrhenatherum elatius); Agrostis (e.g. Agrostis alba); Phleum (e.g. Phleum pratense); Phalaris (e.g. Phalaris arundinacea); Paspalum (e.g. Paspalum notatum); Sorghum (e.g. Sorghum halepensis); and Bromus (e.g. Bromus inermis).* Thus, in some embodiments, the agent of interest is a tolerogenic antigen.

### Exemplary methods of production

In some embodiments, recombineering can be used to produce a bacteriophage λ encoding the fusion protein at the native *gene D* locus. A nucleic acid molecule encoding the heterologous antigen, and optionally a nucleic acid molecule encoding a linker, are inserted into the native gene *D.* One method of recombineering involves transforming a gram-negative bacterial cell (such as, but not limited to, an *E. coli* cell) with a plasmid including an origin of replication, and a lambda genome having DNA encoding functional Beta and optionally Exo, and Gam, or functional fragments or variants thereof, operably linked to the de-repressible promoter (such as, but not limited to, the P_{L} promoter). De-repression of the de-repressible promoter (for example, the induction of transcription from the P_{L} promoter by inactivation of cI) induces expression of *Exo, Bet* and *Gam.* In some embodiments de-repression may be selectively activated for this purpose. Another method of recombineering involves the introduction of a phage or a phage including the P_{L} promoter operably linked to a nucleic acid encoding Beta, and optionally Exo and Gam, or functional fragments or variants thereof.

In recombineering, a polynucleotide which is homologous to a target DNA D gene sequence (capable of undergoing homologous recombination with the target D gene sequence) is introduced into the cell. The polynucleotide can be targeted to the N-terminus of the C terminus of the D gene sequence. Cells in which homologous recombination has occurred are either selected or found by direct screening of cells. In particular embodiments, the nucleic acid introduced into the cell may be single-stranded DNA, double-stranded DNA, or DNA with 5' overhangs. These methods are disclosed, for example, in PCT Publication No. WO 02/14495 A2 and in U.S. Patent Publication No. US-2003-0224521-A1

Briefly, the recombineering methodology utilizes recombination functions (such as phage recombination functions) under control of a de-repressible promoter to generate recombination products using homologies of at least 20 base pairs. Thus, in one embodiment, recombineering uses a cell including Beta under the control of a de-repressible promoter. In a specific, non-limiting example, expression of Beta alone (without Exo and Gam) is under the control of the de-repressible promoter (*e.g*., the nucleic acid encoding Beta is operably linked to the de-repressible promoter). In another embodiment, expression of Beta, in addition to Gam and/or Exo, is under the control of the de-repressible promoter. In yet another embodiment, DNA bound to a Beta protein is introduced into a host cell.

The length of the homologous sequence can be varied; two homology arms are present flanking the sequence encoding the heterologous antigen. In several embodiments, the homology is at least 20, at least 25, at least 30, at least 40, at least 50, at least 75 or at least 100 nucleotides in length. However, larger regions of homology can also be utilized. Thus, in one embodiment, between about 20 and about 1,000 nucleotides of homologous sequence is utilized, between about 30 and about 1,000 nucleotides of homologous sequence is utilized. In one specific, non-limiting example, the ssDNA is about 20, about 25, about 30, about 40, about 50, about 75 or about 100 nucleotides in length. In one embodiment, the homologous nucleic acid is a single-stranded nucleic acid. In another embodiment, the homologous nucleic acid is a double-stranded nucleic acid. Double-stranded nucleic acids include molecules that are completely double-stranded, as well as nucleic acid molecules that have a 5' or a 3' overhang.

A single-stranded nucleic acid or double-stranded nucleic acid including sufficient homology to the target D gene sequence, and encoding the heterologous antigen, is introduced into the host cell. "Sufficient homology" is any region of sufficient identity to the target sequence such that recombination can occur. In several embodiments, sufficient homology includes a sequence of at least 20 nucleotides in length, wherein at most five, at most three, at most two, at most one nucleotide, or no nucleotides differ from the target nucleic acid sequence. In additional embodiments, sufficient homology includes a sequence of at least 25 nucleotides in length, wherein at most five, at most three, at most two, at most one nucleotide, or no nucleotides differ from the target nucleic acid sequence. Similarly, sufficient homology can readily be determined for a nucleic acid of at least 30, at least 40, at least 50, or at least 100 nucleotides in length.

If the single-stranded nucleic acid or double-stranded nucleic acid differs from the target nucleic acid, these differences can be clustered (i.e., at one area in the target nucleic acid) or can be scattered in the sequences (for example two nucleotide differences from the target sequence, wherein each difference is located at different areas in the sequence). In another embodiment, sufficient homology includes about a 100%, 99%, 98%, or 97% sequence identity between the homologous nucleic acid (e.g., the single-stranded or the double-stranded nucleic acid) and the target D gene nucleic acid sequence. In another specific, non-limiting example, sufficient homology includes at least 90% sequence identity between the single-stranded or double-stranded nucleic acid and the target D gene nucleic acid, such as nucleic acid sequences that are at least 91%, at least 92%, at least 93%, at least 94%, at least 95%, at least 96%, at least 97%, at least 98%, or at least 99% identical. A homologous nucleic acid sequence can differ from a target D gene nucleic acid by substitutions, deletions and/or additions of nucleotides. In another embodiment, the single-stranded nucleic acid (or double-stranded nucleic acid) is labeled, such as with a biotinylated nucleotide, a methylated nucleotide, or a DNA adduct.

The homologous nucleic acid (*e.g.*, the single-stranded nucleic acid or double-stranded nucleic acid) can be introduced into the host cell by any means known to one of skill in the art. In one embodiment, the host cell is deficient in mismatch repair, such as a cell that can repair mismatched nucleotides at a reduced frequency as compared to a wild-type cell (such as at least 50%, 60%, 70%, 80%, 90%, 95%, 98%, or 99% reduction in mismatch repair). In one specific, non-limiting example, mismatch repair is reduced at least 90% as compared to a wild-type cell. A host cell deficient for mismatch repair can include a mutation in a nucleic acid sequence encoding a protein involved in mismatch repair, such that the protein has reduced function (or its function is eliminated). In several embodiments, the function of one or more mismatch repair proteins is decreased at least 80%, such as at least 90%, 95%, 96%, 97%, 98%, 99%, or is completely absent in the host cell deficient for mismatch repair as compared to a wild-type cell. In this context, a wild-type cell is a cell of the same species that does not include a mutation in the gene encoding the protein involved in mismatch repair.

The homologous nucleic acid (*e.g.*, the single-stranded nucleic acid or double-stranded nucleic acid) is introduced into the host cell, the de-repressible promoter is de-repressed, and recombinants are generated *in vivo.* Thus, in one specific, non-limiting example, if the de-repressible promoter is P_{L}, and the repressor is cI857, the host cell is treated with heat to induce the expression of Beta (see Copeland et al., Nature Reviews 2:769, 2001, and Ellis et al., Proc. Natl. Acad. Sci. 98:6742-6746, 2001,), and optionally Exo and Gam. Generally, the homologous nucleic acid, whether it is a single-stranded nucleic acid or a double-stranded nucleic acid, differs from the target nucleic acid by at least one nucleotide, but is sufficiently homologous to undergo recombination with the target sequence (see above).

Recombinants can be detected by any known method. If recombination has occurred in a nucleic acid encoding a marker, such as a nucleic acid encoding a polypeptide involved in antibiotic resistance, detection can be performed by drug selection. However, detection can also be performed by direct screening (*e.g.*, colony hybridization or sequencing). Detection can also be performed by detecting a label on the nucleic acid (*e.g.*, when DNA includes a DNA adduct or a marker such as biotin).

As has been described (see PCT Publication No. WO 02/14495 A2, a single base change has been substituted in the *galK* gene and a 3.3 kbp insertion removed from the *galK* gene using single-stranded oligos. Single-stranded oligos have also been used to precisely remove five different *Tn10* insertions at different places on the *E. coli* chromosome. Whereas Exo, Beta, and Gam facilitate recombination of PCR amplified dsDNA cassettes with flanking homologies, only Beta is required for ssDNA recombination. Any of these methods can be used to incorporate a nucleic acid molecule encoding the heterologous antigen into a region of the bacteriophage λ, such as in the D gene.

The bacteriophage λ can further encode a side tail fiber (stf) fusion protein and/or a gpV fusion protein, wherein the side tail fiber fusion protein and/or the gpV fusion protein is expressed on the tail of the bacteriophage λ. Exemplary amino acid sequences for stf and gpV are provided below:
Stf protein: 
MAVKISGVLKDGTGKPVQNCTIQLKARRNSTTVVVNTVGSENPDEAGRYSMDVEYGQYSVILQVD
GFPPSHAGTITVYEDSQPGTLNDFLCAMTEDDARPEVLRRLELMVEEVARNASVVAQSTADAKKS
AGDASASAAQVAALVTDATDSARAASTSAGQAASSAQEASSGAEAASAKATEAEKSAAAAESSKN
AAATSAGAAKTSETNAAASQQSAATSASTAATKASEAATSARDAVASKEAAKSSETNASSSAGRA
ASSATAAENSARAAKTSETNARSSETAAERSASAAADAKTAAAGSASTASTKATEAAGSAVSASQS
KSAAEAAAIRAKNSAKRAEDIASAVALEDADTTRKGIVQLSSATNSTSETLAATPKAVKVVMDETN
RKAHWTVRH (SEQ ID NO: 36)
V protein:

MPVPNPTMPVKGAGTTLWVYKGSGDPYANPLSDVDWSRLAKVKDLTPGELTAESYDDSYLDDEDADWTATGQGQKSAGDTSFTLAWMPGEQGQQALLAWFNEGDTRAYKIRFPNGTVDVFRGWVSSIGKAVTAKEVITRTVKVTNVGRPSMAEDRSTVTAATGMTVTPASTSVVKGQSTTLTVAFQPEGVTDKSFRAVSADKTKATVSVSGMTITVNGVAAGKVNIPVVSGNGEFAAVAEITVTAS (SEQ ID NO: 37)

The stf or the gpV fusion protein can include any of the heterologous antigens disclosed herein. In some embodiments, the fusion protein including the D protein and the fusion protein including the stf or gpV protein both include the same heterologous antigen. In other embodiments, the fusion protein including the D protein and the fusion protein including the stf or gpV protein include the different heterologous antigen. The different heterologous antigens can be from the same organism (bacteria, virus, parasite, etc.) or the same tumor. The different heterologous antigens can be from the different organisms (bacteria, virus, parasite, etc.) or the different tumors. Recombineering, as discussed above, can be used to produce a phage that includes a stf fusion protein and/or a gpV fusion protein.

In some embodiments, in addition to the fusion protein, the bacteriophage λ further comprises wild-type D protein in the capsid head. Without being bound by theory, for some antigens, the wild-type D protein stabilizes the capsid head. This wild-type D protein is different from the fusion protein, and is not encoded by the bacteriophage λ genome. The wild-type D protein can be provided in trans, such as from a plasmid or nucleic acid inserted into the bacterial chromosome in a host cell, from which the bacteriophage is produced. Such host cells are provided herein.

In some embodiments, the D protein comprises an amino acid sequence at least 90%, 91%, 92%, 93%, 94%, 95%, 96%, 97%, 98%, 99% or 99% identical to SEQ ID NO: 1. In a non-limiting example, the D protein is at least 95% identical to SEQ ID NO: 1, such as 98% or 99% identical to SEQ ID NO: 1. The D protein can include at most 1, 2, 3, 4, 5, 6, 7, 8, 9, or 10 conservative amino acid substitutions in SEQ ID NO: 1, provided the D protein is stably incorporated into the head of bacteriophage λ. The D protein can comprise, or consist of, the amino acid sequence of SEQ ID NO: 1. This D protein is not fused to a heterologous antigen. In some embodiments, the wild-type D protein is operably linked to an inducible promoter, such as P_{BAD}. In some embodiments, the nucleic acid sequence encoding the wild-type D protein is operably linked to P_{LAC}.

The disclosed bacteriophage λ can infect gram negative bacteria, such as, but not limited to, an enteric bacteria. In one specific, non-limiting example the bacteria are *E. coli.* The *E. coli* can include a gene encoding wild-type D on a plasmid, such that wild-type D is expressed. In some embodiments, the gene encoding wild-type D in the bacteria is operably linked to an inducible promoter. In a non-limiting example, the inducible promoter is the arabinose promoter (P_{BAD}). In some embodiments, the host cell can be treated with arabinose to activate this promoter, to titrate a sufficient amount of wild-type D protein, so that it is incorporated in the resulting bacteriophage λ. Exemplary conditions are provided in the examples section.

In some embodiments, *fhuA* is genetically inactivated in the chromosome of the host bacterial cell. In other embodiments, *lamB* is genetically inactivated in the chromosome of the host bacterial cell. This inhibits inactivation of bacteriophage λ particles by LamB protein present in the debris of lysates following prophage induction and cell lysis. In further embodiments, both*fhuA* and *lamB* are inactivated in the chromosome of the host bacterial cell. The host cell can be an *E. coli.*

In some embodiments, the S gene mutation, *Sam7,* is included in the λ prophage to prevent holin synthesis. In the absence of lysis, the bacteriophage λ *Sam7* particles remain trapped in the host cell and can be pelleted by centrifugation and concentrated to smaller volumes. Usually, chloroform can be added to this smaller volume of cells to cause lysis generating higher concentrations of phage particles in the resulting lysate. A liter of the initial culture can produce 10¹³ to 10¹⁴ phage. By concentrating the cells before lysis, the phage released are in a smaller volume. However, this concentrated crude lysate also becomes concentrated for cellular proteins. The phage in this lysate can be further purified away from the other cellular contents by precipitation with polyethylene glycol (PEG), and the pelleted phage solubilized in buffer (See, for example, Protocol 6 In: Molecular Cloning, A Laboratory Manual. Sambrook J & Russell D.W. Cold Spring Harbor, NY: Cold Spring Harbor Laboratory Press, 3rd Edition, 2001). In some embodiments, the bacteriophage λ can be further purified by CsCl density gradient ultra-centrifugation followed by dialysis to remove the CsCl (See Protocol 8. In: Molecular Cloning, A Laboratory Manual: Sambrook J & Russell D.W. Cold Spring Harbor, NY: Cold Spring Harbor Laboratory Press, 3rd Edition, 2001). The bacteriophage λ particle yield can be further characterized by measuring its efficiency of plaque formation and/or by quantitative PCR analysis of phage DNA present in the particles.

In other embodiments, the disclosed bacteriophage λ can be introduced into bacteria and integrate into the bacterial chromosome as a prophage. These bacterial cells can be propagated *in vitro*, such as in LB broth. To collect the phage displaying the heterologous antigen on the surface, the lytic phase is induced. For example, the bacterial cells can be heated to induce the lytic phase.

In one non-limiting method, bacterial cells are grown to a fixed OD of about 0.3 at about 32°C in a LB-broth. Then cells then are shifted to about 42°C for about 30 minutes for thermal induction. This allows the prophage to undergo lytic cycle producing virions.

In some non-limiting examples, when complementation of D-protein is used, 0.2% arabinose is added at the time when the culture is shifted to about 42°C for thermal induction. After about 30 minutes the temperature is adjusted to about 37°C and the culture growth is continued, such as for additional about 3 to about 4 hours.

In further non-limiting examples, the cells then are concentrated to a small volume and then lysed either by freeze thaw or by treating with about 10% chloroform. Released phage particles are then separated and purified.

Formation of a plaque on a lawn of bacteria starts by a single cell infected by a phage particle, with lysis and release of progeny phage. Subsequently, adjacent cells are infected by the released phage, leading to more cycles of lysis. A plaque indicates that the phage must have gone through several lytic cycles to produce at least ~10⁷ particles to make a visible plaque. Suspension cultures of bacterial cells can also be used. In some embodiments, such as when the host cell includes an inducible promoter operably linked to a nucleic acid sequence encoding a wild-type D protein in the bacterial chromosome or on a plasmid, the host cells are cultured in the presence of the inducer. In one embodiment, the inducible promoter is P_{BAD} and the inducer is arabinose.

In some embodiments, when wild type D protein is expressed in the host cell form an inducible promoter, such as P_{BAD} or P_{LAC}, expression is induced from the promoter, such as by adding arabinose or lactose. In more embodiments, expression of wild type D protein is induced at the same time as thermal induction of phage production.

In some embodiments, when wild-type D protein is expressed in the host cells from PBAD, about 0.2 %to about 0.00625% arabinose is added to the host cells, such as in LB broth. In other embodiments, about 0.2% to about 0.005% arabinose is added to the host cells, such as in LB broth. In further embodiments, about 0.2 % to about 0.1% arabinose is added to the host cells, such as in LB broth. In a particular non-limiting example, about 0.2% arabinose is added to the host cells.

Bacteriophage particles can be produced by providing a permissive host bacterial cell, such as *E. coli,* wherein the permissive host bacterial cell provides growth of bacteriophage λ and further includes the cIₜ₅857 repressor. The host bacterial cell is then exposed to conditions such that bacteriophage particle expression and assembly is initiated, and the bacteriophage particles can be recovered from the culture. The bacteriophage λ particles are then isolated, and optionally purified, for use in immunogenic compositions.

### Immunogenic Compositions and Methods of Inducing an Immune Response

Methods are disclosed herein for inducing an immune response to an antigen. These methods include administering to the subject an immunogenic composition that includes an effective amount of a bacteriophage λ as disclosed herein, wherein the head of the bacteriophage λ includes the heterologous antigen, to induce the immune response. The immunogenic compositions disclosed herein can be used in any subject, including those with a tumor, an infection with a pathogen, an autoimmune disease, or an allergy. However, the immunogenic compositions can also be administered to healthy subjects, such as those in need of vaccination, to induce an immune response. Thus, any subject can be treated using the immunogenic compositions disclosed herein. In some examples, such a composition includes a pharmaceutically acceptable carrier, such as water or saline. In some examples, the immunogenic composition includes an adjuvant. In some embodiments, an adjuvant is not required. Without being bound by theory, the λ phage particle itself may act as the adjuvant. In some examples, the composition is sterile. The subject can have a specific disease or disorder or can be at risk of developing the disease or disorder. Treating a disorder or disease can include reducing or eliminating one or more symptoms of the condition.

The heterologous antigen included in the bacteriophage λ is selected for inducing an immune response in a particular subject of interest, such as a subject with a disease condition, such as a tumor, allergy, or an infection. The subject can be healthy, but an immune response would be advantageous, such as to reduce a subsequent infection.

The subject can be a human or a veterinary subject. Thus, subjects also include animals such as household pets (such as dogs, cats, rabbits and ferrets), livestock or farm animals (such as cows, pigs, sheep, chickens and other poultry), horses (such as thoroughbred, Arabian or Saddlebred horses), laboratory animals (such as mice, rats, rabbits, bats, etc.), zoo or wild animals (such as giraffes, zebras, lions and tigers) and birds (such as chickens, turkeys, pigeons, parrots and parakeets). Subjects also include fish and other aquatic species. In one example the subject is a mammal. In one example the subject is a human.

The disclosed bacteriophage λ can be administered systemically or locally. The particular mode selected can depend upon the particular active agent selected, the particular condition being treated, and the dosage required for therapeutic efficacy. Any mode of administration can be utilized that is medically acceptable, such that the desired response is induced without causing clinically unacceptable adverse effects. One mode of administration is a parenteral route. The term "parenteral" includes subcutaneous injections, intravenous, intramuscular, intraperitoneal, intra sternal injection or infusion techniques. Other modes of administration include oral, mucosal, rectal, vaginal, sublingual, intranasal, intratracheal, inhalation, ocular, and transdermal. One exemplary mode of administration is intratumoral. Additional modes of administration are to a mucosa, such as nasal, vaginal or rectal administration.

Tests for diagnosing the conditions are known. These laboratory tests include without limitation microscopic analyses, cultivation dependent tests (such as cultures), nucleic acid detection tests, and antigen detection tests. These include wet mounts, stain-enhanced microscopy, immune microscopy (*e.g*., FISH), hybridization microscopy, particle agglutination, enzyme-linked immunosorbent assays (ELISAs), urine screening tests, DNA probe hybridization, and serologic test. A practitioner can take a medical history and conduct a complete physical examination in addition to running the laboratory tests to select a subject for treatment.

In some embodiments, the subject will benefit from immune stimulation, particularly and an antigen specific immune response. Such a response may be a humoral or a cellular immune response. A subject can be treated that has a tumor (a solid tumor or non-solid tumor such as a lymphoma or leukemia), an infection (such as bacterial, viral, parasitic, nematode, or fungal infection), an autoimmune disorder, an allergy or allergic condition.

In some embodiments, the subject has a tumor or is at risk for developing a tumor. These subjects include, for instance, subjects having a genetic abnormality that has been demonstrated to be associated with a higher likelihood of developing a cancer, subjects having a familial disposition to cancer, subjects exposed to cancer causing agents (i.e., carcinogens) such as tobacco, asbestos, or other chemical toxins, and subjects previously treated for cancer and in apparent remission. In some embodiments these subjects are administered a therapeutically effective amount of an immunogenic composition that includes and effective amount of a disclosed bacteriophage λ, wherein the heterologous antigen tumor antigen. Suitable tumor antigens are disclosed above. Optionally, the subject can be further administered an additional chemotherapeutic agent, radiation therapy, surgery, or biologic, such as a monoclonal antibody. Such additional therapies can be administered before, concurrently with, or after administration of the bacteriophage λ provided herein. The subject can be administered a checkpoint inhibitor, such as an antibody that specifically binds CTLA-4, an antibody that specifically binds PD-1, an antibody that specifically binds VEGF, an antibody that specifically binds EGFR, or an antibody that specifically binds PD-L1. In some examples, the subject is further administered a steroid. In some examples, the disclosed methods further include administering to the subject with a cancer one or more biologics, such as monoclonal antibodies, such as a therapeutically effective amount of one or more of: 3F8, Abagovomab, Adecatumumab, Afutuzumab, Alacizumab , Alemtuzumab, Altumomab pentetate, Anatumomab mafenatox, Apolizumab, Arcitumomab, Bavituximab, Bectumomab, Belimumab, Besilesomab, Bevacizumab, Bivatuzumab mertansine, Blinatumomab, Brentuximab vedotin, Cantuzumab mertansine, Capromab pendetide, Catumaxomab, CC49, Cetuximab, Citatuzumab bogatox, Cixutumumab, Clivatuzumab tetraxetan, Conatumumab, Dacetuzumab, Detumomab, Ecromeximab, Eculizumab, Edrecolomab, Epratuzumab, Ertumaxomab, Etaracizumab, Farletuzumab, Figitumumab, Galiximab, Gemtuzumab ozogamicin, Girentuximab, Glembatumumab vedotin, Ibritumomab tiuxetan, Igovomab, Imciromab, Intetumumab, Inotuzumab ozogamicin, Ipilimumab, Iratumumab, Labetuzumab, Lexatumumab, Lintuzumab, Lorvotuzumab mertansine, Lucatumumab, Lumiliximab, Mapatumumab, Matuzumab, Mepolizumab, Metelimumab, Milatuzumab, Mitumomab, Morolimumab, Nacolomab tafenatox, Naptumomab estafenatox, Necitumumab, Nimotuzumab, Nofetumomab merpentan, Ofatumumab, Olaratumab, Oportuzumab monatox, Oregovomab, Panitumumab, Pemtumomab, Pertuzumab, Pintumomab, Pritumumab, Ramucirumab, Rilotumumab, Rituximab, Robatumumab, Satumomab pendetide, Sibrotuzumab, Sonepcizumab, Tacatuzumab tetraxetan, Taplitumomab paptox, Tenatumomab, TGN1412, Ticilimumab (tremelimumab), Tigatuzumab, TNX-650, Trastuzumab, Tremelimumab, Tucotuzumab celmoleukin, Veltuzumab, Volociximab, Votumumab, and Zalutumumab. In some examples, the disclosed methods further include administering to the subject with a cancer one or more chemotherapeutic agents, such as a therapeutically effective amount of one or more mitotic inhibitors, alkylating agents (such as nitrogen mustards (*e.g*., mechlorethamine, cyclophosphamide, melphalan, uracil mustard or chlorambucil), alkyl sulfonates (*e.g.*, busulfan), nitrosoureas (*e.g.,* carmustine, lomustine, semustine, streptozocin, or dacarbazine)), anti-metabolites (such as folic acid analogs (such as methotrexate), pyrimidine analogs (such as 5-FU or cytarabine), and purine analogs, such as mercaptopurine or thioguanine), intercalating antibiotics, growth factor inhibitors, cell cycle inhibitors, enzymes, topoisomerase inhibitors, biological response modifiers, anti-hormones (*e.g*., anti-androgens), anti-angiogenesis agents, vinca alkaloids (such as vinblastine, vincristine, or vindesine), epipodophyllotoxins (such as etoposide or teniposide), antibiotics (such as dactinomycin, daunorubicin, doxorubicin, bleomycin, plicamycin, or mitomycin C), enzymes (such as L-asparaginase), platinum coordination complexes (such as cis-diamine-dichloroplatinum II also known as cisplatin), substituted ureas (such as hydroxyurea), methyl hydrazine derivatives (such as procarbazine), adrenocrotical suppressants (such as mitotane and aminoglutethimide), hormones or antagonists (such as adrenocorticosteroids (such as prednisone), progestins (such as hydroxyprogesterone caproate, medroxyprogesterone acetate, and magestrol acetate), estrogens (such as diethylstilbestrol and ethinyl estradiol), antiestrogens (such as tamoxifen), and androgens (such as testerone proprionate and fluoxymesterone)), and/or immunomodulators (such as AS-101 (Wyeth-Ayerst Labs.), bropirimine (Upjohn), gamma interferon (Genentech), GM-CSF (granulocyte macrophage colony stimulating factor), IL-2, human immune globulin, IMREG, SK&F 106528, and TNF)).

The subject can have, or be at risk for developing, a hematological tumor. Examples of hematological tumors that can be treated using the methods disclosed herein include leukemias, such as acute leukemias (such as 11q23-positive acute leukemia, acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

The subject can have, or be at risk for developing, a solid tumor. Examples of solid tumors that can be treated using the methods disclosed herein include sarcomas and carcinomas, such as fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyrgioma, ependymoma, pinealoma, hemangioblastoma, acoustic neuroma, oligodendroglioma, meningioma, melanoma, neuroblastoma and retinoblastoma). In several examples, a tumor is melanoma, lung cancer, lymphoma breast cancer or colon cancer. In other examples, the tumor is a glioblastoma.

Thus, in some examples, the methods decrease the size, volume and/or weight of a tumor by at least 10%, at least 20%, at least 30%, at least 50%, at least 50%, at least 75%, at least 90%, at least 95%, at least 98%, at least 99% or 100%, for example relative to the size, volume and/or weight of the tumor prior to treatment. In some examples, the methods decrease the size, volume and/or weight of a metastasis by at least 10%, at least 20%, at least 30%, at least 50%, at least 50%, at least 75%, at least 90%, at least 95%, at least 98%, at least 99% or 100%, for example relative to the size, volume and/or weight of the metastasis prior to treatment. In some examples, the methods increase the survival time of a subject with cancer by at least 3 months, at least 6 months, at least 9 months, at least 12 months, at least 18 months, at least 24 months, at last 36 months, at least 48 months, or at least 60 months, for example relative to the survival time in an absence of the treatment provided herein. In some examples, combinations of these effects are achieved.

The subject can have an infection or be at risk of contracting an infection due to a risk of exposure to a pathogen. These subjects include those that live in an area where such pathogens are known to exist and where such infections are common. These subjects also include those that engage in high risk activities such as sharing of needles, engaging in unprotected sexual activity, routine contact with infected samples of subjects (*e.g.*, medical practitioners), people who have undergone surgery, including but not limited to abdominal surgery. Thus, the subject may have or may be at risk of developing an infection such as a bacterial infection, a viral infection, a fungal infection, a parasitic infection, nematode infection, or a mycobacterial infection. The subject can have a viral infection, a fungal infection, a parasitic infection, nematode infection, or a mycobacterial infection. In some embodiments, these subjects are administered a therapeutically effective amount of an immunogenic composition disclosed herein, wherein the agent of interest is an antigen from a pathogen. In some examples, the subjects are further administered one or more anti-infective agents such as anti-bacterial agents, anti-viral agents, anti-fungal agents, anti-parasitic agents, anti-nematode agents, and anti-mycobacterial agents.

In some embodiments, the subject has or is at risk of having, a bacterial infection, such as an *E. coli* infection, a *Staphylococcal* infection, a *Streptococcal* infection, a *Pseudomonas* infection, *Clostridium difficile* infection, *Legionella* infection, *Pneumococcus* infection, *Haemophilus* infection, *Klebsiella* infection, *Enterobacter* infection, *Citrobacter* infection, *Neisseria* infection, *Shigella* infection, *Salmonella* infection, *Listeria* infection, *Pasteurella* infection, *Streptobacillus* infection, *Spirillum* infection, *Treponema* infection, *Actinomyces* infection, *Borrelia* infection, *Corynebacterium* infection, *Nocardia* infection, *Gardnerella* infection, *Campylobacter* infection, *Spirochaeta* infection, *Proteus* infection, *Bacteriodes* infection, *H. pylori* infection, or *B. anthracis* infection.

In some embodiments, the subject has or is at risk of having, a mycobacterial infection such as a tuberculosis or leprosy, respectively caused by *M. tuberculosis* or *M. leprae.*

In additional embodiments, the heterologous antigen is a viral antigen. In some embodiments, the subject has or is at risk of having, a viral infection such as a severe acute respiratory coronavirus (SARS CoV)-2 infection, Herpes simplex virus 1 infection, a Herpes simplex virus 2 infection, cytomegalovirus infection, hepatitis A virus infection, hepatitis B virus infection, hepatitis C virus infection, human papilloma virus infection, Epstein Barr virus infection, rotavirus infection, adenovirus infection, influenza virus infection, influenza A virus infection, H1N1 (swine flu) infection, respiratory syncytial virus infection, measles, varicella-zoster virus infections, small pox infection, monkey pox infection, SARS infection or avian flu infection.

In other embodiments, the heterologous antigen is a *Candidiasis,* ringworm, histoplasmosis, blastomycosis, paracoccidioidomycosis, crytococcosis, aspergillosis, chromomycosis, mycetoma infections, pseudallescheriasis, or tinea versicolor infection. In yet other embodiments, the subject has or is at risk of having, a parasite infection such as an infection with an amoeba, *Trypanosoma cruzi, Fascioliasis, Leishmaniasis, Plasmodium infections, Onchocerciasis, Paragonimiasis, Trypanosoma brucei infection, Pneumocystis, Trichomonas vaginalis infection, Taenia infection, Hymenolepsis, Echinococcus, Schistosomiasis, neurocysticercosis, Necator americanus* infection, *or Trichuris trichuria* antigen. In these embodiments, the subject has or is at risk of having an infection with these agents.

Combinations of heterologous antigens are also of use. In some embodiments, the head of the bacteriophage λ includes 2, 3, 4, or 5 heterologous antigens. The heterologous antigens can be from the same, or different, infections agents. The heterologous antigens can be from the same, or different tumors.

Thus, in some examples, the methods increase an immune response (such as a Th1 response) against one or more infectious agents, such as an increase of at least 10%, at least 20%, at least 30%, at least 50%, at least 50%, at least 75%, at least 90%, at least 95%, at least 100%, at least 200%, at least 300%, at least 400%, or at least 500%, for example relative to the immune response prior to treatment.

The heterologous antigen can be an allergen. In these embodiments, the subject can have, or be at risk of developing, an allergy or asthma. An allergy is an acquired hypersensitivity to an allergen. Allergic conditions include but are not limited to eczema, allergic rhinitis or coryza, hay fever, bronchial asthma, urticaria (hives) and food allergies, and other atopic conditions.

The heterologous antigen can stimulate a Th1 response, immunoinhibitory or immunosuppressant agents including agents that inhibit a Th2 response, anti-inflammatory agents, leukotriene antagonists, IL-4 receptors, anti-IL-4 antibodies, IL-4 antagonists, anti-IL-5 antibodies, soluble IL-13 receptor-Fc fusion proteins, anti-IL-9 antibodies, CCR3 antagonists, CCR5 antagonists, VLA-4 inhibitors, and other downregulators of IgE, such as but not limited to anti-IgE, cytokines such as IL-12 and IFN-gamma, steroids including corticosteroids such as prednisolone. In some examples, the methods increase an immune response against the heterologous antigen presented by the bacteriophage λ, such as an increase at least 10%, at least 20%, at least 30%, at least 50%, at least 50%, at least 75%, at least 90%, at least 95%, at least 100%, at least 200%, at least 300%, at least 400%, or at least 500%, for example relative to the immune response prior to treatment.

The foregoing lists are not intended to be exhaustive but rather exemplary. Those of ordinary skill in the art will identify other diseases that can be treated. Generally, a therapeutically effective amount of the immunogenic composition is administered to the subject, in a dosage sufficient to provide desirable results, such as an immune response. The therapeutically effective amount can vary with the particular condition being treated or prevented, the age and physical condition of the subject being treated, the severity of the condition, the duration of the treatment, the nature of the concurrent or combination therapy (if any), the specific route of administration and like factors within the knowledge and expertise of the health practitioner. The bacteriophage λ can be administered with or without an adjuvant.

In specific non-limiting examples, an adjuvant is not utilized. Thus, the bacteriophage λ can be administered in the absence of an adjuvant. In some examples, the immunogenic composition does not include an adjuvant. In additional examples, the subject is not administered an adjuvant, either as part of the immunogenic composition or separately.

In other embodiments, an adjuvant is used with the bacteriophage λ. Thus, in some examples a bacteriophage λ containing composition includes an adjuvant. Adjuvants, such as aluminum hydroxide (ALHYDROGEL^{®}, available from Brenntag Biosector, Copenhagen, Denmark and AMPHOGEL^{®}, Wyeth Laboratories, Madison, NJ), Freund's adjuvant, MPL^{™} (3-O-deacylated monophosphoryl lipid A; Corixa, Hamilton, IN), IL-12 (Genetics Institute, Cambridge, MA) TLR agonists (such as TLR-9 agonists), CpG oligodeoxynucleotides, and other suitable adjuvants well known in the art.

An adjuvant can include a Tol-like receptor (TLR) agonist. For example, the TLR agonist can be a TLR-4 agonist such as a synthetic derivative of lipid A (see, *e.g.,* WO 95/14026, and WO 01/46127) an alkyl Glucosaminide phosphate (AGP; see, *e.g.,* WO 98/50399 or U.S. Pat. No. 6,303,347; 6,764,840). Other suitable TLR-4 ligands, capable of causing a signaling response through TLR-4 are, for example, lipopolysaccharide from gram-negative bacteria and its derivatives, or fragments thereof, in particular a non-toxic derivative of LPS (such as 3D-MPL). Other suitable TLR agonists are: heat shock protein (HSP) 10, 60, 65, 70, 75 or 90; surfactant Protein A, hyaluronan oligosaccharides, heparan sulphate fragments, fibronectin fragments, fibrinogen peptides and β-defensin-2, and muramyl dipeptide (MDP). In one embodiment the TLR agonist is HSP 60, 70 or 90. Other suitable TLR-4 ligands are as described in WO 2003/011223 and in WO 2003/099195.

Additional TLR agonists (such as an agent capable of causing a signaling response through a TLR signaling pathway) are also useful as adjuvants, such as agonists for TLR2, TLR3, TLR7, TLR8 and/or TLR9. Accordingly, in one embodiment, a composition containing a disclosed bacteriophage λ further includes an adjuvant which is selected from the group consisting of: a TLR-1 agonist, a TLR-2 agonist, TLR-3 agonist, a TLR-4 agonist, TLR-5 agonist, a TLR-6 agonist, TLR-7 agonist, a TLR-8 agonist, TLR-9 agonist, or a combination thereof.

In one embodiment, a TLR agonist is used that is capable of causing a signaling response through TLR-1, for example one or more of from: Tri-acylated lipopeptides (LPs); phenol-soluble modulin; Mycobacterium tuberculosis LP; S-(2,3-bis(palmitoyloxy)-(2-RS)-propyl)-N-palmitoyl-(R)-Cys-(S)-Ser-(S)-L- ys(4)--OH, trihydrochloride (Pam3Cys) LP which mimics the acetylated amino terminus of a bacterial lipoprotein and OspA LP from Borrelia burgdorferi. In another embodiment, a TLR agonist is used that is capable of causing a signaling response through TLR-2, such as one or more of a lipoprotein, a peptidoglycan, a bacterial lipopeptide from M tuberculosis, B burgdorferi or T pallidum; peptidoglycans from species including Staphylococcus aureus; lipoteichoic acids, mannuronic acids, Neisseria porins, bacterial fimbriae, Yersina virulence factors, CMV virions, measles haemagglutinin, and zymosan from yeast. In some embodiments, a TLR agonist is used that is capable of causing a signaling response through TLR-3, such as one or more of double stranded RNA (dsRNA), or polyinosinic-polycytidylic acid (Poly IC), a molecular nucleic acid pattern associated with viral infection. In further embodiments, a TLR agonist is used that is capable of causing a signaling response through TLR-5, such as bacterial flagellin. In additional embodiments, a TLR agonist is used that is capable of causing a signaling response through TLR-6, such as one or more of mycobacterial lipoprotein, di-acylated LP, and phenol-soluble modulin. Additional TLR6 agonists are described in PCT Publication No. WO 2003/043572. In an embodiment, a TLR agonist is used that is capable of causing a signaling response through TLR-7, such as one or more of a single stranded RNA (ssRNA), loxoribine, a guanosine analogue at positions N7 and C8, or an imidazoquinoline compound, or derivative thereof. In one embodiment, the TLR agonist is imiquimod. Further TLR7 agonists are described in PCT Publication No. WO 2002/085905. In some embodiments, a TLR agonist is used that is capable of causing a signaling response through TLR-8. Suitably, the TLR agonist capable of causing a signaling response through TLR-8 is a single stranded RNA (ssRNA), an imidazoquinoline molecule with anti-viral activity, for example resiquimod (R848); resiquimod is also capable of recognition by TLR-7. Other TLR-8 agonists which can be used include those described in PCT Publication No. WO 2004/071459.

In further embodiments, an adjuvant includes a TLR agonist capable of inducing a signaling response through TLR-9. For example, the adjuvant can include HSP90, bacterial or viral DNA, and/or DNA containing unmethylated CpG nucleotides (*e.g.*, a CpG oligonucleotide). For example, CpG-containing oligonucleotides induce a predominantly Th1 response. Such oligonucleotides are well known and are described, for example, in PCT Publication Nos. WO 95/26204, WO 96/02555, WO 99/33488 and U.S. Patent Nos. 5,278,302, 5,666,153, and. 6,008,200 and 5,856,462. Accordingly, oligonucleotides for use as adjuvants in the disclosed compositions include CpG containing oligonucleotides, for example, containing two or more dinucleotide CpG motifs. Also included are oligonucleotides with mixed internucleotide linkages.

Other adjuvants that can be used in immunogenic compositions include saponins, such as QS21. In some examples, saponins are used as an adjuvant, *e*.*g*., for systemic administration. Use of saponins (*e.g*., use of Quil A, derived from the bark of the South American tree Quillaja Saponaria Molina) as adjuvants is familiar to the person of ordinary skill in the art (see, *e.g.,* US 5,057,540 and EP 0 362 279 B1. EP 0 109 942 B1; WO 96/11711; WO 96/33739). The haemolytic saponins QS21 and QS17 (HPLC purified fractions of Quil A) have been described as potent systemic adjuvants, and the method of their production is disclosed in U.S. Pat. No. 5,057,540 and EP 0 362 279 B1.

Mineral salts such as an aluminum or calcium salts, in particular aluminum hydroxide, aluminum phosphate and calcium phosphate, can be used as adjuvants.

Another class of suitable Th1 biasing adjuvants for use in compositions includes outer membrane proteins (OMP)-based immunostimulatory compositions. OMP-based immunostimulatory compositions are particularly suitable as mucosal adjuvants, *e*.*g*., for intranasal administration. OMP-based immunostimulatory compositions are a genus of preparations of (OMPs, including some porins) from Gram-negative bacteria, *e*.*g*., Neisseria species, which are useful as a carrier or in compositions for immunogens, such as bacterial or viral antigens (see, *e.g.,* U.S. Pat. No. 5,726,292; U.S. Pat. No. 4,707,543). Further, proteosomes have the capability to auto-assemble into vesicle or vesicle-like OMP clusters of about 20 nm to about 800 nm, and to noncovalently incorporate, coordinate, associate (*e.g.,* electrostatically or hydrophobically), or otherwise cooperate with protein antigens (Ags), particularly antigens that have a hydrophobic moiety. Proteosomes can be prepared, for example, as described in the art (see, *e.g.,* U.S. Patent No. 5,726,292 or U.S. Patent No. 5,985,284; U.S. Published Patent Application No. 2003/0044425.).

Proteosomes are composed primarily of chemically extracted outer membrane proteins (OMPs) from Neisseria meningitidis (mostly porins A and B as well as class 4 OMP), maintained in solution by detergent (Lowell G H. Proteosomes for Improved Nasal, Oral, or Injectable Vaccines. In: Levine M M, Woodrow G C, Kaper J B, Cobon G S, eds, New Generation Vaccines. New York: Marcel Dekker, Inc. 1997; 193-206). Proteosomes can be formulated with a variety of antigens such as purified or recombinant proteins derived from viral sources. The gradual removal of detergent allows the formation of particulate hydrophobic complexes of approximately 100-200 nm in diameter (Lowell G H. Proteosomes for Improved Nasal, Oral, or Injectable Vaccines. In: Levine M M, Woodrow G C, Kaper J B, Cobon G S, eds, New Generation Vaccines. New York: Marcel Dekker, Inc. 1997; 193-206).

Combinations of different adjuvants can also be used. For example, QS21 can be formulated together with 3D-MPL. The ratio of QS21:3D-MPL will typically be in the order of 1:10 to 10:1; such as 1:5 to 5:1, and often substantially 1:1. Typically, the ratio is in the range of 2.5:1 to 1:1 3D-MPL:QS21 (such as AS01 (GlaxoSmithKline). Another combination adjuvant formulation includes 3D-MPL and an aluminum salt, such as aluminum hydroxide (such as AS04 (GlaxoSmithKline). When formulated in combination, this combination can enhance an antigen-specific Thl immune response.

In some instances, the adjuvant formulation a mineral salt, such as a calcium or aluminum (alum) salt, for example calcium phosphate, aluminum phosphate or aluminum hydroxide. In some embodiments, the adjuvant includes an oil and water emulsion, *e.g.,* an oil-in-water emulsion (such as MF59 (Novartis) or AS03 (GlaxoSmithKline). One example of an oil-in-water emulsion comprises a metabolisable oil, such as squalene, a tocol such as a tocopherol, *e.g.,* alpha-tocopherol, and a surfactant, such as sorbitan trioleate (Span 85) or polyoxyethylene sorbitan monooleate (Tween 80), in an aqueous carrier.

In some embodiments, the subject has a tumor, and an amount is administered that reduces the tumor volume or load (as for example determined by imaging the tumor). Effective amounts may also be assessed by the presence and/or frequency of tumor cells in the blood or other body fluid or tissue (e.g., a biopsy). If the tumor is impacting the normal functioning of a tissue or organ, then the effective amount may be assessed by measuring the normal functioning of the tissue or organ. The immunogenic composition can reduce tumor volume, and/or decrease metastasis.

In some embodiments, a therapeutically effective amount is the amount required to lessen or eliminate one or more, and preferably all, symptoms. For example, in a subject having an allergy or experiencing an asthmatic attack, an effective amount of an agent may be that amount that lessens or eliminates the symptoms associated with the allergy or the asthmatic attack. They may include sneezing, hives, nasal congestion, and labored breathing. Similarly, in a subject with an infection, an effective amount of an agent may be that amount that lessens or eliminate the symptoms associated with the infection. These may include fever and malaise. If the heterologous antigen is an antigen from a pathogen, the effective amount may be that amount that triggers an immune response against the antigen and preferably provides short and even more preferably long term protection against the pathogen from which the antigen derives.

The disclosed methods include a single administration, or multiple administrations. As an example, an immunogenic composition can be administered in a prime dose and a boost dose.

For prophylactic and therapeutic purposes, the immunogenic composition including the bacteriophage λ can be administered to the subject in a single bolus delivery, via continuous delivery (for example, continuous transdermal, mucosal or intravenous delivery) over an extended time period, or in a repeated administration protocol (for example, by an hourly, daily or weekly, repeated administration protocol). The therapeutically effective dosage of the immunogenic composition can be provided as repeated doses within a prolonged prophylaxis or treatment regimen that will yield clinically significant results to alleviate one or more symptoms or detectable conditions associated with a targeted disease or condition. Determination of effective dosages in this context is typically based on animal model studies followed up by human clinical trials and is guided by administration protocols that significantly reduce the occurrence or severity of targeted disease symptoms or conditions in the subject. Suitable models in this regard include, for example, murine, rat, porcine, feline, ferret, non-human primate, and other accepted animal model subjects known in the art. Alternatively, effective dosages can be determined using *in vitro* models (for example, immunologic and histopathologic assays). Using such models, only ordinary calculations and adjustments are required to determine an appropriate concentration and dose to administer a therapeutically effective amount of the immunogenic composition (for example, amounts that are effective to elicit a desired immune response or alleviate one or more symptoms of a targeted disease). In alternative embodiments, an effective amount or effective dose of the immunogenic composition may simply inhibit or enhance one or more selected biological activities correlated with a disease or condition, as set forth herein, for either therapeutic or diagnostic purposes.

In one embodiment, a suitable immunization regimen includes at least three separate inoculations with one or more immunogenic compositions including a bacteriophage λ, with a second inoculation being administered more than about two, about three to eight, or about four, weeks following the first inoculation. Generally, the third inoculation is administered several months after the second inoculation, and in specific embodiments, more than about five months after the first inoculation, more than about six months to about two years after the first inoculation, or about eight months to about one year after the first inoculation. Periodic inoculations beyond the third are also desirable to enhance the subject's "immune memory." The adequacy of the vaccination parameters chosen, *e*.*g*., formulation, dose, regimen and the like, can be determined by taking aliquots of serum from the subject and assaying antibody titers during the course of the immunization program. If such monitoring indicates that vaccination is sub-optimal, the subject can be boosted with an additional dose of immunogenic composition, and the vaccination parameters can be modified in a fashion expected to potentiate the immune response. It is contemplated that there can be several boosts, and that each boost can include a bacteriophage λ including the same or different heterologous antigens.

For prime-boost protocols, the prime can be administered as a single dose or multiple doses, for example two doses, three doses, four doses, five doses, six doses or more can be administered to a subject over days, weeks or months. The boost can be administered as a single dose or multiple doses, for example two to six doses, or more can be administered to a subject over a day, a week or months. Multiple boosts can also be given, such one to five, or more. Different dosages can be used in a series of sequential inoculations. For example, a relatively large dose in a primary inoculation and then a boost with relatively smaller doses. The immune response against the selected heterologous antigen can be generated by one or more inoculations of a subject with an immunogenic composition disclosed herein.

For both prime and boost, actual dosage can vary according to factors such as the disease indication and particular status of the subject (for example, the subject's age, size, fitness, extent of symptoms, susceptibility factors, and the like), time and route of administration, other drugs or treatments being administered concurrently, as well as the specific pharmacology of the bacteriophage λ for eliciting the desired activity or immune response in the subject. Dosage regimens can be adjusted to provide an optimum prophylactic or therapeutic response. An effective amount is also one in which any toxic or detrimental side effects of the disclosed antigen and/or other biologically active agent is outweighed in clinical terms by therapeutically beneficial effects.

Immunogenic compositions are provided that include a therapeutically effective amount of a bacteriophage λ and a pharmaceutically acceptable carrier, such as a non-naturally occurring carrier. Exemplary non-limiting therapeutically effective doses of the bacteriophage λ 0.01 to 10.0 µg/gm, such as 0.1 to 1.0 µg/gm. Additional exemplary therapeutically effective doses are 0.25 to 0.75 µg/gm such as 0.4 to 0.6 µg/gm. One of skill in the art can readily determine the absolute dose based on the weight of the subject, such as a human subject weighing from 50 kg to 100kg, such as a 70 kg subject. Thus, for a 70 kg subject, 700 µg to 700 mg, 7 mg to 70 mg, 17.5 mg to 52.5 mg, or 28 mg to 42 mg, for example 35 mg, can be administered. Exemplary doses are about 10⁷ to about 10¹⁴ phage particles per dose, about 10⁸ to about 10¹⁴ phage particles per dose, about 10⁹ to about 10¹⁴ phage particles per dose, about 10¹⁰ to about 10¹⁴ phage particles per dose, about 10¹¹ to about 10¹⁴ phage particles per dose, about 10¹² to about 10¹⁴ phage particles per dose, or about 10¹³ to about 10¹⁴ phage particles per dose. Exemplary doses include about 10⁷, 10⁸, 10⁹, 10¹⁰, 10¹¹, 10¹², or 10¹³ phage particles per dose. In other embodiments, the dose is about 10⁹ phage particles per dose.

In further embodiments, the immunogenic composition is formulated for intranasal administration. Thus, the method can include administering the therapeutically effective amount of the composition intra-nasally. For intranasal administration, the compositions can be used in intranasal drops, aerosols, and mists, and employ a protocol or device adapted to this delivery. An applicable method for direct delivery upper third of the nasal cavity is intranasal intubation. Applicable delivery methods include aerosols, power aerosols, spray aerosols, metered spray and suspension spays.

The components of the immunogenic compositions are comingled in a manner that precludes interaction that would substantially impair their desired pharmaceutical efficiency. Suitable buffering agents include acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); and parabens (0.01-0.25% w/v).

The disclosed immunogenic compositions can be formulated for oral administration. Such carriers enable formulation as tablets, pills, dragees, capsules, liquids, gels, syrups, slurries, films, suspensions and the like, for oral ingestion by a subject to be treated. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). Optionally the oral formulations may also be formulated in saline or buffers for neutralizing internal acid conditions or may be administered without any carriers. These compositions include capsules made of gelatin, as well as soft, sealed capsules made of gelatin and a plasticizer, such as glycerol or sorbitol. The capsules can contain the disclosed bacteriophage λ suspended in suitable liquids, such as aqueous solutions, buffered solutions, fatty oils, liquid paraffin, or liquid polyethylene glycols. In addition, stabilizers may be added. All formulations for oral administration should be in dosages suitable for such administration. For buccal administration, the compositions may take the form of tablets or lozenges formulated in conventional manner.

For administration by inhalation, the immunogenic composition can be delivered in the form of an aerosol spray presentation from pressurized packs or a nebulizer, with the use of a suitable propellant, such as dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount.

When it is desirable to deliver the immunogenic composition systemically, it may be formulated for parenteral administration by injection, e.g., by bolus injection or continuous infusion. Formulations for injection may be presented in unit dosage form, e.g., in ampoules or in multi-dose containers. Pharmaceutical parenteral formulations include aqueous solutions of the ingredients. Aqueous injection suspensions may contain substances which increase the viscosity of the suspension, such as sodium carboxymethyl cellulose, sorbitol, or dextran. Alternatively, suspensions of particles may be prepared as oil-based suspensions. Suitable lipophilic solvents or vehicles include fatty oils such as sesame oil, or synthetic fatty acid esters, such as ethyl oleate or triglycerides.

The disclosed immunogenic compositions also may be in powder form or lyophilized form for constitution with a suitable vehicle, e.g., sterile pyrogen-free water, before use. The immunogenic compositions may also be formulated in rectal or vaginal compositions such as suppositories or retention enemas, for example containing conventional suppository bases such as cocoa butter or other glycerides.

### Kits

Kits are provided that include one or more of the disclosed bacteriophage λ. The bacteriophage λ may be supplied in a container in the kit, and optionally the kit can include one or more antigens, adjuvants, or other therapeutic agents. The particles may be provided in a buffer or in a lyophilized form. In several embodiments the container has a sterile access port (for example the container may be an intravenous solution bag or a vial having a stopper pierceable by a hypodermic injection needle). The kit can include a means for administration, such as a needle and/or syringe. Instructions for use, in in written form or on a computer-readable medium, also can be included in the kit. The label or package insert can indicate that the composition is used for treating the particular condition. The package insert typically includes instructions customarily included in commercial packages of therapeutic products that contain information about the indications, usage, dosage, administration, contraindications and/or warnings concerning the use of such therapeutic products. The kits may also include additional components to facilitate the particular application for which the kit is designed. For example, the kits may additionally include buffers and other reagents routinely used for the practice of a particular method. The kit may include several doses, such as suitable for use in a prime boost strategy.

The present disclosure is illustrated by the following non-limiting examples.

### EXAMPLES

### Example 1

### Construction of λ and the host for making display phages

Phage and bacterial strains described below are listed in Table 1B. Strain SJ_XTL175 was used as the starting strain for the construction of the host bacterium used here. SJ_XTL175 is an MG1655 derivative, which is described in Li et al. (2016), *Scientific Reports, 6.* https://doi.org/10.1038/srep39076. SJ_XTL175 contains the following allelic modifications: Δ*lacI lacY*_{A177C} Δ*araE araFGH<>spec^{R} araD<>tetA-sacB-amp.* SJ_XTL175 was further modified by recombineering to replace the arabinose operon genes *araB araA araD<>tetA-sacB-amp* with gene *D* of λ. The *araC* gene remains intact allowing AraC regulation of the λ *D* gene. In this construct, the ATG start codon of *araB* and the rest of *araB araA araD<>tetA-sacB-amp* DNA to the *araD* TAA stop codon is replaced with gene *D* of λ from its ATG start codon to its TAA stop codon (FIG. 1). This places *D* of λ under AraC regulation and arabinose inducible control (FIG. 2). The other mutations (Δ*lacI lacY* A177C Δ*araE araFGH<>*spec) already present in SJ_XTL175 confer on this new strain (XTL981) the ability to tune the λ D-protein expression level in every cell in response to the extracellular arabinose concentration (Li et al., 2016, *supra*).

Strain XTL981 described above has been further engineered to optimize λ phage production and the generation of λ particles carrying D-fusion proteins. First, XTL981 was infected with λ*cI857 Sam7* phage to generate lysogens, and standard techniques were used to identify a lysogen in which a single phage genome was integrated into its native attachment site (*attB*) on the bacterial chromosome (Powell et al., Nucleic Acids Research 1994;22(25):5765-5766.).

Next, additional changes were made to the bacterial chromosome and to the prophage λ*cI857 Sam7* genome using recombineering technologies. As mentioned above the *lamB* gene was deleted to ensure that λ particles released in lysates from the XTL981 lysogen following induction would not be lost by adsorbing to LamB receptors present in the lysate. The *fhuA* gene, encoding the phage T1 receptor protein was deleted to avoid the spread of the frequently airborne T1 bacterial virus. T1 frequently contaminates cell cultures during large-scale fermentation preparations, completely destroying cultures, and delaying further use of the fermentation complex. The temperate phage Φ80 integration site *att80* was deleted to avoid invasion by Φ80, a common contaminant in *E. coli* cell cultures. The nonessential λ genes *ea47, ea59,* and *ea31* were precisely removed in a DNA segment of 4285bp from the b region of the λ prophage (see Appendix II of Lambda II edited by Hendrix RW, Roberts J, Stahl F, Weisberg R, Cold Spring Harbor, NY: Cold Spring Harbor Laboratory; 1983). This deletion provides room in the phage genome for cloning and encapsidation of DNA segments to be fused to the D gene or two other potential genes of λ, gene V and *stf,* described below. The major capsid *E* gene was modified by a single base change causing a Glu158 (GAG) to Lys158 (AAG) amino acid change, which enables λ particles to have a longer presence in the mammalian bloodstream (Merril et al., Proc Natl Acad Sci U S A. 1996 Apr 16;93(8):3188-92).

All of the genetic changes made to XTL981 were made by the same general method of recombineering (Li et al., Nucl. Acids Res. 41: e204 doi:10.1093/nar/gkt1075, 2013). A *tetA-sacB* cassette was inserted at the site on the bacterial chromosome or prophage genome to be deleted or altered by selecting for tetracycline resistance and ensuring that the drug resistant recombinants had become sensitive to sucrose because of the *sacB* gene. The entire *tetA-sacB* cassette was then removed by recombineering and replaced with a deletion or alteration of the targeted sequence selecting for sucrose resistance. The single strand DNA oligonucleotide carries the appropriate homology sequences to target the chromosome or prophage genome for recombination and also generate the genome change. Sequences of the oligonucleotides used for these genome deletions are described in Table 1A. These sequences define the point of the deletion by the symbol (Δ) and the sequences flanking the Δ define the exact position and extent of the deletion in the *E. coli* chromosome or prophage genome targeted.

XTL1026 is the strain containing all the modifications to the host and the λ prophage that are described above. The strain also contains pKM208, which is a pSC101-based plasmid that expresses Red functions Exo Bet and Gam from the *lac* promoter for recombineering (Murphy and Campellone, BMC Mol Biol. 2003 Dec 13;4:11). The D gene in the λ prophage was the target for creating *D* gene fusions to express fusion antigenic proteins joined at either the N- or C-terminus of λD. Two strains were derived from XTL1026 in which the λD gene was modified for generating λ*D* gene fusions. The counter-selectable *tetA-sacB* marker cassette was inserted just upstream or downstream of the D gene in the prophage by selecting for tetracycline resistance. These two Tet^{R} prophage strains are respectively, XTL1059 and XTL1048; both become sucrose sensitive because of the *sacB* gene function linked to the *tetA* marker. Using recombineering technology, the *tetA-sacB* cassette was replaced using PCR fragments or gblocks or ss-oligos containing the coding sequence of the foreign DNA fused to λ gene D. (Li et al., Nucleic Acids Res. 2013 Dec; 41(22): e204). These sucrose resistant recombinants were tetracycline sensitive and were engineered to fuse the foreign DNA to the D gene in the same reading frame. When placed at the N terminus, the fusion DNA has an upstream 5' ATG start codon, and when placed at the C terminus, the fusion DNA has a downstream 3' TAA stop codon. Additional DNA coding sequences are included so as to generate a five amino acid linker sequence (GlyGlyGlyGlySer (SEQ ID NO: 3)) between the λD protein and the foreign protein. Once fusions were generated, the λ*D*-fusion segments were amplified by PCR and sequenced to verify the accuracy of the gene fusions.

**Table 1A. Oligonucleotides**

| Oligo name | Oligo sequence* | Purpose | SEQ ID NO: |
|---|---|---|---|
| XT928 | CGGCGTAAACGCCTTATCCGGCCCAGGTTTTGCT AΔTCTATCTCCTGAGTCATTGCTTTTCTTTTTTCAC A | *lamB* deletion | 7 |
| XT924 | AAATGGGCACGGAAATCCGTGCCCCAAAAGAGA AAΔTGGTATATCTCTGATGTAAAGTGAATGATAA CGTA | *fhuA* deletion | 8 |
| XT992 | CTGGCTCAACCGTTATTGCTGAATTTGAATCGCT GΔGGTCAGTGCATTCACCACCACAAGCAGATAAT AAC | *att80* deletion | 9 |
| XT957 | ATTGCGGTTATTTTAATAAAATTAAGGGTTACTA TΔTGAGTCATAGAACTTCCATTATTCTCCTGAAGA TA | λ*ea59 ea31 b* deletion | 10 |
| XT934 | ACTCCGTGCCGCCGGACTGCGTGATGTTATTCTC CTTACTGCGGCCCATATCCACCTCAACCGGATCG AAGGC | λ*geneE* mutation | 11 |
| XT914 | CGCTTTTTATCGCAACTCTCTACTGTTTCTCCATA | *ara P_{BAD}* | 12 |
| | CCCGTTTTTTTGGATAGGAGGATGAAACGatgacgag caaagaaacctttacc | λgene *D* | |
| XT915 | TGAGTATAGCCTGGTTTCGTTTGATTGGCTGTGGT TTTATACAGTCATTAaacgatgctgattgccgttccg | *ara P_{BAD}* λ*gene D* | 13 |
| XT102 1 | CAGCCAATCAAACGAAACCAGGCTATACTCAAG CCTGGTTTTTTGATGGAgttccggatctgcatcgcag | *ara P_{BAD} λD* retrieval | 14 |
| XT102 2 | TGCAGAATCCCTGCTTCGTCCATTTGACAGGCAC ATTATGCAAGCATTGCtgtgataaactaccgcattaaagc | *ara P_{BAD} λD* retrieval | 15 |
| XT111 5 | GCCGCACAGGCGGCCTTTAGTGATGAAGGGTAA AGΔCCCTTACACTGGTGTGTTCAGCAAATCGTTA ACGG | *λD* deletion | 16 |
| MR27 3 | CGCAACTCTCTACTGTTTCTCCATACCCGTTTTTT TGGΔAATGACTGTATAAAACCACAGCCAATCAAA CGAAAC | *λD* deletion at arabinose locus | 17 |

| | | | |
|---|---|---|---|
| *In this table, the breakage-point between two homology arms in the oligonucleotides used in the study is indicated with the symbol Δ, but is not included any sequence identifier, to comply with patent requirements. Using these oligonucleotides, various genes that are listed in column 3 of this table were deleted. | | | |

**Table 1B. Strains**

| Strains | Relevant Genetic Markers |
|---|---|
| LE392 | *tyrT(supF58) glnV(supE44) hsdR514(r-m+) lacY1 galK2 galT22 metB1 trpR55* |
| XTL1212 | LE392 *araBAD<> λ* D /pLXT42 |
| SJ_XTL175 | MG1655 *lacY*A177C, *specR<>*Δara*FGH*, Δ*lacI* Δ*araE*, *araC⁺ P_{BAD} araB araA araD<>tet-sacB-amp* |
| XTL981 | SJ_XTL175 *araC⁺ P_{BAD}* - λ *D* |
| XTL1026 | MG1655 Δ*lacI*, *araFGH<>spec,* Δ*araE*, Δ*lamB*, *ΔfhuA, lacY* A177C *araC+P_{BAD}* -λ*D*, λ*CI857 Sam7 (geneE E158K,* Δ*ea59ea31*) /pKM208 a pSC101 based plasmid expresses Red functions *exo bet and gam* from the *lac* promoter for recombineering |
| XTL1048 | XTL1026 *D*-linker_*tet-sacB* The *tet-sacB* cassette is inserted with a linker coding sequence at λ D gene TAA stop /pKM208 |
| XTL1059 | XTL1026 *tet-sacB*-linker-*D* The *tet-sacB* cassette is inserted with a linker coding sequence at λ D gene ATG start /pKM208 |
| XTL1158 | XTL1026 cured of plasmid pKM208 |
| XTL1178 | XTL1158 *λD*+ pLXT42 |
| XTL1205 | XTL1048 *λD* deletion |
| XTL1219 | XTL1048 *λD* deletion / pLXT42 |
| XTL1195 | XTL1059 λ*D* fused to CLL antigen at the N-terminus |
| MR40271 | MG1655 Δ*lacI*, *araFGH<>spec,* Δ*araE*, Δ*lamB*, *ΔfhuA, lacY* A177C *araC+*, Δ*araBAD,* λ*CI857 Sam7 (geneE E158K,* Δ*ea59ea31*) |
| MR40272 | MG1655 Δ*lacI*, *araFGH<>spec,* Δ*araE*, Δ*lamB*, *ΔfhuA, lacY* A177C *araC+P_{BAD}* -λ*D*, λ*CI857 Sam7 (geneE E158K,* Δ*ea59ea31, ΔD)* |
| MR40273 | MG1655 Δ*lacI*, *araFGH<>spec,* Δ*araE*, Δ*lamB*, Δ*fhuA*, *lacY* A177C *araC+,* Δ*araBAD*, λ*CI857 Sam7 (geneE E158K,* Δ*ea59ea31,* Δ*D*)/ pLXT42 |
| MR40274 | MR40273 / pLXT42 with D fused to COVID antigen at the C-terminus |
| MR40275 | MR40273 / pLXT42 with D fused to CLL antigen at the N-terminus |
| MR40276 | MR40273 / pLXT42 with D fused to malaria antigen at the C-terminus |
| MR40277 | MR40271 λ*D* fused to COVID antigen at the C-terminus |
| MR40278 | MR40271 λ*D* fused to CLL antigen at the N-terminus |
| MR40279 | MR40271 λ*D* fused to malaria antigen at the C-terminus |
| XTL1380 | XTL1048 λ D fused to COVID antigen at the C-terminus |
| MR40261 | XTL1059 λ D fused to CLL antigen at the N-terminus |
| MR40253 | XTL1048 λ D fused to malaria antigen at the C-terminus |
| XTL1312-XTL1323 | XTL1048 λ D fused to various segments of S protein of SARS-CoV-2 |
| XTL1324 | XTL1048 λ D fused to a full-length E protein of SARS-CoV-2 |
| XTL1293-XTL1294 | XTL1048 λ D fused to two different segments of N protein of SARS-CoV-2 |

### Example 2

### Retrieval of P_{BAD} -D from the chromosome onto pBR322 generates pLXT42 plasmid

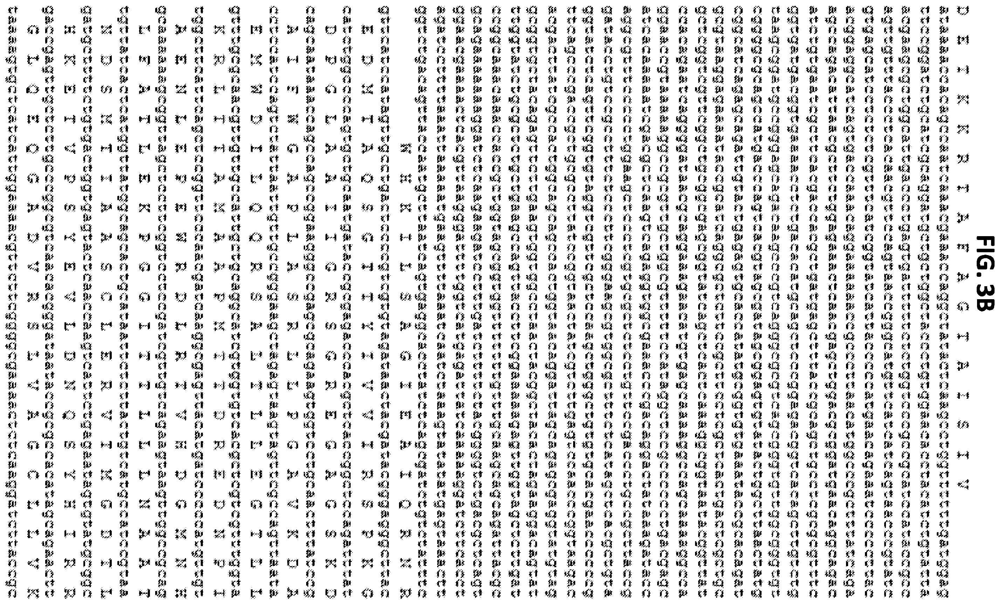

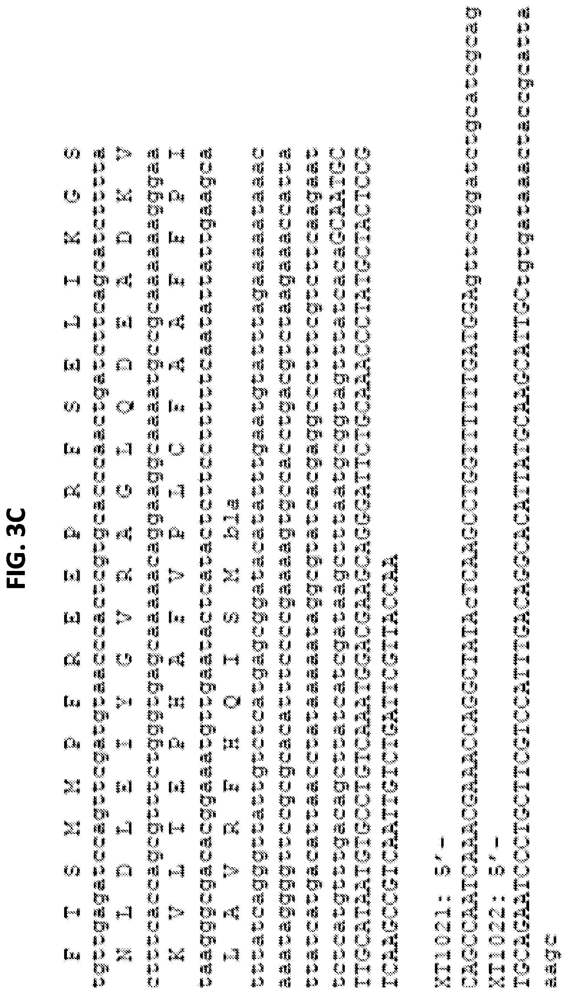

The genome segment of strain XTL981 containing the *araC* gene and the arabinose regulated gene *D* of λ was precisely transferred by Red-mediated recombination to the pBR322 plasmid backbone using a method of recombineering described as retrieval (Thomason et al, Curr. Protoc. Mol. Biol. 106:1.16.1-1.16.39. 2014). To achieve this, the pBR322 replication origin was amplified with PCR primers XT1021 and XT1022. The PCR product was purified with a Qiagen PCR kit. The PCR product was electroporated into XTL981 with RED system expressed, and the cells grown at 32 °C overnight in 10 ml LB. Plasmid was isolated from the overnight culture with Qiagen QIAprep Spin Miniprep kit, and the plasmid transformed into DH5α and selected on ampicillin agar. The plasmid contained the *araC⁺ P_{BAD}-D* region by sequence analysis. This generated plasmid pLXT42 (see FIG. 3). λ D protein can be provided either from a single copy of gene *D* on the chromosome or from the multiple copies of gene *D* on the plasmid. In each case, λ gpD levels are tuned to the arabinose concentration in the medium.

### Example 3

### In-frame deletion of the entire D gene from its native locus in λ

A precise in-frame deletion of the entire λ*D* gene coding region was created within the λ prophage in strain XTL1205 and MR40272. Table 1A provides the sequence of oligo XT1115, which was used to generate the *D* gene deletion by recombineering. The *D* deletion prophage (λΔ*D*) had no homology to rescue by recombination the *D*+ gene present either on the plasmid pLXT42 or on the chromosome at arabinose locus of *E. coli.* Thus, if the λΔ*D* prophage is induced from XTL1205 with arabinose present, the *D* gene at the arabinose locus expresses D protein, and phage particles were generated. These particles only form plaques on a LE392 suppressor derivative (XTL1212) carrying the pLXT42 plasmid, which provided D protein *in trans* in the presence of arabinose. LE392 itself suppresses the *Sam7* defect of the phage but does not provide any λ D function. The results showed that enough D can be made from the arabinose inducible promoter from an ectopic locus to enable stable particle formation even when λ is deleted for *D* gene.

### Example 4

### In-frame deletion of the entire D gene from its native locus on λ and the arabinose locus in the chromosome

For the deletion of λ *D* from the chromosome, strain MR40272 was modified by recombineering to replace the λ *D* gene at the arabinose operon with the counter selectable marker *tetA-sacB-amp.* The *araC* gene remains intact allowing AraC regulation. Then oligo MR27, see Table 1A, was used to generate the D gene deletion by recombineering as described above. Thus, a precise in-frame deletion of the entire λ*D* gene coding region was created within the λ prophage and the one present in the arabinose locus of the bacterial chromosome, generating a new intermediate strain. Next, this intermediate strain was transformed with the plasmid pLXT42 to generate a strain MR40273.

### Example 5

### Ectopic expression of D protein for complementation

The λΔ*D* prophage, which is unable to express D protein, was used to determine how well D protein made *in trans* from plasmid pLXT42 or from the chromosome at arabinose locus at different times relative to prophage induction (FIGs. 4A-4B) and at different levels (FIG. 5) could assemble stable capsids and particles containing the defective λΔ*D* genome.

When λD protein from the plasmid is fully induced by arabinose (0.2%) at various times before and after temperature induction of the λ*cI857 Sam7* Δ*D* prophage, a high yield of phage particle is obtained for the λ*cI857 Sam7* λΔ*D* prophage in strain XTL1219 expressing D protein induced by arabinose from high copy plasmid pLXT42 (FIG. 4A). These defective λΔ*D* particles are visualized as plaques and titered on agar plates containing arabinose and the plating cells XTL1212. Note, when titered on LE392 plating bacteria there are no plaques found, indicating that λ*D*⁺ recombinants are not generated. A control λ*cI857 Sam7* λ*D⁺* lysogen XTL1178 (Table 1B) was also induced for λ in the presence or absence of arabinose. High levels of D protein made in the presence of arabinose had no inhibitory effect when arabinose was added at the time of temperature induction of the λ*D*⁺ prophage or even 1.5 hr before temperature induction. Complementation by D protein *in trans* from the arabinose system is ~10-fold less effective at all times than when D protein is made from the induced prophage itself (compare induction of XTL1178 λ*D*⁺ without arabinose (never) to the induction of XTL1219 λΔ*D* with arabinose present).

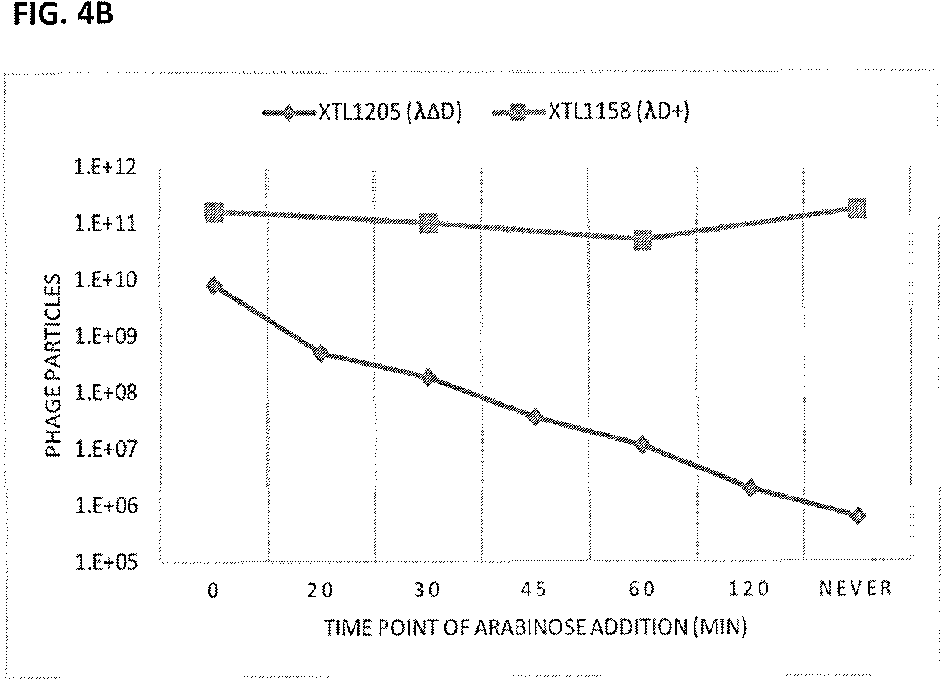

λD protein is induced from a single copy of the λ*D* gene under arabinose operon control by adding arabinose (0.2%) at various times at or after temperature induction of the λ*cI857 Sam7* Δ*D* prophage (FIG. 4B). Complementation for phage particle formation was determined as in FIG. 4A by plating for plaques on strain XTL1212, which provides D function from the plasmid pLXT42. A single copy of λ*D* expressed from the chromosomal arabinose operon is limiting (with or without arabinose) for complementing the λ*cI857 Sam7* λΔ*D* prophage after temperature induction. Under this condition, optimal complementation occurs by inducing with arabinose at the same time the prophage is induced by temperature. In this experiment, chloroform was added to lyse cells at 3.5 hrs after the shift to 42°C. D function became more limiting the later arabinose was added after temperature induction of the λ*cI857 Sam7* λΔ*D* prophage. The red line in Fig 4B indicating the titer of a λ*cI857 Sam7* λ*D*+ prophage induced in the same way represents the control.

When λD protein from the plasmid was induced by adding arabinose at 30 min after temperature induction, phage yields decrease in concert with arabinose concentration (FIG. 5). Phage yield vs D expression (arabinose concentration) drops off in non-linearly, indicating that D protein becomes limiting and suggests that particles assemble D cooperatively.

Formation of a plaque on a lawn of bacteria starts by a single cell infected by a phage particle, with lysis and release of progeny phage. Subsequently, adjacent cells are infected by the released phage, leading to more cycles of lysis. A plaque indicates that the phage must have gone through several lytic cycles to produce at least ~10⁷ particles to make a visible plaque. A λ particle lacking the *D* gene must be complemented from the plasmid pLXT42 (or from ectopic locus in bacterial chromosome), which expresses enough D by induction with arabinose to allow efficient plaque formation by λ particles lacking the *D* gene. It was found that the D protein supplied *in trans* from the plasmid after fully inducing D protein expression with arabinose (0.2%) resulted in an ~10-fold reduced λ Δ*D* mutant phage yield compared to a λ phage yield in which the intact *D*⁺ gene is expressed in its normal location on the λ genome. Expression of D from the plasmid did not increase or decrease the normal high yield of a wild type λ *D*⁺ phage. However, because yields of phage are 10-fold reduced when D protein is made *in trans* from a high copy plasmid to complement a λ Δ*D* mutant as compared to induction of the prophage carrying an intact *D* gene.

All of the D-fusion constructs were generated at the wild-type *D* site in the λ prophage genome in order to express D-fusion protein *in cis* coordinately with the rest of the phage capsid functions, including the *E* gene. This is different from the system disclosed in U.S. Patent 7,410,801, where the D-fusion construct was expressed *in trans* from a multicopy plasmid or from a λ infecting phage with the *D*-fusion gene cloned in the non-essential *b* region of λ.

The following two conditions reduced the yield of phage particles: (i) the distal location of the *D* gene in the b region of λ makes the source of D ectopic; (ii) the ectopic location of the *D* gene does not allow its coordinated expression with its native neighbor *E* gene.

Previous work used a phage that was an infecting phage with an amber mutation in gene *D* to prevent wild type D protein expression from its native location. U.S. Patent 7,410,801 discloses phage wherein the D-fusion protein was expressed from the lambda phage *b* region, far from its native location. In such systems, high titer phage preparations by infection is much less efficient than the currently disclosed system, wherein high titer phage are made by inducing prophages carrying a temperature sensitive repressor, the *Sam7* allele defective in cell lysis, and the D-fusion gene in the native D locus.

### Example 6

### λ display phages

Several λ D-display phages were constructed using the λ prophage vector system and the presently disclosed methods by displaying, for example, different D-fusion proteins for use as phage-based vaccines representing different human diseases. First, several antigen displaying phages were made and confirmed for their proper construction by DNA sequencing and Western blot analysis. Second, it was confirmed that the D-fusion proteins retain the function of wild type D protein in phage growth as assayed by their plaque forming ability. The fusion proteins functioned the same way as wild type D protein when supplied in either cis or trans. In one example, a fusion protein, when somewhat defective in virion stabilization, was stabilized by providing extra amount of wild type D expressed from ectopic location.

*Case1. λ D-fusions as potential vaccines for human CLL leukemia disease.* The antigen exposed on the CLL (NALM6) cell surface is CDR3 peptide of the IGHV gene. The defined antigenic segment CDR3 was fused to the N-terminal sequence of *λ*D protein through the GGGS linker in the prophage. The phage was labelled as XTL1195. The amino acid sequence of the fusion is shown in FIG. 6. The Western-blot results shown in FIG. 7 authenticate that the engineered phages indeed make a fused protein as expected.

*Case 2. λ D-fusions as potential vaccines for malaria.* One of the malaria parasite *P. falciparum* antigens is pfGARP (a glutamic acid rich region). Different lengths of the known antigenic segment of pfGARP were genetically fused to the C-terminus or N-terminus of *λ*D at the prophage level with a single or triple GGGGS linker sequence between the antigen and D protein sequences. The N-terminal portion of the identified antigenic segment of pfGARP was fused to *λ*D at its C-terminal end and it is labelled as strain MR40279. The sequence of the fusion is shown in FIG. 8. The expression of D fused to the segment of pfGARP is shown in FIG. 9. In the second and third construct, the C-terminus of pfGARP was genetically fused to the C-terminus and N-terminus of *λ*D, respectively, in the prophage. Single or triple GGGGS linker sequence was used between the fusion joints. The constructs were labelled as XTL1106 and XTL1107. The amino acid sequence of these fusions is shown in FIG. 10 and FIG. 11.

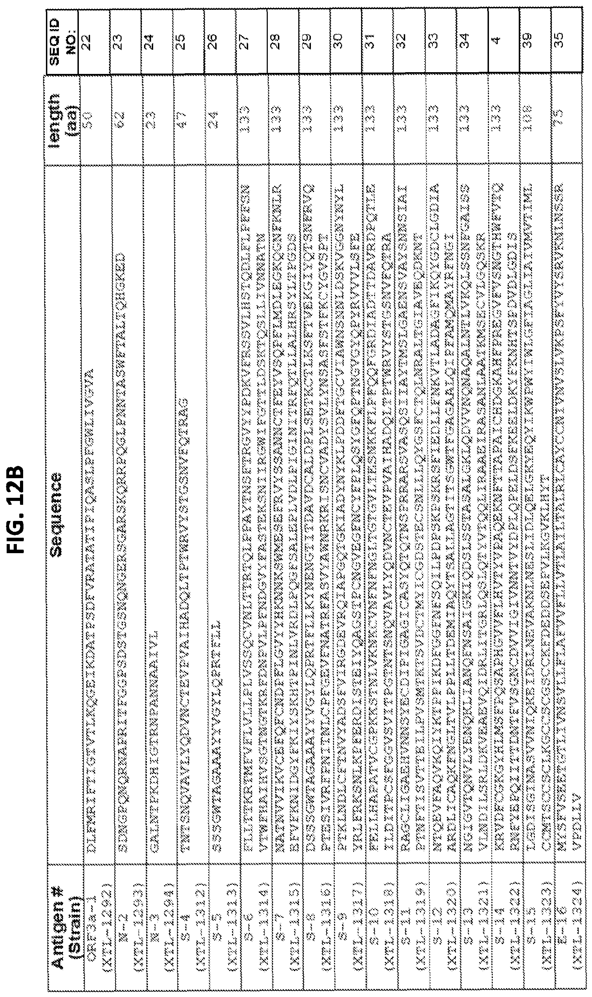

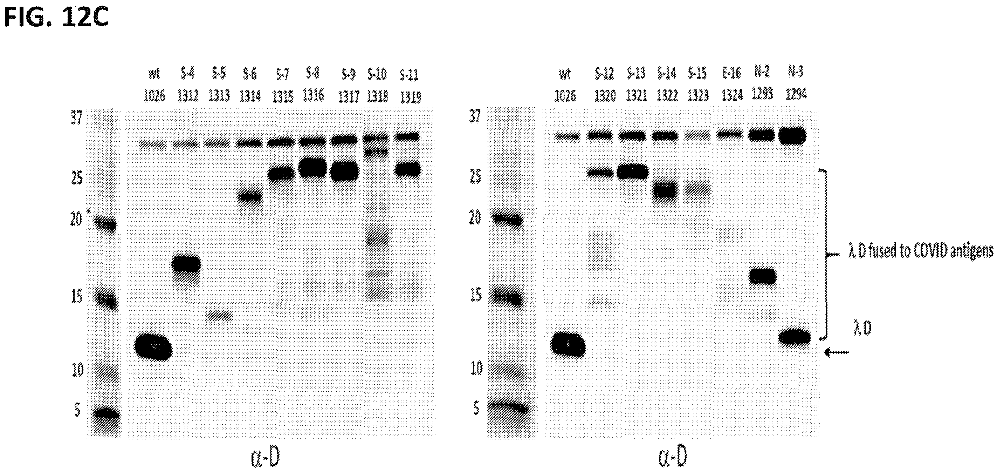

*Case 3. λ D-fusions as potential vaccines for COVID-19.* Segments of several proteins of SARS-COV-2 virus, Spike (S), envelope (E), and nucleocapsid (N) have been cloned for D-display on phage λ (Fig. 12B). After sequence verification of fused clones, they were tested by Western-blot to confirm stable expression. Some of the expressed D-fusion proteins were seen to be unstable (see lane S-10 in Fig 12C).

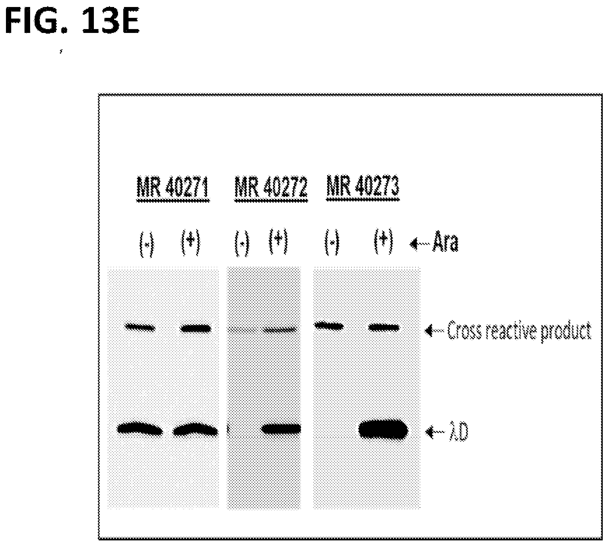

The stability of D-fusion and virion particles was evaluated by the following:
1) Western-blot was used to identify degradation products of D-fusions (see Fig 12C).
2) The ability to form a plaque (see the phage titers without inducing wild type D protein in Figs. 13-15).
3) The number of virions was determined by measuring DNA content by PCR analysis.
These analyses were used to determine if expression of exogenous wild type D-protein was needed or not, see Fig. 15A.

Some examples of segments of the S, E, and N protein are shown in FIG. 12A and 12B. The sequence of each displaying peptide and their length is shown in FIG. 12. Any of these heterologous proteins can be expressed by a bacteriophage λ provided herein and used as an immunogenic composition for these antigens.

### Example 7

### Optimizing functionality of display phages

The strain containing gpD-fusion prophage was grown at 32° in L Broth to 2x10⁸ cell/ml before shifting to 42° for 30 min and back to 39° for 3hrs. A test lysate was prepared by adding chloroform and the phage numbers titered on a *supF* strain (XTL1212) which suppresses the *Sam7* defect to allow lysis and plaque formation. The functional quality of the D-fusion protein can affect how well λ capsid particles are formed and how efficiently the phage can generate plaques. XTL1212 also contains the plasmid pLXT42, which produces λD protein to complement D-fusion phage particles that may be somewhat defective in D function and cannot form plaques efficiently on their own.

The protein fusions generated at either the N-end or C-end of the *D* gene could cause defects in active virion formation and plaque-forming ability. This may be predicted, since D interacts with E to stabilize the phage capsid. Thus, some D-fusion proteins may not fold well and may interact poorly with E to stabilize the capsid. A certain level of wild type λD protein supplied *in trans* from the arabinose system can, in some circumstances, help stabilize the particles made by the prophage carrying a defective D-fusion (Zanghi et al. Nucleic Acids Res. 2005; 33(18): e160). However, this system has not been used in a controlled way, such as with the arabinose promoter. In the present system, when wild type λD protein is optionally expressed from a plasmid, its expression can be turned on at a precise time by addition of arabinose and the level of D expression is directly tuned to the concentration of arabinose added. These controlled parameters allow the D protein expression from the *ara* operon to be turned on at an optimum time relative to when D would normally be made following temperature induction of the prophage. The ability of the phages expressing the D or D-fusion protein from *cis* vs *trans* locations to produce progeny was demonstrated. The suitability of expressing wild type D-protein from the single copy at arabinose locus to maximize the yield of D-fusion display phages was evaluated.

### 1. Yield of phage vs. genetic source of D protein during phage growth: Phage yield is superior when the expression of the D or its fusions is from its native genetic locus on λ rather than from an ectopic locus

The effect on phage yield was investigated by prophage induction upon the source of D protein expression of the D gene being present either in its native locus in phage λ or in an ectopic locus elsewhere in the λ prophage, specifically the bacterial chromosome, or a plasmid. Phage yield was compared between an induced prophage carrying a wild type D gene in its native locus or in an induced prophage deleted for the D gene, but having D expressed upon addition of arabinose from the *ara* promoter either from the chromosomal *ara* operon or from a plasmid (FIGS. 13A-13D). The results showed that the phage yield was unaffected by the presence of arabinose when the wild type gene D is only present at its native locus (FIG. 13D, column 2 strain MR 40271). In a strain where the D gene was deleted from its native locus, arabinose induced ectopic expression of wild type D either from the bacterial chromosome (FIG. 13D, row 2 column 3, strain MR 40272) or from a plasmid (FIG. 13D, row 2 column 4, strain MR 40273) restores phage yield to a high level. However, this phage yield was reduced by 3-fold or 10-fold (4.0 x 10¹⁰ pfu/ml or 1.2 x 10¹⁰ pfu/ml vs 1.2 x 10¹¹ pfu/ml) when D was expressed from the *ara* promoter in the bacterial chromosome or from a plasmid, respectively, as compared to its expression from the native locus. The reduction in phage yield by ectopic expression of D in the presence of arabinose was observed even when there was an increased production of D protein from the ectopic locus (FIG. 13E, compare lane 1 or 2 with lane 4 and lane 6). The expression of D from a multicopy plasmid in strain MR40273 was significantly higher than the normal physiological level in strain MR 40271. This demonstrated that the expression of D from its native locus on λ was more efficient for phage morphogenesis than excessive expression of D from an ectopic locus.

The effect of the location of the D gene on phage yield is also true for phages displaying D-fusion antigens (FIG. 14A and FIG. 14D). A 23 amino acid peptide from the Spike protein of SARS COVID-2 virus (FKEELDKYFKNHTSPDVDLGDIS, SEQ ID NO: 38) was fused to the D protein of phage λ and either expressed from the native D-locus in the prophage (strains MR40277) or from the *ara* promoter present in a plasmid in the cell (strain MR40274) (see FIG. 14B and FIG. 14E). In the latter case, the wild type D gene on the λ prophage was deleted. Consistently, expression of the D-fusion from its native locus resulted in a10-fold higher phage yield compared to its expression from the plasmid in the presence of arabinose (1.0 x 10¹¹ pfu/ml vs 1.4 x 10¹⁰ pfu/ml in FIG. 14B, column 1and FIG. 14E column 2) even though the level of D-fusion protein was observed to be higher when expressed from the multicopy plasmid (FIG. 14F, lane 2).

The increased expression and yield of the D gene when at its native locus was demonstrated for other D-fusion phages tested, regardless of the antigenic peptide length. The phage yield was compared on the expression of D fused with the 22 amino acid CDR3 region of the leukemia B-cell receptor (strain MR 40278) and D fused with 143 amino acid of peptide derived from the P. *falciparum* glutamic-acid-rich (pfGARP) antigen (strain MR 40279). In both cases, phage yield, when the expression of the D-fusion was at its native locus, was better than the expression from the plasmid in the presence of arabinose. The phage yield during expression of the CDR3 fusion from the native D locus (4.0 x10⁸ pfu/ml, FIG. 14B, strain MR 40278) was higher than from the plasmid (3.8 x 10⁷ pfu/ml, FIG. 14E, strain MR 40275). However, the general phage yield for expression of the pfGARP fusion from the native D locus (1.4 x 10⁶ pfu/ml, FIG. 14B, strain MR40279) or from the plasmid (1.0 x 10⁷ pfu/ml, FIG. 14E, strain MR 40276) was relatively poor as compared to other fusions. Without being bound by theory, this may be caused by the relatively greater instability of the specific longer peptide fused to D as observed in the expression profile shown in FIG. 14c, lane 3, and F, lane 6.

### 2. Total phage yield

A platform was constructed that gives maximum yield of λ particles that display stable antigens for downstream applications. However, the amino acid sequence and/or the length of the displayed antigenic peptide fused to the λD protein can sometimes result in an unstable phage head and consequently lower phage yields. The phage yield in such cases can be increased by providing an incremental amount of wild type D protein in the same cell. To do this, the wild type *λD* gene is also expressed from an ectopic locus in the bacterial chromosome giving the fusion phage a chance to assemble a stable phage head with a mixture of wild type and D-fusion proteins (FIG. 15A). A single copy of the wild type D gene was placed under the inducible *ara* promoter (P_{BAD}) in the host chromosome. For example, a prophage containing the fusion of a 143 amino acid segment from the *P. falciparum* glutamic-acid-rich (pfGARP) antigen region to protein D at the native D locus in the prophage was unable to produce high yield of phage upon prophage induction (1.4 x 10⁶ pfu/ml, FIG. 14B, strain MR40279). However, providing additional expression of the wild type D protein from the *ara* promoter in the chromosome increased the phage yield by ~ 4300-fold in the presence of arabinose (6 x 10⁹ pfu/ml, FIG. 15B, strain MR40253). In another example, a 22 amino acid peptide derived from the CDR3 region of the B-cell receptor of chronic lymphocytic leukemia (CLL) cells was fused to the amino terminal end of the D protein. The prophage harboring the D-fusion of the CLL antigen at its native D-locus was unable to produce phage in high yield (4.0 x 10⁸ pfu/ml, FIG. 14B strain, MR40278), but this phage yield was enhanced by around 15-fold when the wild type D-protein was simultaneously expressed from the ectopic chromosomal *ara* promoter upon arabinose addition (6.0 x 10⁹ pfu/ml, FIG. 15B, strain MR 40261). These examples demonstrate that the production of antigen displaying phage can be substantially increased (if the antigen is unstable) by supplying wild type D protein in cell during phage growth. The evidence for enhancement by the wild type D protein for production of functional fusion display phages in the two examples are demonstrated by corresponding Western blots experiments (FIG. 15C, lanes 2, 4, and 6). In summary, the production and stability of some D-fusion particles from the native locus can, in some circumstances, be improved when wild-type D is also expressed from an ectopic locus.

In the present system, the *D* gene-fusion is transcribed and translated from the λ genome and expressed in *cis* from its normal location upstream of gene *E* on the λ genome and in concert with all of the other λ phage capsid proteins. It was demonstrated that wild type D protein, when expressed in *cis* with gene *E,* generates an ~ 10-fold greater yield of phage particles than when D protein is provided in *trans* from a plasmid to a λ that is deleted for the *D* gene. Thus, expressing wild-type D protein or a D-fusion *in cis* on λ can be used for optimal capsid assembly and phage yields.

Two other λ structural proteins can also be used to display fusions with foreign proteins. The gpV tail protein can be used as a vehicle for such fusions (Maruyama et. al., Proc. Natl. Acad. Sci. USA, vol 91, 8273-8277, 1994). It has been demonstrated that protein segments can be fused near the distal carboxy end of the gpV protein in a phage that also has a fused GFP protein to λ D. Thus, a dual fusion display phage was generated. The side tail fiber protein (Stf) of λ that attaches to the end of the phage tail can also be used to attach longer protein segments. Stf is non-essential for λ (Hendrix and Duda, Science vol. 258, 1145-1148, 1992), and the cloning results indicate that protein segments can be added at its distal end, especially those that fold into trimers like the tail fiber protein itself. Thus, display potential antigens can be displayed, on one, two, or all three different λ proteins, D, gpV and Stf.

The current pandemic has demonstrated that rapid vaccine development to target the Covid-19 has become vital for the entire world. Vaccines currently licensed for human use are eucaryotic virus-based, protein-based, or mRNA based. However, certain limitations are associated with each of these vaccine platforms that make them less suitable to fast vaccine production or creates logistical issues due to special storage requirement. Thus, there is a need for creating an optimized vaccine technology. A method has been developed that is less expensive for the production of antigen, large amounts, and is stable at ambient temperatures.

The phage used as a vector for vaccine production is temperate phage λ. Phages, and in particular λ, are non-toxic when introduced into mammalian systems, and are thus safe as a vaccine when used to generate an antibody response in mammals. Furthermore, it is demonstrated herein that peptides and even full-length proteins can be displayed on the surface of the λ virion's capsid when covalently joined to the major capsid protein D on the phage head particle. The disclosed phage-host system allows easy construction of phages for the display of protein antigens. Several strains were developed using the disclosed system that display different proteins on the virion surface. These phages were characterized for their accuracy, activity, stability, and feasibility to generate antigens to different disease types. The use of exemplary antigens for CLL leukemia, malaria, and COVID-19 demonstrate the versatility of this system as vaccines for a variety of diseases, including, but not limited to, cancer, allergies, viral infections, bacterial infections, and other infectious diseases.

### Example 8

### Identification of COVID-19 spike epitope using SARS-CoV-2 infected human serum

A panel of human serums from four healthy donors and four individuals positive for SAR-CoV-2 infected patients were obtained. To identify if any of the SARS CoV-2 antigen displaying phages would react to the serum samples, a 4-20% SDS gradient gel was run for each phage sample listed in FIG. 12B. The samples were then transferred to a nitrocellulose membrane for a standard Western blotting. The membrane was blocked with 2.5% non-fat milk buffer, then triple washed with the TNE buffer (10mM Tis-HCl, 50mM NaCl, 2.5mM EDTA), prior to incubating with 1000-fold dilution of human serum samples extracted either from a healthy donor (COVID-SP-005) or an individual positive for SARS-CoV-2 (COVID-SP-009). The membrane was incubated with serum samples minimum for three hours and then washed with a wash buffer (10mM Tis-HCl, 50mM NaCl, 2.5mM EDTA). It was then incubated with 10,000-fold diluted goat anti-human IgG secondary-horse radish peroxidase (HRP) conjugated antibody for an hour and washed with a wash buffer containing 0.1% TWEEN^{®}. The membrane was then developed using a clarity enhanced chemiluminescence substrate from BIORAD^{®}.

Only phage strain XTL-1322, displaying 133 amino acid (aa) long peptide from near the C-terminal end of the spike protein, reacted with the SARS CoV-2-positive serum but not with the serum of a healthy donor (FIG. 16A). However, the titer of this phage was not optimal, possibly due to the instability of the phage. To narrow down the epitope and construct a more stable phage, the DNA fragment encoding the 133 aa long peptide was further dissected into overlapping DNA fragments encoding smaller peptide fragments (Table 2).

**Table. 2 Phage strains displaying SARS CoV-2 antigens. All antigens in this list are fused at the C-terminus of the lambda D protein.**

| **Antigen** | **Strain #** | **Sequence (SEQ ID NO)** | **length (aa)** |
|---|---|---|---|
| SARS CoV-2 | XTL-1322 | KRVDFCGKGYHLMSFPQSAPHGVVFLHVTY VPAQEKNFTTAPAICHDGKAHFPREGVFVSN | 133 |
| | | GTHWFVTQRNFYEPQIITTDNTFVSGNCDVV IGIVNNTVYDPLQPELDSFKEELDKYFKNHT SPDVDLGDIS (SEQ ID NO: 4) | |
| SARS CoV-2 | XTL-1375 | KRVDFCGKGYHLMSFPQSAPHGVVFLHVTY VPA (SEQ ID NO: 40) | 33 |
| SARS CoV-2 | XTL-1376 | VVFLHVTYVPAQEKNFTTAPAICHDGKAHF PRE (SEQ ID NO: 41) | 33 |
| SARS CoV-2 | XTL-1377 | CHDGKAHFPREGVFVSNGTHWFVTQRNFYE PQI (SEQ ID NO: 42) | 33 |
| SARS CoV-2 | XTL-1378 | VTQRNFYEPQIITTDNTFVSGNCDVVIGIVNN T (SEQ ID NO: 43) | 33 |
| SARS CoV-2 | XTL-1379 | CDVVIGIVNNTVYDPLQPELDSFKEELDKYF KN (SEQ ID NO: 44) | 33 |
| SARS CoV-2 | XTL-1380 | FKEELDKYFKNHTSPDVDLGDIS (SEQ ID NO: 45) | 23 |
| SARS CoV-2 | XTL-1381 | MSFPQSAPHGVVFLHVTYVPAQEKNFTTAP AICHDGKAHFPREGVFVSNGTHWFVTQRNF YEPQIITTDNTFVSGNCDVVIGIV (SEQ ID NO: 46) | 84 |
| SARS CoV-2 | XTL-1382 | YDPLQPELDSFKEELDKYFKNHTSPDVDLG D (SEQ ID NO: 47) | 31 |

These DNA fragments were recombined in-frame at the 3'end of the lambda D gene as described previously. The newly generated phage strains XTL-1375, XTL-1376, XTL-1377, XTL-1378, XTL-1379, XTL-1380, and XTL-1381were further probed by Western blot using the serum sample obtained from a patient positive for SARS-CoV-2 (COVID-SP-009) (FIG. 16B). Only XTL-1379 and XTL-1380 phage strains reacted with a SARS CoV-2 positive serum sample, further narrowing down displaying peptide length down 33 and 23 aa long. The peptide displayed by these two phage strains overlapped with each other by 11 amino acids (FIG. 18). Upon sequence alignment with spike protein of different variants such as alpha, beta, delta, omicron, as well as SARS-CoV-1, it was determined that the 11 amino acid overlapping segment between XTL-1379 and XTL-1380 was conserved among all other variants of SARS-CoV. A linear epitope was defined in the COVID-19 spike protein that is conserved among variants and can be a target for an immune response, such as antibodies. It was hypothesized that this segment of peptide triggered the generation of specific antibody among other infected patients. Therefore, XTL-1322, XTL-1379, and XTL-1380 phage strains were probed with four different serum samples extracted from SARS CoV-2-positive subjects and compared with four different serum samples extracted from healthy donors. It was determined that each subject, that was confirmed SARS CoV-2-positive, had circulating antibody that specifically cross reacted with the peptide fragment displayed by XTL-1322, XTL-1379, and XTL-1380 whereas none of these phages reacted with the serum from healthy donors (FIG. 17).

Because an overlapping peptide segment was identified that displayed between phage strains XTL-1379 and XTL-1380, a new hybrid strain XTL-1382 was engineered by recombineering that displayed the overlapping 11 amino acid peptide (SEQ ID NO: 69) flanked by consecutive 10 amino acids in either end of the peptide (FIG. 18). When the same amount of XTL-1382 phage particles was again probed with SARS-CoV-2-positive serum (COVI-SP-009) and compared with the strains XTL-1379 and XTL-1380, it was found that the newly engineered hybrid strain XTL-1382 strongly reacted with the antibody present in the serum, as indicated by the band intensity in FIG. 18. The SARS CoV-2 positive serum sample did not react with the vector phage XTL-1026 (wt). These results indicate that phage strain XTL-1382 displays high-affinity epitope that can trigger the generation of an effective immune response upon immunization.

### Example 9

### Immunogenicity Study

Four phage candidates (XTL-1026 as a control, XTL-1379, XTL-1380, XTL-1382) were selected for immunogenicity study. For each phage, a three dose regimen was selected, i.e,, 10⁷ particles in 500ml PBS buffer per dose, 10⁸ particles in 500ml PBS buffer per dose, and 10⁹ particles in 500ml PBS buffer per dose. Each dose regimen was injected into a set of three female BALB/c mice of 6 to 10 weeks old. Roughly 100 ml of blood sample was collected from the tail vein right before the IP injection. The injection was given on day 0 and boosted twice on day 21 and day 70. The blood sample was also collected on day 42, and 66 following the first booster and day 96 following the second booster (FIG. 19A). The blood samples were collected in non-EDTA, non-heparin containing tubes, that were centrifuged to collect the serum samples, and stored at -80 degree until serological analysis were performed.

The method for the ELISA that was performed is disclosed above in Example 8. In the ELISA, the Nunc MAXISORP^{®} flat-bottom plates were coated with synthetic peptide that were displayed on the phage head surface. In the case of vector control XTL-1026, the plate was coated with the peptide displayed in phage XTL-1382. For the baseline control, the wells were left alone with PBS buffer alone. The data points that are autocorrected from baseline were obtained by measuring the optical density at 450nm. Representative data shown in FIG. 19B indicated that phages XTL-1380 and XTL-1382 induced a demonstrable response to immunization on day 21, 42 and 66, compared to the vector phage XTL-1026 and the displaying phage XTL-1379 when mice were injected with the dose regimen of 10⁹ particles per injection. A strong response was not observed in ELISA when the serum samples obtained from a set of mice injected with lower dose (10⁷ and 10⁸ particles per injection) were evaluated. To confirm the validity of this response, Western blot analysis was performed using the serum collected on day 42, 66, and 96 of a selected mice from three different group (FIG. 16C). A specific response was observed to the antigen that was injected into the mice. For example, the serum of mice injected with XTL-1380 reacted strongly with the phage displaying 1380 peptide, with minimal cross reaction with the phage XTL-1382, and vice versa. The control phage XTL-1026 did not generate any antibodies that reacted with the displaying peptides. The lower panel in FIG. 16C also indicates that the response for a specific antibody generation was elevated after the second booster on day 70 when the serum sample from day 96 was analyzed indicating the presence of long circulating high titer antibody.

The following additional phage were designed and tested:

**Table 3**

| **Additional Phages** | | | | |
|---|---|---|---|---|
| **Antigen** | **Strain #** | **Sequence** | **length (aa)** | **Fusion end** |
| SARS-CoV-2 | XTL-1383 | GGFNFSQILPDPSKPSKRSFIEDLL FNKVT (SEQ ID NO: 48) | 30 | C-terminus |
| SARS-CoV-2 | XTL-1384 | CDIPIGAGICASYQTQTNSPRRAR SVASQS (SEQ ID NO: 49) | 30 | C-terminus |
| SARS-CoV-2 | XTL-1385 | NNLDSKVGGNYNYLYRLFRKSN LKPFERDI (SEQ ID NO: 50) | 30 | C-terminus |
| SARS-CoV-2 | XTL-1386 | NLKPFERDISTEIYQAGSTPCNGV EGFNCY (SEQ ID NO: 51) | 30 | C-terminus |
| SARS-CoV-2 | XTL-1387 | NGVEGFNCYFPLQSYGFQPTNGV GYQPYRV (SEQ ID NO: 52) | 30 | C-terminus |

| **Glioblastoma multiforme** | | | | |
|---|---|---|---|---|
| EGFRvIII | MS-41 | LEEKKGNYVVTDH (SEQ ID NO: 53) | 13 | C-terminus |
| EGFRvIII | MS-42 | LEEKKYNYVVTDH (SEQ ID NO: 54) | 13 | C-terminus |
| EGFRvIII | MS-43 | LEEKKGNYVVTDH (SEQ ID NO: 53) | 13 | N-terminus |
| EGFRvIII | MS-44 | LEEKKYNYVVTDH (SEQ ID NO: 54) | 13 | N-terminus |

### Example 10

### Mouse Models

An animal model is used to test the immune response produced a phage containing a heterologous antigen. The method includes: (a) injecting an individual vaccine into animals, (b) collecting serum samples at various time points, (c) assaying the presence and duration of antibody in response to vaccine injection by ELISA, Western blot, or another method, and (d) assaying the pharmacological effectiveness *in vitro* and/or *in vivo* by neutralization capacity of the antibody present in the serum.

### Immunization protocol:

BALB/c mice (6 to 10-week-old) are used as the test animals. Mice are grouped in a set of three for immunization with antigen-displaying phage particles as an experimental vaccine or, for baseline control, using a non-displaying phage vector or the composition buffer (or saline) alone. A dosing regimen of 10⁷, 10⁸, 10⁹, or 10¹⁰ phage particles (per animal, one group of three animals for each dose) are administered in a fixed volume. Phages are injected intraperitoneally (IP), intramuscularly (IM), intravenously (IV), or subcutaneously (SC). These modes of injection have been shown to deliver phages systemically to virtually all organs and tissues. Mice are inoculated with the dose at three week intervals over a period of six weeks, for a total of three doses, at time 0, 3 weeks and 6 weeks. Sera are collected prior to each inoculation as well as after the final dose.

The vaccine composition is tested with or without adjuvant will be tested. The proteinaceous lambda particle can itself serve as an effective natural adjuvant.

### Serological Analysis:

To measure the humoral response, 96-microwell plates are used for an ELISA assay. The Nunc MaxiSorp flat-bottom plates are coated with the commercially synthesized peptide that is displayed on the phage head surface. For coating, about 150 ng of peptide in PBS buffer is incubated overnight at 4°C. For the baseline control, the plate is coated with an equimolar amount of purified λ D protein or left blank with PBS buffer alone. Coated plates are blocked with PBS containing 5% low-fat milk and 1% bovine serum albumin (BSA) for 1 hour at 37°C. Serial dilutions of serum from vaccinated mice or unvaccinated control mice in blocking buffer are added to the separate wells and incubated for 1 to 2 hours at 37°C. The wells are washed three times with PBS containing 0.1% of Tween-20 and then PBS alone. The bound antibodies are detected with peroxidase activity using horseradish peroxidase (HRP)-conjugated anti-mouse IgG at an optical density of 450 nm. The absorption difference between the groups of mice will be analyzed for statistical significance.

The presence of a specific antibody to the displayed antigen is tested by Western-blot analysis. An equimolar amount of antigen displaying λ phages, and the parental λ phage (no encoded antigen) undergo electrophoresis in denaturing conditions of 4-20% Bis-Tris NuPAGE gel. The resolved protein samples in the gel are then transferred into a nitrocellulose membrane for Western blotting. Non-specific interaction is blocked using 5% low-fat milk. After washing, the membrane is probed with mouse serum from the experimental and control group to allow binding with the D-fusion protein. The bound antibodies are detected by activity of horseradish peroxidase (HRP)-conjugated anti-mouse IgG on a chemiluminescent substrate.

### Neutralization assay:

An antibody neutralization assay is developed to determine the pharmacological effectiveness of the humoral antibody present in the serum. In general, positive antibody titers are determined as values above the background level and are depicted as reciprocal end-dilutions. Based on a reference to the known titer, different dilutions of serum specific to the antigen are co-incubated with the disease-causing agent (virus, bacteria, or parasite). Following incubation, the ability of the test agent to infect the immortalized subject cell lines is tested *in vitro.* The reduction in the severity of infection indicates the neutralization ability of the antibody raised by vaccination. The end result can be determined using imaging, cell viability, or other techniques.

Once the generation of neutralization antibody due to vaccination is determined, a follow-up *in vivo* study is performed wherein the mice are immunized with the bacteriophage λ and challenged with the disease-causing agent. The difference in the protection from morbidity between the control and experimental group of mice is analyzed for statistical significance.

In view of the many possible embodiments to which the principles of our invention may be applied, it should be recognized that illustrated embodiments are only examples of the invention and should not be considered a limitation on the scope of the invention. Rather, the scope of the invention is defined by the following claims. We therefore claim as our invention all that comes within the scope of these claims.

## Claims

1. A bacteriophage lambda (λ), comprising:
a head,
a tail, and
a lambda genome comprising a nucleic acid sequence encoding a fusion protein comprising a D protein linked to a heterologous antigen, wherein the nucleic acid sequence is inserted into a native *gene D* locus adjacent to *gene E* in the bacteriophage lambda genome, and wherein expression of the fusion protein results in the head of the bacteriophage lambda comprising the fusion protein.

2. The bacteriophage λ of claim 1, wherein:
(a) about 1 kilobases (kb) to about 5 kb of the *b* region of the lambda genome is deleted, wherein optionally genes *ea47, ea31, and ea59* are deleted from the *b* region of the lambda genome;
(b) gene *E* encodes an E protein comprising a lysine at position 158;
(c) the fusion protein comprises the D protein, a linker, and the heterologous antigen,
wherein the linker is between the D protein and the heterologous antigen, and wherein the linker is 5 to 15 amino acids in length, wherein optionally the linker comprises or consists of amino acid sequence GGGGS (SEQ ID NO: 3); and/or
(d) the *Sam7* gene is genetically inactivated in the lambda genome.

3. The bacteriophage λ of any one of claims 1-2, wherein:
(a) the nucleic acid sequence comprises a nucleic acid sequence encoding the D protein and a nucleic acid sequence encoding the heterologous antigen, wherein the nucleic acid sequence encoding the D protein is 5' of the nucleic acid sequence encoding the heterologous antigen in the lambda genome; or
(b) the nucleic acid sequence comprises a nucleic acid sequence encoding the D protein and a nucleic acid sequence encoding the heterologous antigen, wherein the nucleic acid sequence encoding the D protein is 3' of the nucleic acid sequence encoding the heterologous antigen in the lambda genome.

4. The bacteriophage λ of any one of claims 1-3, wherein:
(a) the heterologous antigen is 5 to 1500 amino acids in length, wherein optionally the heterologous antigen is 28 to 200 amino acids in length; and/or
(b) the D protein comprises an amino acid sequence at least 95% identical to SEQ ID NO: 1, wherein optionally the D protein comprises the amino acid sequence of SEQ ID NO: 1.

5. The bacteriophage λ of any one of claims 1-4, wherein the heterologous antigen is a virus antigen, wherein optionally the virus antigen is an antigen from severe acute respiratory syndrome coronavirus (SARS-CoV) 2, human immunodeficiency virus, rotavirus, influenza virus, Dengue virus, Zika virus, Chikungunya virus, Foot and Mouth Disease (FMD) virus, or a porcine virus.

6. The bacteriophage λ of claim 5, wherein the virus antigen is from SARS-CoV-2, wherein optionally the SARS-CoV-2 antigen comprises one of SEQ ID NO: 4, SEQ ID NOs: 39-52, and SEQ ID NO: 69.

7. The bacteriophage λ of any one of claims 1-4, wherein the heterologous antigen is a tumor antigen, wherein optionally the tumor antigen is an antigen from melanoma, bladder cancer, breast cancer, colon cancer, carcinoma, sarcoma, renal cancer, thyroid cancer, brain cancer, lung cancer, prostate cancer, pancreatic cancer, a lymphoma, or a leukemia.

8. The bacteriophage λ of claim 7, wherein the tumor antigen is a) a chronic lymphocytic leukemia antigen; or b) a glioblastoma antigen, wherein optionally:
i) the chronic lymphocytic leukemia antigen comprises SEQ ID NO: 6; or
ii) the glioblastoma antigen is an epithelial growth factor receptor version III antigen comprising one of SEQ ID NOs: 53-54.

9. The bacteriophage λ of any one of claims 1-4, wherein the heterologous antigen is a bacterial antigen or a parasitic antigen, wherein optionally the parasitic antigen is a *Plasmodium* antigen or a *Leishmania* antigen, and wherein preferably the *Plasmodium* antigen comprises SEQ ID NO: 5.

10. The bacteriophage λ of any one of claims 1-9, wherein:
(a) the bacteriophage λ further encoding a side tail fiber (stf) fusion protein and/or a gpV fusion protein, wherein the side tail fiber fusion protein and/or the gpV fusion protein is expressed on the tail of the bacteriophage lambda; or
(b) the head further comprises wild-type D protein, wherein optionally the wild type D protein comprises the amino acid sequence of SEQ ID NO: 1.

11. A host bacterial cell infected with the bacteriophage λ of any one of claims 1-10,
wherein the lambda genome becomes integrated into a chromosome of the host bacterial cell as a prophage.

12. The host bacterial cell of claim 11, further comprising:
(a) a nucleic acid sequence encoding wild type D protein inserted into the chromosome of the host bacterial cell, and optionally further comprising an arabinose promoter operably linked to the nucleic acid sequence encoding the wild type D protein;
(b) genetic inactivation of *fhuA* in the chromosome of the host bacterial cell; and/or
(c) genetic inactivation of *lamB* in the chromosome of the host bacterial cell.

13. An immunogenic composition comprising an effective amount of the bacteriophage λ of any one of claims 1-10, and a pharmaceutically acceptable carrier.

14. The immunogenic composition of claim 13 for use in treating or preventing a tumor, an infection, an autoimmune disorder, an allergy or an allergic condition in a subject, wherein an immune response to the heterologous antigen is induced in the subject.

15. A method of preparing bacteriophage λ, comprising:
propagating the host bacterial cell of any one of claims 11-12;
inducing the lytic phase of the bacteriophage λ; and
isolating bacteriophage λ phage particles,
wherein optionally the host bacterial cell comprises a plasmid encoding wild-type D protein.

## Patentansprüche

1. Bakteriophagen Lambda (λ), umfassend:
einen Kopf,
einen Schwanz, und
ein Lambda-Genom, das eine Nukleinsäuresequenz umfasst, die ein Fusionsprotein kodiert, das ein an ein heterologes Antigen gebundenes D-Protein umfasst, wobei die Nukleinsäuresequenz in einen nativen *Gen-D-Locus* neben dem *Gen E* in dem Bakteriophagen-Lambda-Genom insertiert wird, und wobei die Expression des Fusionsproteins dazu führt, dass der Kopf des Bakteriophagen Lambda das Fusionsprotein umfasst.

2. Bakteriophagen λ nach Anspruch 1, wobei:
(a) etwa 1 Kilobase (kb) bis etwa 5 kb der *b*-Region des Lambda-Genoms deletiert sind, wobei optional die Gene *ea47, ea31 und* ea59 aus der *b*-Region des Lambda-Genoms deletiert sind;
(b) das Gen *E* ein E-Protein kodiert, das ein Lysin an Position 158 umfasst;
(c) das Fusionsprotein das D-Protein, einen Linker und das heterologe Antigen umfasst, wobei der Linker zwischen dem D-Protein und dem heterologen Antigen liegt, und wobei der Linker eine Länge von 5 bis 15 Aminosäuren hat, wobei der Linker optional die Aminosäuresequenz GGGGS (SEQ ID NO: 3) umfasst oder daraus besteht; und/oder
(d) das *Sam7*-Gen in dem Lambda-Genom genetisch inaktiviert ist.

3. Bakteriophagen λ nach einem der Ansprüche 1-2, wobei:
(a) die Nukleinsäuresequenz eine Nukleinsäuresequenz, die das D-Protein kodiert, und eine Nukleinsäuresequenz, die das heterologe Antigen kodiert, umfasst, wobei die Nukleinsäuresequenz, die das D-Protein kodiert, 5' der Nukleinsäuresequenz liegt, die das heterologe Antigen in dem Lambda-Genom kodiert; oder
(b) die Nukleinsäuresequenz eine Nukleinsäuresequenz, die das D-Protein kodiert, und eine Nukleinsäuresequenz, die das heterologe Antigen kodiert, umfasst, wobei die Nukleinsäuresequenz, die das D-Protein kodiert, 3' von der Nukleinsäuresequenz liegt, die das heterologe Antigen in dem Lambda-Genom kodiert.

4. Bakteriophagen λ nach einem der Ansprüche 1-3, wobei:
(a) das heterologe Antigen eine Länge von 5 bis 1500 Aminosäuren aufweist, wobei das heterologe Antigen optional 28 bis 200 Aminosäuren lang ist; und/oder
(b) das D-Protein eine Aminosäuresequenz umfasst, die mindestens 95 % identisch zu SEQ ID NO: 1 ist, wobei das D-Protein optional die Aminosäuresequenz von SEQ ID NO: 1 umfasst.

5. Bakteriophagen λ nach einem der Ansprüche 1-4, wobei das heterologe Antigen ein Virusantigen ist, wobei es sich bei dem Virusantigen optional um ein Antigen aus dem Coronavirus des schweren akuten Respirationssyndroms (SARS-CoV) 2, dem humanen Immundefizienzvirus, dem Rotavirus, dem Influenzavirus, dem Dengue-Virus, dem Zika-Virus, dem Chikungunya-Virus, dem Virus der Maul- und Klauenseuche (FMD) oder einem Schweinevirus handelt.

6. Bakteriophage λ nach Anspruch 5, wobei das Virusantigen von SARS-CoV-2 stammt, wobei optional das SARS-CoV-2-Antigen eine der SEQ ID NO: 4, SEQ ID NOs: 39-52, und SEQ ID NO: 69 umfasst.

7. Bakteriophage λ nach einem der Ansprüche 1-4, wobei das heterologe Antigen ein Tumorantigen ist, wobei das Tumorantigen optional ein Antigen aus Melanom, Blasenkrebs, Brustkrebs, Dickdarmkrebs, Karzinom, Sarkom, Nierenkrebs, Schilddrüsenkrebs, Hirnkrebs, Lungenkrebs, Prostatakrebs, Bauchspeicheldrüsenkrebs, einem Lymphom oder einer Leukämie ist.

8. Bakteriophagen λ nach Anspruch 7, wobei das Tumorantigen a) ein chronisches lymphatisches Leukämie-Antigen; oder b) ein Glioblastom-Antigen ist, wobei optional:
i) das Antigen der chronischen lymphatischen Leukämie SEQ ID NO: 6 umfasst; oder
ii) das Glioblastom-Antigen ein Epithelwachstumsfaktor-Rezeptor-Version-III-Antigen ist, das eine der SEQ ID NOs: 53-54 umfasst.

9. Bakteriophagen λ nach einem der Ansprüche 1-4, wobei das heterologe Antigen ein bakterielles Antigen oder ein parasitäres Antigen ist, wobei das parasitäre Antigen optional ein *Plasmodium-Antigen* oder ein *Leishmania-Antigen* ist, und wobei das *Plasmodium-*Antigen vorzugsweise SEQ ID NO: 5 umfasst.

10. Bakteriophagen λ nach einem der Ansprüche 1-9, wobei:
(a) der Bakteriophagen λ weiter ein Fusionsprotein der seitlichen Schwanzfaser (stf) und/oder ein gpV-Fusionsprotein kodiert, wobei das Fusionsprotein der seitlichen Schwanzfaser und/oder das gpV-Fusionsprotein an dem Schwanz des Bakteriophagen Lambda exprimiert wird; oder
(b) der Kopf weiter Wildtyp-D-Protein umfasst, wobei das Wildtyp-D-Protein optional die Aminosäuresequenz von SEQ ID NO: 1 umfasst.

11. Bakterielle Wirtszelle, die mit dem Bakteriophagen λ nach einem der Ansprüche 1-10 infiziert ist, wobei das Lambda-Genom als Prophage in ein Chromosom der bakteriellen Wirtszelle integriert wird.

12. Bakterielle Wirtszelle nach Anspruch 11, weiter umfassend:
(a) eine Nukleinsäuresequenz, die ein Wildtyp-D-Protein kodiert, das in das Chromosom der bakteriellen Wirtszelle insertiert ist, und die optional weiter einen Arabinose-Promotor umfasst, der funktionsfähig mit der Nukleinsäuresequenz verbunden ist, die das Wildtyp-D-Protein kodiert;
(b) genetische Inaktivierung von *fhuA* in dem Chromosom der bakteriellen Wirtszelle; und/oder
(c) genetische Inaktivierung von *lamB* in dem Chromosom der bakteriellen Wirtszelle.

13. Immunogene Zusammensetzung, umfassend eine wirksame Menge des Bakteriophagen λ nach einem der Ansprüche 1-10, und einen pharmazeutisch annehmbaren Träger.

14. Immunogene Zusammensetzung nach Anspruch 13 zur Verwendung bei der Behandlung oder Vorbeugung eines Tumors, einer Infektion, einer Autoimmunerkrankung, einer Allergie oder eines allergischen Zustands bei einem Subjekt, wobei eine Immunantwort auf das heterologe Antigen in dem Subjekt induziert wird.

15. Verfahren zur Herstellung eines Bakteriophagen λ, umfassend:
Vermehren der bakteriellen Wirtszelle nach einem der Ansprüche 11-12;
Induzieren der lytischen Phase des Bakteriophagen λ; und
Isolieren von Bakteriophagen λ-Phagenpartikeln,
wobei die bakterielle Wirtszelle optional ein Plasmid umfasst, das das Wildtyp-D-Protein kodiert.

## Revendications

1. Bactériophage lambda (λ), comprenant :
une tête,
une queue, et
un génome lambda comprenant une séquence d'acide nucléique codant pour une protéine de fusion comprenant une protéine D liée à un antigène hétérologue, dans lequel la séquence d'acide nucléique est insérée dans un locus de *gène D* natif adjacent au *gène E* dans le génome du bactériophage lambda, et dans lequel l'expression de la protéine de fusion se traduit par le fait que la tête du bactériophage lambda comprend la protéine de fusion.

2. Bactériophage λ selon la revendication 1, dans lequel :
(a) environ 1 kilobase (kb) à environ 5 kb de la région *b* du génome lambda sont supprimées, dans lequel, facultativement, les gènes *ea47, ea31 et ea59* sont supprimés de la région *b* du génome lambda ;
(b) le gène *E* code pour une protéine E comprenant une lysine en position 158 ;
(c) la protéine de fusion comprend la protéine D, un liant, et l'antigène hétérologue, dans lequel le liant se trouve entre la protéine D et l'antigène hétérologue, et dans lequel le liant est de 5 à 15 acides aminés de longueur, dans lequel, facultativement, le liant comprend ou consiste en une séquence d'acides aminés GGGGS (SEQ ID NO : 3) ; et/ou
(d) le gène *Sam7* est génétiquement inactivé dans le génome lambda.

3. Bactériophage λ selon l'une quelconque des revendications 1 et 2, dans lequel :
(a) la séquence d'acide nucléique comprend une séquence d'acide nucléique codant pour la protéine D et une séquence d'acide nucléique codant pour l'antigène hétérologue, dans lequel la séquence d'acide nucléique codant pour la protéine D est en 5' de la séquence d'acide nucléique codant pour l'antigène hétérologue dans le génome lambda ; ou
(b) la séquence d'acide nucléique comprend une séquence d'acide nucléique codant pour la protéine D et une séquence d'acide nucléique codant pour l'antigène hétérologue, dans lequel la séquence d'acide nucléique codant pour la protéine D est en 3' de la séquence d'acide nucléique codant pour l'antigène hétérologue dans le génome lambda.

4. Bactériophage λ selon l'une quelconque des revendications 1 à 3, dans lequel :
(a) l'antigène hétérologue est de 5 à 1 500 acides aminés de longueur, dans lequel, facultativement, l'antigène hétérologue est de 28 à 200 acides aminés de longueur ; et/ou
(b) la protéine D comprend une séquence d'acides aminés au moins 95 % identique à SEQ ID NO : 1, dans lequel, facultativement, la protéine D comprend la séquence d'acides aminés de SEQ ID NO : 1.

5. Bactériophage λ selon l'une quelconque des revendications 1 à 4, dans lequel l'antigène hétérologue est un antigène de virus, dans lequel, facultativement, l'antigène de virus est un antigène de coronavirus 2 du syndrome respiratoire aigu sévère (SARS-CoV), de virus de l'immunodéficience humaine, de rotavirus, de virus de la grippe, de virus de la dengue, de virus Zika, de virus du chikungunya, d'aphthovirus, ou d'un virus porcin.

6. Bactériophage λ selon la revendication 5, dans lequel l'antigène de virus est du SARS-CoV-2, dans lequel, facultativement, l'antigène de SARS-CoV-2 comprend l'une de SEQ ID NO : 4, de SEQ ID NO : 39-52, et de SEQ ID NO : 69.

7. Bactériophage λ selon l'une quelconque des revendications 1 à 4, dans lequel l'antigène hétérologue est un antigène de tumeur, dans lequel, facultativement, l'antigène de tumeur est un antigène de mélanome, de cancer de la vessie, de cancer du sein, de cancer du côlon, de carcinome, de sarcome, de cancer du rein, de cancer de la thyroïde, de cancer du cerveau, de cancer du poumon, de cancer de la prostate, de cancer du pancréas, d'un lymphome, ou d'une leucémie.

8. Bactériophage λ selon la revendication 7, dans lequel l'antigène de tumeur est a) un antigène de leucémie lymphoïde chronique ; ou b) un antigène de glioblastome, dans lequel, facultativement :
i) l'antigène de leucémie lymphoïde chronique comprend SEQ ID NO : 6 ; ou
ii) l'antigène de glioblastome est un antigène de récepteur du facteur de croissance épithélial de version III comprenant l'une de SEQ ID NO : 53-54.

9. Bactériophage λ selon l'une quelconque des revendications 1 à 4, dans lequel l'antigène hétérologue est un antigène bactérien ou un antigène parasitaire, dans lequel, facultativement, l'antigène parasitaire est un antigène *Plasmodium* ou un antigène *Leishmania,* et dans lequel, de préférence, l'antigène *Plasmodium* comprend SEQ ID NO : 5.

10. Bactériophage λ selon l'une quelconque des revendications 1 à 9, dans lequel :
(a) le bactériophage λ code en outre pour une protéine de fusion de fibre de queue latérale (stf) et/ou une protéine de fusion gpV, dans lequel la protéine de fusion de fibre de queue latérale et/ou la protéine de fusion gpV est exprimée sur la queue du bactériophage lambda ; ou
(b) la tête comprend en outre une protéine D de type sauvage, dans lequel, facultativement, la protéine D de type sauvage comprend la séquence d'acides aminés de SEQ ID NO : 1.

11. Cellule bactérienne hôte infectée par le bactériophage λ selon l'une quelconque des revendications 1 à 10, dans laquelle le génome lambda devient intégré dans un chromosome de la cellule bactérienne hôte en tant qu'un prophage.

12. Cellule bactérienne hôte selon la revendication 11, comprenant en outre :
(a) une séquence d'acide nucléique codant pour une protéine D de type sauvage insérée dans le chromosome de la cellule bactérienne hôte, et comprenant en outre facultativement un promoteur d'arabinose lié de manière fonctionnelle à la séquence d'acide nucléique codant pour la protéine D de type sauvage ;
(b) l'inactivation génétique de *fhuA* dans le chromosome de la cellule bactérienne hôte ; et/ou
(c) l'inactivation génétique de *lamB* dans le chromosome de la cellule bactérienne hôte.

13. Composition immunogène comprenant une quantité efficace du bactériophage λ selon l'une quelconque des revendications 1 à 10, et un support pharmaceutiquement acceptable.

14. Composition immunogène selon la revendication 13 pour une utilisation dans un traitement ou une prévention d'une tumeur, d'une infection, d'un trouble auto-immun, d'une allergie ou d'une affection allergique chez un sujet, dans laquelle une réponse immunitaire à l'antigène hétérologue est induite chez le sujet.

15. Procédé de préparation de bactériophage λ, comprenant :
la propagation de la cellule bactérienne hôte selon l'une quelconque des revendications 11 et 12 ;
l'induction de la phase lytique du bactériophage λ ; et
l'isolement des particules de phage du bactériophage λ,
dans lequel, facultativement, la cellule bactérienne hôte comprend un plasmide codant pour une protéine D de type sauvage.
